# EUROPEAN PATENT SPECIFICATION

(11) **EP 1 934 234 B1**
(45) Date of publication and mention of the grant of the patent: **30.11.2011**
(21) Application number: 06796084.9
(22) Date of filing: 13.09.2006
(51) Int. Cl.: C07F 5/02, A61K 31/69, A61K 51/00

(54) **NOVEL AMINE-BORANE COMPOUNDS AND USES THEREOF**
NEUE AMINBORANVERBINDUNGEN UND ANWENDUNGEN DAVON
NOUVEAUX COMPOSES D'AMINE-BORANE ET UTILISATIONS DE CEUX-CI

(30) Priority: 13.09.2005 US 716081 P; 14.09.2005 US 716487 P
(43) Date of publication of application: 25.06.2008
(73) Proprietor: Yissum Research Development Company, of The Hebrew University of Jerusalem, 91390 Jerusalem (IL); Soreq Nuclear Research Center Israel Atomic Energy Commission, 81800 Yavne (IL)
(72) Inventor: SREBNIK, Morris, 90805 Mevasseret Zion (IL); TAKROURI, Khuloud, 97300 Jerusalem (IL); SHALOM, Eli, 75795 Rishon-LeZion (IL)
(74) Representative: Machtalère, Georges
(86) International application number: PCT/IL2006/001074
(87) International publication number: WO 2007/032005

(56) References cited:
- TAKROURI, KHULOUD ET AL: "Synthesis and Antifungal Activity of a Novel Series of Alkyldimethylamine Cyanoboranes and Their Derivatives" JOURNAL OF MEDICINAL CHEMISTRY , 49(16), 4879-4885 CODEN: JMCMAR; ISSN: 0022-2623, 2006, XP002419318
- SHALOM, ELI ET AL: "Preparation of Novel Fluoro Derivatives of Amine Cyanoboranes and Amine Carboxyborane Esters" ORGANOMETALLICS , 24(23), 5776-5779 CODEN: ORGND7; ISSN: 0276-7333, 2005, XP002419319
- DATABASE CA [Online] CHEMICAL ABSTRACTS SERVICE, COLUMBUS, OHIO, US; SHIBLI, AMAL ET AL: ".alpha.-Amino boronates as cyanoborane complexes: crystal structure and inhibition properties for the serine proteases: .alpha.-chymotrypsin and trypsin" XP002419326 retrieved from STN Database accession no. 2006:791981 & APPLIED ORGANOMETALLIC CHEMISTRY , 20(7), 459-462 CODEN: AOCHEX; ISSN: 0268-2605, 2006,
- DATABASE CA [Online] CHEMICAL ABSTRACTS SERVICE, COLUMBUS, OHIO, US; HALL, IRIS H. ET AL: "The anti-inflammatory activity of boron derivatives in rodents" XP002419327 retrieved from STN Database accession no. 123:510 cited in the application & METAL-BASED DRUGS , 2(1), 1-12 CODEN: MBADEI; ISSN: 0793-0291, 1995,
- DATABASE CA [Online] CHEMICAL ABSTRACTS SERVICE, COLUMBUS, OHIO, US; SPIELVOGEL, BERNARD F. ET AL: "The cytotoxicity of amine-cyanoboranes, amine-cyanoalkylboranes, and aminomethyl-phosphonate cyanoborane adducts against the growth of murine and human tissue culture cells" XP002419328 retrieved from STN Database accession no. 116:120392 & PHARMAZIE , 46(8), 592-4 CODEN: PHARAT; ISSN: 0031-7144, 1991,
- DATABASE CA [Online] CHEMICAL ABSTRACTS SERVICE, COLUMBUS, OHIO, US; SOOD, ANUP ET AL: "Synthesis and hypolipidemic activity of amine-carboxyboranes, and their amides and esters in rodents" XP002419329 retrieved from STN Database accession no. 115:247450 cited in the application & ARCHIV DER PHARMAZIE (WEINHEIM, GERMANY) , 324(7), 423-32 CODEN: ARPMAS; ISSN: 0365-6233, 1991,
- DATABASE CA [Online] CHEMICAL ABSTRACTS SERVICE, COLUMBUS, OHIO, US; HALL, IRIS H. ET AL: "The antineoplastic activity of trimethylamine carboxyboranes and related esters and amides in murine and human tumor cell lines" XP002419330 retrieved from STN Database accession no. 115:105431 & ANTI-CANCER DRUGS , 1(2), 133-41 CODEN: ANTDEV; ISSN: 0959-4973, 1990,
- DATABASE CA [Online] CHEMICAL ABSTRACTS SERVICE, COLUMBUS, OHIO, US; HALL, IRIS H. ET AL: "Hypolipidemic activity of trimethylamine-carbomethoxyborane and related derivatives in rodents" XP002419331 retrieved from STN Database accession no. 107:228725 & JOURNAL OF PHARMACEUTICAL SCIENCES , 76(5), 359-65 CODEN: JPMSAE; ISSN: 0022-3549, 1987,

## Description

### FIELD AND BACKGROUND OF THE INVENTION

The present invention relates to novel amine-borane compounds and to uses thereof in a variety of therapeutic and diagnostic applications.

In the past few decades, amine-borane compounds have been considered as highly sought synthetic targets. Amine-borane compounds exhibit high similarity to organic compounds mainly due to the atomic radii and the characteristics of the B-N bond, which resemble those of carbon-carbon bonds. Thus, for example, a H₂B-NH₂ bond resembles a H₂C=CH₂ double bond, while a H₃B←NH₃ bond resembles a H₃C-CH₃ single bond.

Amine-borane compounds such as, for example, α-aminoboronic acids, amine-carboxyboranes, amine-cyanoboranes, and related compounds are therefore isoelectronic and isostructural analogs of many biologically active compounds such as amino acids, neurotransmitters, nucleosides, and nucleic acids and hence can mimic the biological activity of such compounds in the body. Doing so, these boron compounds may act as inhibitors, antagonists and otherwise effectors of many biological systems and hence have been widely recognized as highly potential therapeutic agents.

For example, α-amino alkylboronic acids are analogs of α-amino acids which may act as inhibitors of enzymes involved in amino acid and peptide metabolism. α-Amino alkylboronic acids, in which the carboxyl group of the corresponding amino acids is replaced by a boronic acid function, constitute a unique class of amino acid mimics from which a number of potent enzyme inhibitors were synthesized. The inhibitory activity of such compounds mainly stems from the fact that the tetrahedral adduct of electrophilic boronic acid is a good mimic of the putative tetrahedral transition state or intermediate encountered in the enzymatic hydrolysis or formation of peptides. Since the peptide hydrolysis and formation invariably involves the tetrahedral high energy species in the course of the reaction, these amino acid mimics serve as a general key element for inhibitors of a broad spectrum of proteases and peptide ligases.

Additional amine-boranes and compounds having a bis-borane, and mainly cyano and carboxy borane derivatives of these families are disclosed in U.S. Patent Nos. 4,301,129, 4,312,989, 4,368,194, 4,550,186, 4,647,555, 4,658,051, 4,740,504, 4,774,354, 4,855,493, 4,977,268, 5,280,119 and 5,312,816 and in Hall, I.H. et al., J. Pharm. Sci. 1980, 69(9), 1025.; Sood, C.K. et al., J. Pharm. Sci. 1991, 80(12),1133; Dembitsky, V.M., et al., Tetrahedron 2003, 59,579; E. Shalom et al., Organometallics. 2004, 23, 4396-4399; Berdy, J., Handbook of Antibiotic Compounds. Part IV, CRC, 1980; Fink, K. et al., Science, 1948, 108, 358-9 ; Hunt, S., Methods Enzymol. 1984, 107, 413-438; Jimenez, E. C. et al., Biochemistry 1997, 36, 984-989; Takrouri et al., Organometallics, 2004, 23(11), 2817-2820 ; and Gyoeri, B. et al., Inorganic Chemistry, 1998, 37(20), 5131-5141.

The first amine-cyanoboranes and amine-carboxyboranes which stood-out as pharmacologically promising compounds in the early 1980's [1] were adducts of tertiary low-alkyl ammonium salts, typically trimethylammonium chloride, and sodium cyanoborane. Further derivatization of this basic form afforded amines-boranes with aromatic, heterocyclic and silyl substituents on the amine, substitutions of the boron by low-alkyls and bromine, and esters of amine-carboxyboranes [2].

Model studies had shown that these compounds have potent anticancer activity [3, 4-81, antihyperlipodemic activity [9, 11], anti-obesity activity [9], antiosteoporotic activity [9, 12], anti-inflammatory activity [9, 1, 13], hypolipidemic activity [5, 14], anti-neoplastic activity [15, 16] and other promising biological activities [17], yet their exact mechanism of action is still not fully understood.

For example, amine-cyanoboranes and amine-carboxyborane with varying alkyl chain lengths [7], derivatives and metal complexes [4] thereof were examined in various *in vitro* and *in vivo* experiments [9, 18], and shown to be effective anti-neoplastic and cytotoxic agents with selective activity against single-cell and solid tumors derived from murine and human leukemias, lymphomas, sarcomas, and carcinomas. These agents inhibited DNA and RNA synthesis in preference to protein synthesis in L1210 lymphoid leukemia cells, possibly targeting inosine-monophosphate dehydrogenase. Similar effects on phosphoribosyl-pyrophosphate amido transferase, orotidine-monophosphate decarboxylase, and both nucleoside and nucleotide kinases were also observed, as well as deoxyribonucleotide pool levels reduction in the cells and DNA strand scission.

In experiments conducted with rodents, amine carboxyboranes and amine-cyanoboranes were found to act as potent hypolipidemic agents, lowering both serum cholesterol and triglyceride concentrations, in addition to lowering cholesterol content of very low-density lipoprotein and low-density lipoprotein (LDL) and elevating high-density lipoprotein (HDL) cholesterol concentrations [19]. De novo regulatory enzymes involved in lipid synthesis (e.g., hypocholesterolemic 3-hydroxy-3-methyl-CoA reductase, acyl-CoA cholesterol acyltransferase and sn-glycerol-3-phosphate acyltransferase) were also found to be inhibited by these compounds. Concurrently, these compounds modulated LDL and HDL receptor binding, internalization, and degradation, so that less cholesterol was delivered to the plaques and more was broken down from esters and conducted to the liver for biliary excretion. Tissue lipids in the aorta wall of the rat were reduced and fewer atherosclerotic morphology lesions were present in quail aortas after treatment with the agents. Cholesterol resorption from the rat intestine was reduced in the presence of these compounds. Genetic hyperlipidemic mice demonstrated the same types of reduction after treatment with these compounds. The amine-borane compounds effectively lowered lipids in tissue based on the inhibition of regulatory enzymes in pigs.

The amine-boranes were further found to act as effective anti-inflammatory agents against septic shock, induced edema, pleurisy, and chronic arthritis at 2.5 to 8 mg/kg. Lysosomal and proteolytic enzyme activities were also inhibited. More significantly, the amine-borane compounds were found to exhibit dual inhibitory activity of prostaglandin cyclooxygenase and 5'-lipoxygenase. These compounds also affected cytokine release and white cell migration. Subsequent studies showed that the amine-boranes were potent anti-osteoporotic agents reducing calcium resorption as well as increasing calcium and proline incorporation into mouse pup calvaria and rat UMR-146 collagen [9].

In another study, acyclic amine-carboxyboranes were shown to be more efficacious anti-inflammatory agents and in reducing local pain in mice than indomethacin, pentoxifylline, and phenylbutazone, while on the other hand the heterocyclic amine derivatives as well as amine-carbamoylboranes, amine-carboalkoxyboranes, and amine-cyanoboranes were generally less efficacious. These amine-borane compounds were also pharmaceutically active in rat induced with edema, rat induced with chronic arthritis, and in pleurisy screens [15,20]. For example, trimethylamine-carbomethoxyborane, Me₃NBH₂CO₂Me, was found to be an effective hypolipidemic amine-borane in rodents and to exhibit a safe therapeutic index, making it a promising therapeutic agent [21].

Furthermore, amine-carboxyboranes and amine-cyanoboranes were shown to act in synergy with tumor necrosis factor alpha (TNFα) in cytotoxicity and inhibition of DNA synthesis in selected types of cancer cells. The synergistic effect was found to be dependent on the presence of a high affinity TNFα receptor on the membrane of the cells. This event correlated with increased DNA protein linked breaks, DNA fragmentation and cell death [8].

Hall et al., [Anti-Cancer Drugs, 1(2), 133-41, 1990] teach trimethylamine carboxyboranes including their esters and amides, having antineoplastic activity *in vivo* and *in vitro* in several cancerous cells and tissues.

Apart from having an evident direct therapeutic value, boron containing compounds, and especially compounds which can interact with biological systems, can be used as radiotherapy agents in Neutron Capture Therapy (NCT). NCT is a form of treatment that requires the infusion of an element such as boron or gadolinium and exposure of the patient to a neutron beam from a nuclear reactor. At the present time there are two therapeutic targets, glioblastoma, a highly malignant tumor whose treatment is not satisfactory, and malignant melanoma. Boron Neutron Capture Therapy (BNCT) achieves the selective irradiation and destruction of malignant tumor cells by using boronated compounds having the ¹⁰B isotope incorporated therein.

The analogous structure of amine-boranes to naturally occurring biological compounds, make them ideal candidates for designing highly specific ligands of high affinity to a variety of biological targets and systems, rendering these compounds valuable tools for nuclear medicinal and research procedures, apart of being ideal candidates for drug discovery and development. Yet, hitherto, radiolabeled amine-boranes that can be used as radiopharmaceutical compounds have not been taught.

While the presently known amine-boranes compounds have shown to be useful therapeutic agents, these compounds are still far from having an acceptable pharmaceutical profile, eligible for use in humans and animals. These compounds suffer from low affinity to their intended target, and therefore require administration of relatively high doses. The non-specificity and high dosage of the presently known amine-borane compounds are typically associated with adverse effects and toxicity.

Shibli et al. [Applied Organometallic Chemistry, 20(7), 459-462, 2006], by some of the present inventors, teach a series of amine-cyanoborane derivatives exhibiting antifungal activity, characterized by having a long alkyl chain attached to the nitrogen of the amine cyanoboranes and carboxyboranes, which is found to enhance antifungal activity, and further teach enhance activity obtained by halogenation of the amine cyanoboranes and by the presence of a double bond at the end of the N-alkyl group.

There is thus a widely recognized need for, and it would be highly advantageous to have novel amine-borane compounds devoid of the above limitations.

### SUMMARY OF THE INVENTION

The present inventors have now designed and successfully prepared and practiced novel classes of amine-borane compounds.

Thus, according to one aspect of the present invention there is provided a compound having a general formula selected from the group consisting of Formula I, II and III: 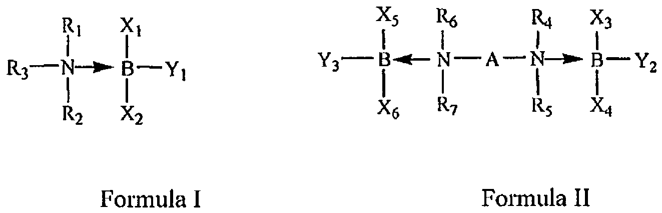 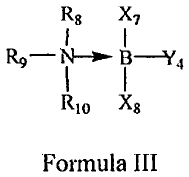 or a pharmaceutically acceptable salt thereof, wherein:
Y₁-Y₄ are each independently selected from the group consisting of a cyano group (-C≡N), a -C(=O)Ra group, amine and alkyl, whereas Ra is hydrogen, halogen, hydroxy, alkoxy, thiohydroxy, thioalkoxy, aryloxy, thioaryloxy, thiol and amine;
X₁-X₈ are each independently selected from the group consisting of hydrogen, alkyl, halogen, cycloalkyl, and aryl, provided that:
   at least one of X₁ and X₂ in Formula I is fluorine;
   R₁-R₁₀ are each independently selected from the group consisting of hydrogen, alkyl, alkenyl, alkynyl, cycloalkyl and aryl or, alternatively, two of R₁-R₃, R₄ and R₅ R₆ and R₇ and/or R₈-R₁₀ form a carbocyclic ring, provided that:
      one or more of R₈-R₁₀ in Formula III is a saturated alkyl having 11-15 carbon atoms; one or more of R₈-R₁₀ in Formula III is an unsaturated alkyl having 5-20 carbon atoms; and/or one or more of R₈-R₁₀ in Formula III comprises at least one fluorine; and
      A is a substituted or non-substituted, saturated or non-saturated hydrocarbon having from 5 to 20 carbon atoms.

According to further features in preferred embodiments of the invention described below, when the compound comprises fluorine, the fluorine is selected from the group consisting of a non-radioactive fluorine and a radioactive fluorine. Preferably, the radioactive fluorine is fluorine-18 [¹⁸F].

According to still further features in the described preferred embodiments the compound has the general Formula I, wherein Y₁ is preferably a cyano group - (C=N) or a -C(=O)Ra group and Ra is preferably selected from the group consisting of hydroxy and alkoxy (e.g., methoxy or ethoxy).

According to still further features in the described preferred embodiments X₁ is fluorine and X₂ is selected from the group consisting of hydrogen, fluorine, bromine and iodine. Preferably X₁ is fluorine and X₂ is bromine.

According to still further features in the described preferred embodiments each of R₁-R₃ is alkyl, preferably selected from the group consisting of methyl, ethyl and n-butyl.

According to still further features in the described preferred embodiments at least one of R₁-R₃ is a C₅-C₂₀ alkyl. The alkyl can be a saturated alkyl or an unsaturated alkyl.

According to still further features in the described preferred embodiments non-limiting examples of compounds having Formula I include dimethyl-undecyl-amine cyanofluorobromoborane, trimethyl-amine cyanofluoroborane, ethyl-dimethyl-amine cyanofluoroborane, butyl-dimethyl-amine cyanofluoroborane, trimethyl-amine carboxyfluoroborane methyl ester, trimethyl-amine carboxyfluoroborane ethyl ester, ethyl-dimethyl-amine carboxyfluoroborane methyl ester, butyl-dimethyl-amine carboxyfluoroborane methyl ester, trimethyl-amine cyanodifluoroborane, trimethyl-amine carboxydifluoroborane methyl ester, trimethyl-amine carboxydifluoroborane ethyl ester, trimethyl-amine cyanofluorobromoborane, trimethyl-amine carboxyfluorobromoborane ethyl ester and triethyl-amine carboxydifluoroborane.

According to still further features in the described preferred embodiments the compound has the general Formula II. Preferably each of Y₂ and Y₃ is a cyano group (-C≡N). Optionally, each of Y₂ and Y₃ is a -C(=O)Ra group, Ra is selected from the group consisting of hydroxy and alkoxy.

According to still further features in the described preferred embodiments each of X₃, X₄, X₅ and X₆ is independently selected from the group consisting of hydrogen, fluorine, bromine and iodine. Preferably one or more of X₃, X₄, X₅ and X₆ is bromine.

According to still further features in the described preferred embodiments each of R₄-R₇ is alkyl (e.g., methyl).

According to still further features in the described preferred embodiments A is a saturated, non-substituted hydrocarbon. Preferably, the hydrocarbon has from 8 to 14 carbon atoms.

According to still further features in the described preferred embodiments non-limiting examples of compounds having Formula II include N,N,N',N'-tetramethyl-decane-1,10-diamine bis-cyanoborane, N,N,N',N'-tetramethyl-decane-1,10-diamine bis-cyanobromoborane, N,N,N',N'-tetramethyl-decane-1,10-diamine bis-cyanodibromoborane, N,N,N',N'-tetramethyl-decane-1,10-diamine bis-carboxyboranes, N,N,N',N'-tetramethyl-dodecane-1,12-diamine bis-cyanoborane and N,N,N',N'-tetramethyl-tetradecane-1,14-diamine bis-cyanoborane.

According to still further features in the described preferred embodiments the compound has the general Formula III.

According to still further features in the described preferred embodiments one or more of R₈-R₁₀ is a saturated alkyl having 11-15 carbon atoms or one or more of R₈-R₁₀ is an unsaturated alkyl having 5-20 carbon atoms.

According to still further features in the described preferred embodiments at least one of R₈-R₁₀ is an unsaturated alkyl.

According to still further features in the described preferred embodiments at least one of R₈-R₁₀ comprises a hydroxy, whereas the hydroxy is at position β to the amine nitrogen.

According to still further features in the described preferred embodiments at least one of R₈-R₁₀ comprises a fluorine, whereas the fluorine is at position β to the amine nitrogen.

According to still further features in the described preferred embodiments Y₁ is selected from the group consisting of a cyano group -(C≡N) and a -C(=O)Ra group, as described hereinabove.

According to still further features in the described preferred embodiments each of X₇ and X₈ is independently selected from the group consisting of hydrogen, fluorine, bromine and iodine. Preferably one or more of X₇ and X₈ is bromine.

According to still further features in the described preferred embodiments non-limiting examples of compounds having Formula III include dimethyl-undecyl-amine cyanoborane, dimethyl-undecyl-amine cyanobromoborane, dimethyl-undecyl-amine cyanodibromoborane, dodecyl-dimethyl-amine cyanoborane, 1-dimethylamino-2-methyl-octan-2-ol cyanoborane, dimethyl-nonyl-amine cyanoborane, dimethyl-tridecyl-amine cyanoborane, dimethyl-pentadecyl-amine cyanoborane, heptadecyl-dimethyl-amine cyanoborane, 1-dimethylamino-dodecan-2-ol cyanoborane, 1-dimethylamino-undecan-2-ol cyanoborane, dimethyl-undecyl-amine cyanofluorobromoborane and (2-fluoro-nonyl)-dimethyl-amine cyanoborane.

According to another aspect of the present invention there is provided a pharmaceutical composition comprising, as an active ingredient, any of the novel compounds described herein and a pharmaceutically acceptable carrier.

According to yet another aspect of the present invention there is provided a use of the novel compounds described herein in the treatment of a medical condition associated with a pathogenic microorganism.

According to an additional aspect of the present invention there is provided a use of the novel compounds described herein for the preparation of a medicament.

The medicament can be, for example, for the treatment of a medical condition associated with a pathogenic microorganism.

The medical condition can be, for example, a bacterial infection, a protozoan infection, a fungal infection, malaria and leishmaniasis.

Preferably the uses described herein are directed at pathogenic microorganism which are drug-resistant to conventional antimicrobial drugs and agents.

According to a further aspect of the present invention there is provided a process of preparing the compound having the general Formula I, the process comprising:
reacting a compound having the general Formula IV: 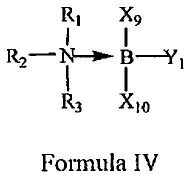 or a pharmaceutically acceptable salt thereof,
   wherein:
   Y₁ is selected from the group consisting of a cyano group -(C≡N), a -C(=O) Ra group, amine and alkyl, whereas Ra is hydrogen, halo, hydroxy, alkoxy, thiohydroxy, thioalkoxy, aryloxy, thioaryloxy, thiol, and amine;
   X₉ and X₁₀ are each independently selected from the group consisting of hydrogen, alkyl, halogen, cycloalkyl, aryl, provided that at least one of X₁ and X₂ is bromine; and
   R₁-R₃ are each independently selected from the group consisting of hydrogen, alkyl, cycloalkyl and aryl or, alternatively, two of R₁-R₃ form a carbocyclic ring,
   with a fluorinating agent, thereby obtaining the compound having general Formula I.

The fluorinating agent is preferably selected from the group consisting of silver fluoride and a mixture of triethylamine and trihydrofluoride,

The reacting is preferably performed in the presence of a non-polar organic solvent and optionally and preferably comprises sonication of the reaction mixture.

According to still a further aspect of the present invention there is provided a process of preparing a compound having the general Formula II, the process comprising:
reacting a compound having the general Formula V: 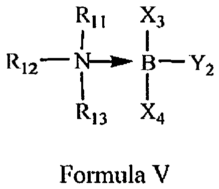 or a pharmaceutically acceptable salt thereof,
   wherein:
   Y₂ is selected from the group consisting of a cyano group -(C≡N), a -C(=O) Ra group, amine and alkyl, whereas Ra is hydrogen, halogen, hydroxy, alkoxy, thiohydroxy, thioalkoxy, aryloxy, thioaryloxy, thiol and amine;
   X₃ and X₄ are each independently selected from the group consisting of hydrogen, alkyl, halogen, cycloalkyl and aryl; and
   R₁₁-R₁₃ are each independently selected from the group consisting of hydrogen, alkyl, cycloalkyl and aryl or, alternatively, two of R₁₁-R₁₃ form a carbocyclic ring, provided that at least one of R₁₁-R₁₃ in Formula V is methyl,
   with a compound having the general Formula VI:

      W₁-A-W₂ Formula VI

      wherein:
      A is a substituted or non-substituted, saturated or non-saturated hydrocarbon having from 5 to 20 carbon atoms; and
      W₁ and W₂ are each independently a functional group,
   in the presence of n-alkyl lithium, thereby obtaining the compound having general Formula II.

According to yet a further aspect of the present invention there is provided a process of preparing a compound having Formula III, wherein at least one of R₈-R₁₀ is a saturated alkyl having 11-15 carbon atoms or an unsaturated alkyl having 5-2-carbon atoms, the process comprising:
reacting a compound having the general Formula V: 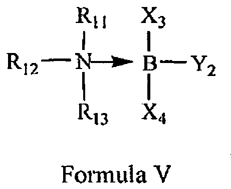 or a pharmaceutically acceptable salt thereof,
   wherein:
   Y₂ is selected from the group consisting of a cyano group -(C≡N), a -C(=O) Ra group, amine and alkyl, whereas Ra is hydrogen, halogen, hydroxy, alkoxy, thiohydroxy, thioalkoxy, aryloxy, thioaryloxy, thiol and amine;
   X₃ and X₄ are each independently selected from the group consisting of hydrogen, alkyl, halogen, cycloalkyl and aryl; and
   R₁₁-R₁₃ are each independently selected from the group consisting of hydrogen, alkyl, cycloalkyl and aryl or, alternatively, two of R₁₁-R₁₃ form a carbocyclic ring, provided that at least one of R₁₁-R₁₃ in Formula V is methyl,
   with a compound having the general Formula VII:

      R₁₄-W₃ Formula VII

      wherein:
      R₁₄ is selected from the group consisting of saturated alkyl having at least 10 carbon atoms or an unsaturated alkyl having at least 5 carbon atoms; and
      W₃ is a functional group,
   in the presence of n-alkyl lithium, thereby obtaining the compound having general Formula III.

According to another aspect of the present invention there is provided a process of preparing a compound having Formula III, wherein at least of R₈-R₁₀ comprises a fluorine, the process comprising:
reacting a compound having the general Formula V: 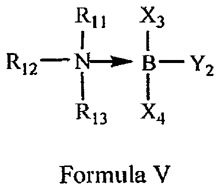 or a pharmaceutically acceptable salt thereof,
   wherein:
   Y₂ is selected from the group consisting of a cyano group -(C≡N), a -C(=O) Ra group, amine and alkyl, whereas Ra is hydrogen, halogen, hydroxy, alkoxy, thiohydroxy, thioalkoxy, aryloxy, thioaryloxy, thiol and amine;
   X₃ and X₄ are each independently selected from the group consisting of hydrogen, alkyl, halogen, cycloalkyl and aryl; and
   R₁₁-R₁₃ are each independently selected from the group consisting of hydrogen, alkyl, cycloalkyl and aryl or, alternatively, two of R₁₁-R₁₃ form a carbocyclic ring, provided that at least one of R₁₁-R₁₃ in Formula V is methyl,
   with a compound having the general Formula VIII:

      R₁₅-W₄-R₁₆ Formula VIII

      wherein:
      W₄ is a carboxy (C=O) group;
      R₁₅ is selected from the group consisting of alkyl, cycloalkyl, and aryl; and
      R₁₆ is selected from the group consisting of hydrogen, alkyl, cycloalkyl and aryl,
   in the presence of n-alkyl lithium, to thereby obtaining a compound having the general Formula III, wherein at least one of R₈-R₁₀ comprises a hydroxy or alkoxy; and
   converting the hydroxy or the alkoxy to the fluorine.

Preferably, the converting comprises reacting the compound having the general Formula III and at least of R₈-R₁₀ being a hydroxy or alkoxy, with a fluorinating agent. Exemplary fluorinating agents include triflic anhydride, 2,6-lutidine and cesium fluoride (CsF). Optionally, the fluorinating agent comprises a radioactive fluorine, and most preferably fluorine-18 [¹⁸F].

According to an additional aspect of the present invention there is provided a radiolabeled compound having the general Formula X or XI: 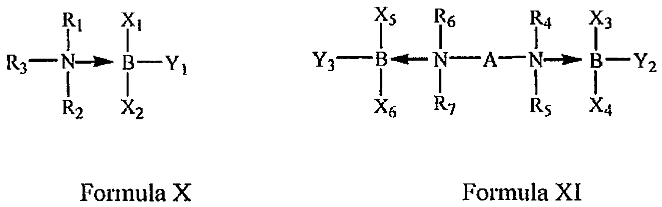 or a pharmaceutically acceptable salt thereof,
wherein:
Y₁-Y₃ are each independently selected from the group consisting of a cyano group (-C≡N), a -C(=O) Ra group, amine and alkyl, whereas Ra is hydrogen, halogen, hydroxy, alkoxy, thiohydroxy, thioalkoxy, aryloxy, thioaryloxy, thiol and amine;
X₁-X₆ are each independently selected from the group consisting of hydrogen, alkyl, halogen, cycloalkyl and aryl,
R₁-R₇ are each independently selected from the group consisting of hydrogen, alkyl, cycloalkyl and aryl or, alternatively, two of R₁-R₃, R₄ and R₅ and/or R₆ and R₇ form a carbocyclic ring, and
A is a substituted or non-substituted, saturated or non-saturated hydrocarbon having from 5 to 20 carbon atoms,
whereas at least one of X₁-X₆, R₁-R₇ and A comprises a radioactive atom and/or at least boron atom is a radioactive boron atom.

According to further features in preferred embodiments of the invention described below, the radioactive atom is selected from the group consisting of radioactive fluorine, radioactive carbon, radioactive bromine and radioactive iodine.

According to still further features in the described preferred embodiments at least one of X₁-X₆ is the radioactive atom.

Preferably the radioactive atom is radioactive fluorine, and more preferably the radioactive fluorine is fluorine-18 [¹⁸F].

According to still further features in the described preferred embodiments at least one of R₁-R₇ comprises the radioactive atom.

According to still further features in the described preferred embodiments the radioactive atom is a radioactive carbon.

According to still further features in the described preferred embodiments the radioactive atom is a selected from the group consisting of as radioactive fluorine, a radioactive bromine and a radioactive iodine. Preferably, such a radioactive atom is at position β to the amine nitrogen.

According to still further features in the described preferred embodiments the compound has the general Formula X.

According to still further features in the described preferred embodiments at least one of X₁ and X₂ is the radioactive atom.

According to still further features in the described preferred embodiments at least one of R₁-R₃ comprises a radioactive atom.

According to still further features in the described preferred embodiments the radioactive atom is a radioactive carbon.

According to still further features in the described preferred embodiments at least one of R₁-R₃ is an alkyl substituted by a radioactive atom.

According to still further features in the described preferred embodiments the radioactive atom is a selected from the group consisting of a radioactive fluorine, a radioactive bromine and a radioactive iodine. Such a radioactive atom is preferably at position β to the amine nitrogen.

According to still further features in the described preferred embodiments at least one of R₁-R₃ is an alkyl.

According to still further features in the described preferred embodiments each of R₁-R₃ is alkyl.

According to still further features in the described preferred embodiments the alkyl is selected from the group consisting of methyl, ethyl and n-butyl.

According to still further features in the described preferred embodiments at least one of R₁-R₃ is a C₅-C₂₀ alkyl.

According to still further features in the described preferred embodiments the alkyl is selected from the group consisting of a saturated alkyl and an unsaturated alkyl.

According to still further features in the described preferred embodiments Y₁ is selected from the group consisting of a cyano group -(C=N) and a -C(=O)Ra group.

According to still further features in the described preferred embodiments Ra is selected from the group consisting of hydroxy and alkoxy.

According to still further features in the described preferred embodiments Y₁ is selected from the group consisting of a cyano group -(C≡N) and a -C(=O)Ra group, wherein Ra is selected from the group consisting of hydroxy and alkoxy (e.g., methoxy and ethoxy).

According to still an additional aspect of the present invention there is provided a pharmaceutical composition comprising, as an active ingredient, any of the radiolabeled compounds described herein and a pharmaceutically acceptable carrier.

According to yet an additional aspect of the present invention there is provided a use of the radiolabeled compounds presented herein in radioimaging (e.g., positron emitting tomography (PET), Computed Tomography (CT), PET/CT and Single Photon Emission Tomography (SPECT)) and/or radiotherapy. In a preferred embodiment, the radiolabeled compounds are designed suitable for use in a radioimaging technique such as positron emitting tomography.

According to yet an additional aspect of the present invention there is provided a use of the radiolabeled compounds presented herein in the preparation of a diagnostic agent.

According to features in the described preferred embodiments the diagnostic agent is for use in a radioimaging technique selected from the group consisting of Positron Emission Tomography (PET), Computed Tomography (CT), PET/CT and Single Photon Emission Tomography (SPECT).

The present invention successfully addresses the shortcomings of the presently known configurations by providing novel classes of amine-borane compounds which possess unique and novel features that render these compounds superior to other amine-borane compounds known in the art and further enable to efficiently produce radiolabeled amine-borane compounds.

Unless otherwise defined, all technical and scientific terms used herein have the same meaning as commonly understood by one of ordinary skill in the art to which this invention belongs. Although methods and materials similar or equivalent to those described herein can be used in the practice or testing of the present invention, suitable methods and materials are described below. In case of conflict, the patent specification, including definitions, will control. In addition, the materials, methods, and examples are illustrative only and not intended to be limiting.

The term "comprising" means that other steps and ingredients that do not affect the final result can be added. This term encompasses the terms "consisting of" and "consisting essentially of".

The phrase "consisting essentially of" means that the composition or method may include additional ingredients and/or steps, but only if the additional ingredients and/or steps do not materially alter the basic and novel characteristics of the claimed composition or method.

As used herein, the singular form "a," "an," and "the" include plural references unless the context clearly dictates otherwise. For example, the term "a compound" or "at least one compound" may include a plurality of compounds, including mixtures thereof.

Throughout this disclosure, various aspects of this invention can be presented in a range format. It should be understood that the description in range format is merely for convenience and brevity and should not be construed as an inflexible limitation on the scope of the invention. Accordingly, the description of a range should be considered to have specifically disclosed all the possible subranges as well as individual numerical values within that range. For example, description of a range such as from 1 to 6 should be considered to have specifically disclosed subranges such as from 1 to 3, from 1 to 4, from 1 to 5, from 2 to 4, from 2 to 6, from 3 to 6 etc., as well as individual numbers within that range, for example, 1, 2, 3, 4, 5, and 6. This applies regardless of the breadth of the range.

Whenever a numerical range is indicated herein, it is meant to include any cited numeral (fractional or integral) within the indicated range. The phrases "ranging/ranges between" a first indicate number and a second indicate number and "ranging/ranges from" a first indicate number "to" a second indicate number are used herein interchangeably and are meant to include the first and second indicated numbers and all the fractional and integral numerals therebetween.

### BRIEF DESCRIPTION OF THE DRAWINGS

The invention is herein described, by way of example only, with reference to the accompanying drawings. With specific reference now to the drawings in detail, it is stressed that the particulars shown are by way of example and for purposes of illustrative discussion of the preferred embodiments of the present invention only, and are presented in the cause of providing what is believed to be the most useful and readily understood description of the principles and conceptual aspects of the invention. In this regard, no attempt is made to show structural details of the invention in more detail than is necessary for a fundamental understanding of the invention, the description taken with the drawings making apparent to those skilled in the art how the several forms of the invention may be embodied in practice.

In the drawings:
FIG. 1 presents a plot showing the antilishmenial effect of Compound **K-I** as determined by a dose-response assay against the *Leishmania donovani* strain;
FIG. 2 presents a plot showing the antimalarial effect of Compound **K-I** as determined by a dose-response assay against the *Plasmodium falciparum* strain;
FIG. 3 presents a schematic illustration of a semiautomatic radiolabeling synthesis system designed to safely and efficiently prepare ¹⁸F-labeled amine-borane compounds according to the present embodiments;
FIG. 4 presents a schematic illustration of a 25 ml platinum dish designed for and utilized in the semiautomatic radiolabeling synthesis of ⁸F-labeted amine-borane compounds according to the present embodiments; and
FIG. 5 presents a series of PET-images obtained for normal, female Sprague-Dawley rats (250-300 grams each) which were injected with an exemplary ¹⁸F-radiolabeled amine-borane compound according to the present embodiments, Compound **[¹⁸F]K-I₄** (4.5 mCi/ml), clearly showing the uptake of the ¹⁸F-radiolabeled amine-borane compound into the bones of the tested rats.

### DESCRIPTION OF THE PREFERRED EMBODIMENTS

The present invention is of novel families of amine-borane compounds, which can be beneficially used in the treatment of a variety of medical conditions and other applications. The present invention is further of novel, radiolabeled amine-borane compounds, which can be used in radioimaging and/or radiotherapy.

As discussed hereinabove, amine-boranes are promising compounds which have a great potential as therapeutic agents, mostly due to their similarity to biologically occurring molecules, such as amino acids, neurotransmitters, nucleosides, and nucleic acids. Amine-boranes are isoelectronic and isostructural analogs of such biomolecules and hence are capable of mimicking the biological activity thereof in the body. However, the use of the presently known amine-boranes as therapeutic agents is oftentimes limited by poor efficacy and inferior pharmacokinetic profile.

In a search for novel amine-borane compounds, the present inventors have designed and successfully prepared and practiced novel families of amine-borane compounds. The present inventors have uncovered that by altering the chemical structure of ainine-borane compounds and adding functionalities thereto, a spectrum of active, efficacious and non-toxic therapeutic agents are obtained.

As used herein, the phrase "amine-borane compounds", which is also referred to herein interchangeably as "aminoboranes", describes any compound that includes at least one boron atom that is substituted by one or more amine group(s), as is defined hereinunder.

While conceiving the present invention, it was envisioned that since halogenated biologically occurring molecules, an in particular naturally occurring organofluoride compounds, are metabolically active substances and are hence very intriguing, particularly in medicinal chemistry, fluorinated amine-borane compounds may provide an improved class of aminoborane compounds, which can be further utilized in radioimaging applications. It was further hypothesized that the activity and pharmacokinetic profile of amine-borane compounds can be improved by incorporating therein an elongated hydrocarbon chain (e.g., a long alkyl).

While reducing the present invention to practice, as is demonstrated in the Examples section that follows, the present inventors have successfully prepared novel families of amine cyanoboranes and amine carboxyboranes, as well as amine bis-boranes, primarily fluorinated and/or including a long hydrocarbon chain.

The present invention therefore provides amine-borane compounds and/or a pharmaceutically acceptable salt thereof, having the general Formula I, II or III: 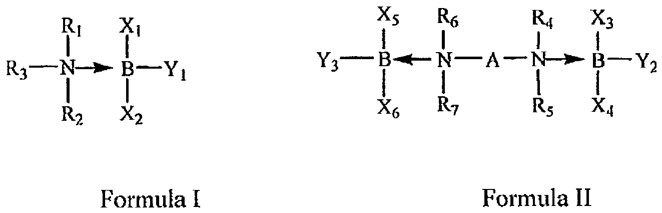 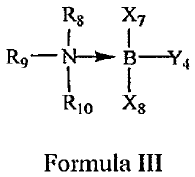 or a pharmaceutically acceptable salt thereof, wherein:
each of Y₁-Y₄ is independently a cyano group (-C≡N), a -C(=O)Ra group, amine or alkyl, whereas Ra is hydrogen, halogen, hydroxy, alkoxy, thiohydroxy, thioalkoxy, aryloxy, thioaryloxy, thiol and amine;
each of X₁-X₈ is independently hydrogen, alkyl, halogen, cycloalkyl, aryl, whereby one or more of X₁ and X₂ in Formula I is fluorine;
each of R₁-R₁₀ is independently hydrogen, alkyl, cycloalkyl and aryl or, alternatively, two of R₁-R₃, R₄ and R₅, R₆ and R₇ and/or R₈-R₁₀ form a carbocyclic ring, whereby one or more of R₈-R₁₀ in Formula III is a saturated alkyl having at 11-15 carbon atoms, one or more of R₈-R₁₀ in Formula III is an unsaturated alkyl having 5-20 carbon atoms and/or one or more of R₈-R₁₀ in Formula III is substituted by fluorine; and
A is a substituted or non-substituted, saturated or non-saturated hydrocarbon having from 5 to 20 carbon atoms.

As used herein, the term "halogen", which is also referred to herein as "halo" describes a radical of fluorine, chlorine, bromine or iodine.

As used herein, the term "hydroxy" describes an -OH group.

The term "alkoxy" describes a -OR group, where R is alkyl or cycloalkyl, as these terms are defined herein.

The term "thiohydroxy" or "thiol" refers to a -SH group.

The term "thioalkoxy" describes a -SR group, where R is alkyl or cycloalkyl.

The term "aryloxy" describes both an -O-aryl and an -O-heteroaryl group, as defined herein.

The term "thioaryloxy" describes both a -S-aryl and a -S-heteroaryl group, as defined herein.

As used herein, the term "amine" describes a -NR'R" group where each of R' and R" is independently hydrogen, alkyl, cycloalkyl or aryl, as these terms are defined herein.

As used herein, the term "alkyl" describes an aliphatic, and unsaturated (alkenyl and alkynyl) hydrocarbon including straight chain and branched chain groups. Preferably, the alkyl group has 1 to 20 carbon atoms. Whenever a numerical range; e.g., "1-20", is stated herein, it implies that the group, in this case the alkyl group, may contain 1 carbon atom, 2 carbon atoms, 3 carbon atoms, etc., up to and including 20 carbon atoms. The alkyl can be substituted or unsubstituted, the alkyl can further be saturated or unsaturated. This term therefore encompasses alkenyl and alkynyl.

The term "alkenyl" describes an unsaturated alkyl having at least two carbon atoms and at least one carbon-carbon double bond.

The term "alkynyl" an unsaturated alkyl having at least two carbon atoms and at least one carbon-carbon triple bond.

The term "cycloalkyl" describes an all-carbon monocyclic or fused ring (i.e., rings which share an adjacent pair of carbon atoms) group where one or more of the rings does not have a completely conjugated pi-electron system.

The term "aryl" describes an all-carbon monocyclic or fused-ring polycyclic (i.e., rings which share adjacent pairs of carbon atoms) groups having a completely conjugated pi-electron system.

The compounds disclosed herein, represented by Formulae I, II and III, possess unique and novel features, as discussed hereinbelow.

In preferred embodiments of the present invention, the novel families of aminoboranes disclosed herein include cyano amino boranes and carboxy amino boranes.

Thus, in each of Formulae I, II and III above, Y₁ is a cyano group (-C≡N) being attached to the boron atom, or a carboxy group, -C(=O)Ra, being attached to the boron atom.

The Ra group may be a hydrogen, defining the -C(=O)Ra group as an aldehyde; an alkoxy or aryloxy group, defining the -C(=O)Ra group as an ester; a halo, defining the -C(=O)Ra group as an acyl halide; a hydroxyl, defining the - C(=O)Ra group as a carboxylic acid; a thiohydroxy (thiol), defining the -C(=O)Ra group as a thiocarboxylic acid; a thioalkoxy or thioaryloxy, defining the -C(=O)Ra group as a thioester; and an amine, defining the -C(=O)Ra group as an amide. Preferably Ra is a hydroxyl or alkoxy, and preferably the alkoxy is methoxy group or ethoxy group.

In additional preferred embodiments of the present invention, the other two substituents on the boron atom, denoted as X₁-X₈ in Formulae I, II and III, are hydrogen or halogen, preferably bromine or fluorine.

In still additional preferred embodiments of the present invention, at least one of X₁-X₈ is fluorine.

While reducing the present invention to practice, the present inventors have surprisingly uncovered that the presence of a fluorine atom in aminoborane compounds imparts to the compounds unique and novel features.

The improved activity of fluorinated aminoboranes is discussed below and is demonstrated in the Examples section that follows, where the activity of various structurally diverse aminoboranes was tested against various microorganisms. These studies, as well as the beneficial effect of the incorporation of a fluorine atom, are further discussed in detail in U.S. Provisional Patent Application No. 60/716,082, filed September 13, 2005, and in a PCT International Patent Application, by the present assignee, having Attorney Docket No. 32561 and entitled "use of amine-borane compounds as anti-microbial agents", which is cofiled on the same date as the instant application.

In particular preferred embodiments of the present invention, the amine-borane compound has the general Formula I above, and one or both of X₁ and X₂ is/are fluorine. Such compounds represent a novel class of aminoboranes.

Preferred compounds having general Formula I and one or more fluorine substituents on the boron atom are those in which at least one and preferably all of R₁, R₂ and R₃ in Formula I, is a saturated or unsaturated low alkyl. Representative examples include compounds in which each of R₁, R₂ and R₃ is a low alkyl such as methyl, ethyl and n-butyl.

Additionally preferred compounds in this class are those in which one or more of R₁, R₂ and R₃ is a saturated and unsaturated high alkyl group having from 5 to 20 carbons.

As is demonstrated in the Examples section that follows, the present inventors have designed and successfully prepared and practiced several exemplary members of this class of compounds. These include, for example, dimethyl-undecyl-amine cyanofluorobromoborane, trimethyl-amine cyanofluoroborane, ethyl-dimethyl-amine cyanofluoroborane, butyl-dimethyl-amine cyanofluoroborane, trimethyl-amine carboxyfluoroborane methyl ester, trimethyl-amine carboxyfluoroborane ethyl ester, ethyl-dimethyl-amine carboxyfluoroborane methyl ester, butyl-dimethyl-amine carboxyfluoroborane methyl ester, trimethyl-amine cyanodifluoroborane, trimethyl-amine carboxydifluoroborane methyl ester, trimethyl-amine carboxydifluoroborane ethyl ester, trimethyl-amine cyanofluorobromoborane, trimethyl-amine carboxyfluorobromoborane ethyl ester and triethyl-amine carboxydifluoroborane.

While further reducing the present invention to practice, the present inventors have uncovered that the incorporation of a long N-alkyl chain to amine-borane compounds further improves the activity thereof. The present inventors have conducted a structure-activity relation studies and have uncovered that the length of such an alkyl chain strongly affects the activity of the compounds. Therefore, the preferred chain length for one or more of R₁-R₃ or R₈-R₁₀ the compounds having the general Formulae I and III, according to the present invention, and particularly to one or more of R₈-R₁₀ in Formula III, is between eleven and fifteen carbon-long chain, and more preferred is a fifteen long (C₁₅) chain, as demonstrated for Compound **K-R** (see, Table 2 hereinbelow).

While further reducing the present invention to practice, it was found that the presence of an unsaturated C=C double bond in an alkyl substitution in one or more of R₁-R₁₀ in Formulae I, II and III and particularly in one or more of R₈-R₁₀ in Formula III, preferably at the terminal position of the unsaturated alkyl group, dramatically enhanced the biologic activity of the amine-borane compound. This finding is demonstrated in the Examples section that follows in Example 2 as presented for Compound **K-D** and Compound **K-V**. Therefore one or more of R₈-R₁₀ in Formula III is preferably an unsaturated alkyl group having 5-20 carbon atoms.

While further reducing the present invention to practice, the present inventors have prepared and tested the efficacy of a number of the above-described diamine bis-cyanoboranes belonging to Formula II, as presented hereinbelow in the Examples section that follows. In general, the efficacy results for these compounds indicated that the compounds having a longer N-alkyl-N chain exhibited higher bioactivity activity. Therefore, the preferred length of A in Formula II (the N-alkyl-N chain), according to the present invention, is a fourteen carbon-long chain (C₁₄), as demonstrated for Compound **K-N** (see, Table 2 hereinbelow).

Thus, according to additional preferred embodiments of the present invention, the amine-borane compound has the general Formula II. Such amine-borane compounds constitutes another class of novel compounds which have a bis-amine-borane functionality, namely, have two amine-borane functionalities, each of which is defined similarly to the amine-borane functionality described hereinabove, whereby the moiety linking these functionalities is a hydrocarbon chain being at least 5 carbons in length.

As is demonstrated in the Examples section that follows, the present inventors have designed and successfully prepared several exemplary members of this class of compounds. These include, for example, N,N,N',N'-tetramethyl-decane-1,10-diamine bis-cyanoborane, N,N,N',N'-tetramethyl-decane-1,10-diamine bis-cyanobromoborane, N,N,N',N'-tetramethyl-decane-1,10-diamine bis-cyanodibromoborane, N,N,N',N'-tetramethyl-decane-1,10-diamine bis-carboxyboranes, N,N,N',N'-tetramethyl-dodecane-1,12-diamine bis-cyanoborane and N,N,N',N'-tetramethyl-tetradecane-1,14-diamine bis-cyanoborane.

According to still additional preferred embodiments of the present invention, the amine-borane compounds have the general Formula III. Such compounds constitutes a novel class of compounds which have a unique and novel feature being primarily the saturated and unsaturated high alkyl group having from 5 to 20 carbons at one or more of R₈-R₁₀, and secondarily may have a substituent at position β to the amine nitrogen on one or more of R₈-R₁₀.

Preferably, the substituent at position β to the amine nitrogen is a hydroxyl or a fluorine; the latter constitutes a unique and novel feature of this group of amine-borane compounds.

The present inventors have designed and successfully prepared several exemplary members of this class of compounds, including, for example, dimethyl-undecyl-amine cyanoborane, dimethyl-undecyl-amine cyanobromoborane, dimethyl-undecyl-amine cyanodibromoborane, dodecyl-dimethyl-amine cyanoborane, 1-dimethylamino-2-methyl-octan-2-ol cyanoborane, dimethyl-nonyl-amine cyanoborane, dimethyl-tridecyl-amine cyanoborane, dimethyl-pentadecyl-amine cyanoborane, heptadecyl-dimethyl-amine cyanoborane, 1-dimethylamino-dodecan-2-ol cyanoborane, 1-dimethylamino-undecan-2-ol cyanoborane, dimethyl-undecyl-amine cyanofluorobromoborane and (2-fluoro-nonyl)-dimethyl-amine cyanoborane.

While further conceiving the present invention, it was envisioned that these novel fluorinated amine-borane compounds, which may possess similar or improved activities as compared with their non-fluorinated counterparts, will offer the possibility of introducing a detectible isotope thereto in the form of ¹⁸F (fluorine-18), thus rendering novel radiolabeled amine-borane compounds.

Therefore, as is further detailed hereinbelow, according to an embodiment of the present invention, an ¹⁸F isotope can replace any or all of the fluorine atoms in each and every one of the compounds having the Formulae I, II and III, as described hereinabove.

While further reducing the present invention to practice, a series of structurally diverse amine-borane compounds according to the present invention were prepared and assayed for antimicrobial activity, as demonstrated in the Examples section that follows. These assays have clearly shown that the compounds presented herein exhibit exceptional and selective antimicrobial activity, mostly in a dose-dependent manner, as compared to amine-borane compounds taught in the art and to conventionally used antifungal agents and drugs.

The compounds according to the present invention were tested for antifungal activity against pathogenic and antibiotic-resistant strains. A number of relationships were observed according to their structural type. The following modifications in the structure significantly enhanced the activity: (i) the N-alkyl chain length in alkyldimethylamine cyanoboranes and diamine bis-cyanoboranes, (ii) halogenation, (iii) the incorporation of the C=C double bond at the end of the N-alkyl chain, and (iv) the conversion of the amine cyanoborane to the amine carboxyborane. However, no significant enhancement of activity was observed with other modifications in the structure, such as addition of a hydroxyl group, an aromatic group, an unsaturated cyclic group, and a silyl group. As presented in details in the Examples section that follows, in Example 2, the most active compounds were dimethylundecylamine cyanoborane (C₁₁H₂₃N(CH₃)₂BH₂CN) Compound **K-I**, with MIC values for the most important human pathogenic fungi ranging from 16.25 to 32.5 µmol per liter, and its dibromo derivative, dimethylundecylamine dibromocyanoborane (C₁₁H₂₃N(CH₃)₂BBr₂CN) Compound **K-I₂**, in which the MIC values ranged from 10.05 to 79 µmol per liter. These compounds possess considerable activity against fungal strains which are resistant to fluconazole. Anti-leishmanial and anti-malarial, as well as reduced toxicity was also observed with these compounds.

Particularly the amine-borane compounds presented herein were found highly effective against several highly virulent fungi such as *Aspergillus fumigatus*.

More particularly the compounds of the present invention were shown to have high antifungal antimicrobial active against drug-resistant fungi strains, such as the highly malicious *Candida glabrafa* and *Candida krusei* which are known to be resistant to fluconazole, a conventional and widely used antifungal drug, as demonstrated in the Examples section that follows.

*Candida glabrata*, *Candida krusei* and *Aspergillus fumigatus* have been recognized as highly pernicious pathogens, and are considered as the one of the most frequent fatal factor among the ever increasing population of immunocompromised individuals. Recent studies have shown *Candida glabrata* and *Candida krusei* to be highly opportunistic pathogens of the urogenital tract, and of the bloodstream (Candidemia) which is especially prevalent in HIV positive people, and the elderly.

Resistance of microorganism to antimicrobial agents is the ability of a microorganism to withstand the antimicrobial effects of any given agents (antibiotics). The antimicrobial action of any given agent is putting an environmental pressure on the target (and also non-targeted) microorganisms. The microorganisms which have a mutation that will allow it to survive will live on to reproduce. These newly evolved strain(s) will then pass this trait to their offspring, which will constitute a fully resistant generation. Resistance can develop naturally via natural selection through random mutation and programmed evolution governed by low-fidelity polymerases which can cause a higher rate of random mutations in the microorganism genetic code. Once such a gene is generated, the microorganism may also transfer the genetic information in a horizontal fashion, namely between individuals, via plasmid exchange, hence resistance is a consequence of evolution via natural selection or programmed evolution.

It has been demonstrated that habitude of antimicrobial agents usage greatly affects the number of resistant organisms which develop. For example, overuse of broad-spectrum antibiotics of low specificity, such as second- and third-generation cephalosporins and fungicides such as fluconazole, greatly accelerated the development of methicillin or fluconazole resistance, even in organisms that have never been exposed to the selective pressure of methicillin or fluconazole *per se*. Other factors contributing to the ever growing emergence of resistance to antimicrobials include incorrect diagnosis, unnecessary prescriptions, improper use of antimicrobials by patients, and the use of antimicrobials in livestock food as additives for growth promotion.

As used herein the term "method" refers to manners, means, techniques and procedures for accomplishing a given task including, but not limited to, those manners, means, techniques and procedures either known to, or readily developed from known manners, means, techniques and procedures by practitioners of the chemical, pharmacological, biological, biochemical and medical arts.

As used herein, the term "preventing" includes barring an organism from acquiring a condition in the first place.

As used herein, the terms "treating" and "treatment" includes abrogating, substantially inhibiting, slowing or reversing the progression of a condition, substantially ameliorating clinical or aesthetical symptoms of a condition or substantially preventing the appearance of clinical or aesthetical symptoms of a condition.

As used herein, the phrase "therapeutically effective amount" describes an amount of the compound being administered which will relieve to some extent one or more of the symptoms of the condition being treated.

According to embodiments of this aspect of the present invention, the medical condition associated with a pathogenic microorganisms include bacterial infections, protozoan infections and a fungal infection, and preferably the medical conditions wherein use of the amine-borane of the present invention is beneficial are malaria and leishmaniasis, as demonstrated and exemplified in the Examples section that follows.

As used herein, the phrase "pathogenic microorganism" describes any microorganism which can cause a disease or infection in a higher organism, such as any animals grown for commercial or recreational purposes, fish, poultry, insects (e.g., bees) and mammals. In particular, the pathogenic microorganisms maybe those which cause diseases and adverse effects in humans. The pathogenic microorganism may belong to any family of organisms such as, but not limited to, prokaryotic organisms, eubacterium, archaebacterium, eukaryotic organisms, yeast, fungi, algae, protozoa, and other parasites.

As used herein the term "associated" in the context of the present invention means that at least one adverse manifestation of the medical condition is caused by a pathogenic microorganism. The phrase "medical condition associated with a pathogenic microorganism" therefore encompasses medical conditions of which the microorganism may be the primary cause of the medical condition or a secondary effect of the main medical condition(s).

Medical conditions associated with a pathogenic microorganism include infections, infestation, contaminations and transmissions by or of pathogenic microorganisms such as those described herein. In general, a disease causing infection is the invasion into the tissues of a plant or an animal by pathogenic microorganisms. The invasion of body tissues by parasitic worms and other higher pathogenic organisms such as lice is oftentimes referred to as infestation.

According to a preferred embodiment of this aspect of the present invention, the pathogenic microorganism is a drug-resistant pathogenic microorganism, as discussed hereinabove.

Acute toxicity assays were conducted for an exemplary compound according to the present invention using rats as an animal model. As demonstrated in the Examples section that follows, the amine-borane compounds presented herein have been shown to be non-toxic at the effective concentration range thereof and higher.

In any of the above aspects of the present invention, the amine-borane compounds of the present invention can be utilized either *per se* or, preferably, as a part of a pharmaceutical composition that further comprises a pharmaceutically acceptable carrier.

Thus, according to additional aspects of the present invention, there are provided pharmaceutical compositions, which are identified for use in the treatment of any of the medical conditions listed above, and which comprise one or more amine-borane compounds and a pharmaceutically acceptable carrier.

In addition there is provided a use of the compounds presented herein in the preparation of a medicament. The medicament can be used to treat any of the conditions known in the art to be treatable by amine-borane as discussed hereinabove, and particularly those associated with pathogenic microorganisms.

As used herein a "pharmaceutical composition" refers to a preparation of amine-borane compounds, with other chemical components such as pharmaceutically acceptable and suitable carriers and excipients. The purpose of a pharmaceutical composition is to facilitate administration of a compound to an organism.

Hereinafter, the term "pharmaceutically acceptable carrier" refers to a carrier or a diluent that does not cause significant irritation to an organism and does not abrogate the biological activity and properties of the administered compound. Examples, without limitations, of carriers are: propylene glycol, saline, emulsions and mixtures of organic solvents with water, as well as solid (e.g., powdered) and gaseous carriers.

As used herein, the term "carrier" refers to a diluent, adjuvant, excipient, or vehicle with which the therapeutic is administered.

Herein the term "excipient" refers to an inert substance added to a pharmaceutical composition to further facilitate administration of a compound. Examples, without limitation, of excipients include calcium carbonate, calcium phosphate, various sugars and types of starch, cellulose derivatives, gelatin, vegetable oils and polyethylene glycols.

Techniques for formulation and administration of drugs may be found in "Remington's Pharmaceutical Sciences" Mack Publishing Co., Easton, PA, latest edition.

Pharmaceutical compositions for use in accordance with the present invention thus may be formulated in conventional manner using one or more pharmaceutically acceptable carriers comprising excipients and auxiliaries, which facilitate processing of the silver-coated enzymes into preparations which, can be used pharmaceutically. Proper formulation is dependent upon the route of administration chosen. The dosage may vary depending upon the dosage form employed and the route of administration utilized. The exact formulation, route of administration and dosage can be chosen by the individual physician in view of the patient's condition. (See e.g., Fingl et al., 1975, in "The Pharmacological Basis of Therapeutics", Ch. 1 p.1).

The pharmaceutical composition may be formulated for administration in either one or more of routes depending on whether local or systemic treatment or administration is of choice, and on the area to be treated. Administration may be done topically (including ophtalmically, vaginally, rectally, intranasally), orally, by inhalation, or parenterally, for example by intravenous drip or intraperitoneal, subcutaneous, intramuscular or intravenous injection.

Formulations for topical administration may include but are not limited to lotions, ointments, gels, creams, suppositories, drops, liquids, sprays and powders. Conventional pharmaceutical carriers, aqueous, powder or oily bases, thickeners and the like may be necessary or desirable.

Compositions for oral administration include powders or granules, suspensions or solutions in water or non-aqueous media, sachets, capsules or tablets. Thickeners, diluents, flavorings, dispersing aids, emulsifiers or binders may be desirable.

Formulations for parenteral administration may include, but are not limited to, sterile solutions which may also contain buffers, diluents and other suitable additives. Slow release compositions are envisaged for treatment.

The amount of a composition to be administered will, of course, be dependent on the subject being treated, the severity of the affliction, the manner of administration, the judgment of the prescribing physician, etc.

Compositions of the present invention may, if desired, be presented in a pack or dispenser device, such as an FDA (the U.S. Food and Drug Administration) approved kit, which may contain one or more unit dosage forms containing the active ingredient. The pack may, for example, comprise metal or plastic foil, such as, but not limited to a blister pack or a pressurized container (for inhalation). The pack or dispenser device may be accompanied by instructions for administration. The pack or dispenser may also be accompanied by a notice associated with the container in a form prescribed by a governmental agency regulating the manufacture, use or sale of pharmaceuticals, which notice is reflective of approval by the agency of the form of the compositions for human or veterinary Administration. Such notice, for example, may be of labeling approved by the U.S. Food and Drug Administration for prescription drugs or of an approved product insert. Compositions comprising a silver-coated enzyme of the invention formulated in a compatible pharmaceutical carrier may also be prepared, placed in an appropriate container, and labeled for treatment of an indicated condition or diagnosis, as is detailed hereinabove.

Thus, according to an embodiment of the present invention, depending on the selected amine-borane compound, the pharmaceutical compositions of the present invention are packaged in a packaging material and identified in print, in or on the packaging material, for use in the treatment of a medical condition associated with a pathogenic microorganism, as is defined hereinabove.

The present invention further encompasses processes of preparing any of the compounds disclosed herein, as is described hereinbelow and is further detailed in the Examples section that follows.

Hence, according to another aspect of the present invention there is provided a process of preparing the compounds having the general Formula I presented above. The process is generally effected by providing a corresponding brominated aminoborane, having one or more bromines on the boron, and converting either one or both bromines into fluorines, via reaction with a fluorinating agent. While some of the brominated starting materials are known, some have been specifically designed for use in the context of the present invention. In general, brominated amine-borane compounds are prepared using known procedures, as indicated in the Examples section that follows.

The process according to this aspect of the present invention is therefore effected by:
reacting, with a fluorinating agent, a brominated compound having the general Formula IV: 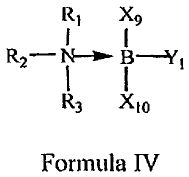 or a pharmaceutically acceptable salt thereof, wherein Y₁ is a cyano group, a -C(=O) Ra group, amine or alkyl, whereas Ra is as defined hereinabove; each of X₉ and X₁₀ is hydrogen, alkyl, halogen, cycloalkyl, or aryl, provided that at least one of X₉ and X₁₀ is bromine; and each of R₁-R₃ are is as defined hereinabove for Formula I.

Basically, this process takes advantage of a trait of the bromo substituent on the boron atom to act as a good leaving group, and be replaced by a fluorine. The bromo substituent is more prone to such a substitution reaction than, for example, a hydrogen or an alkyl substituent, hence the same process can be utilized to replace a single bromo or two bromo substituents on the boron atom. Thus the process of preparing this group of compounds is essentially a bromo-to-fluoro exchange process.

Thus, in one particular, the starting compound can be a compound having Formula IV, wherein one of X₉ and X₁₀ is bromine. This compound can be obtained by reacting a non-halogenated amine-borane compound with 1 equivalent of a brominating agent, and is then reacted with a fluorinating agent, to provide the desired compounds having Formula I. Alternatively, the starting can be a compound having Formulae IV, wherein both X₉ and X₁₀ are bromine. This compound can be obtained by reacting a non-halogenated amine-borane compound with 2 equivalents of a brominating agent, and is then reacted with a fluorinating agent, to provide the desired compound having Formula I.

This bromo-to-fluoro exchange process can be effected by using any fluorinating agent(s) as these are known to a person skilled in the art. Representative examples of fluorinating agents that have been shown suitable for use in the context of the present invention include, without limitation, silver fluoride and a mixture of triethylamine and trihydrofluoride.

According to preferred embodiments, the process according to this aspect of the present invention is performed in an organic solvent and preferably in a non-polar organic solvent such as toluene or benzene. In order to accelerate the reaction, the process is preferably performed while subjecting the reaction mixture to sonication.

As described hereinabove, the compounds described herein can comprise a fluorine which is either radioactive or non-radioactive. Compounds having general Formula I and a radioactive fluorine can be readily prepared using the process descried herein, by performing the fluorination reaction with a fluorinating agent that comprises a radioactive fluorine (e.g., a ¹⁸F isotope). Exemplary fluorinating agents in this respect include, for example, K¹⁸F, H¹⁸F and Ag¹⁸F. The preparation and use of the ¹⁸F-radiolabeled amine-borane compounds presented herein is further discussed hereinbelow.

According to another aspect of the present invention, there is provided a process of preparing the amine bis-borane compounds having the general Formula II presented above. The process, according to this aspect of the present invention, is effected by:
reacting a compound having the general Formula V: 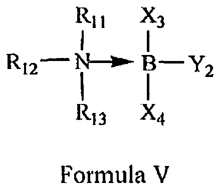
or a pharmaceutically acceptable salt thereof, wherein Y₂ is as defined hereinabove; X₃ and X₄ are is as defined hereinabove; and R₁₁-R₁₃ are each independently hydrogen, alkyl, cycloalkyl or aryl or, alternatively, two of R₁₁-R₁₃ form a carbocyclic ring, provided that at least one of R₁₁-R₁₃ in Formula V is methyl,
with a compound having the general Formula VI:

   W₁-A-W₂ Formula VI

   wherein:
   A is a substituted or non-substituted, saturated or non-saturated hydrocarbon having from 5 to 20 carbon atoms; and W₁ and W₂ are each independently a functional group;
   in the presence of n-alkyl lithium..

As used herein, the phrase "functional group" describes a chemical group that enables the occurrence of a chemical reaction that typically leads to a bond formation. The bond, according to the present invention, is preferably a covalent bond. Chemical reactions that lead to a bond formation include, for example, nucleophilic and electrophilic substitutions, nucleophilic and electrophilic addition reactions, addition-elimination reactions, cycloaddition reactions, rearrangement reactions and any other known organic reactions that involve a functional group.

Herein, the functional group is preferably a leaving group. As used herein, the phrase "leaving group" describes a labile group that readily undergoes detachment from an organic molecule during a chemical reaction, while the detachment is facilitated by the relative stability of the leaving atom thereafter. Typically, any group that is the conjugate base of a strong acid can acts as a leaving group. Preferably, the leaving group is a halo, and most preferably it is bromo.

Thus, according to preferred embodiments of the process according to this aspect of the present invention, the n-alkyl lithium acts as a strong base, which converts the methyl substituting the amine in the starting compound into an anion.

In one particular, two equivalents of such an anion react with a compound having Formula VI, replacing the functional leaving groups W₁ and W₂, to thereby form the bis-borane compound. Alternatively, the same process can be performed in steps, so as to allow the sequential attachment of two different units of the starting amine-borane (having Formula V) to the hydrocarbon moiety in the compound having Formula VI. Thus, for example, a compound having Formula VI can have a protected or inactivated functional group at one end. An activated (ionized) amine-borane compound reacts with the functional end of the compound of Formula VI and thereafter the inactivated functional group is activated, so as to react with another activated amine-borane, so as to form the amine bis-borane compound having Formula II.

Each of the amine-borane compounds used in this process as a starting material, having Formula V above, can be either prepared by known methods or can be one of the novel compounds disclosed herein.

As the above process is basically an alkylating process for one or more of the substituents on the amine, a similar process can be effected to prepare novel amine-borane compounds having a high alkyl substituent.

Hence, according to yet another aspect of the present invention, there is provided a process of preparing the compound having the general Formula III presented above, wherein at least of R₈-R₁₀ is a saturated alkyl having 11-15 carbon atoms or an unsaturated alkyl having 5-20 carbon atoms. The process, according to this aspect of the present invention, is effected by:
reacting a compound having the general Formula V: 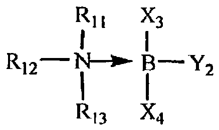 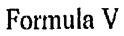 or a pharmaceutically acceptable salt thereof, wherein Y₂ is as defined hereinabove; X₃ and X₄ are as defined hereinabove; and R₁₁-R₁₃ are as defined hereinabove, provided that at least one of R₁₁-R₁₃ in Formula V is methyl,
   with a compound having the general Formula VII:

   R₁₄-W₃ Formula VII

   wherein R₁₄ is selected from the group consisting of saturated alkyl having at least 10 carbon atoms or an unsaturated alkyl having at least 4 carbon atoms; and W₃ is a functional group, as this term is defined hereinabove,
   in the presence of n-alkyl lithium.

The methodology used in this process is similar to that described above with respect to the preparation of compounds having the general Formula II. Such a methodology can be practiced with both saturated and unsaturated high alkyls.

According to yet another aspect of the present invention, there is provided a process of preparing the compound having the general Formula III presented above, wherein at least of R₈-R₁₀ includes hydroxy, alkoxy or fluorine. The process according to this aspect of the present invention is effected by:
reacting a compound having the general Formula V: 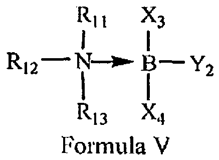 or a pharmaceutically acceptable salt thereof, wherein Y₂ is as defined hereinabove; X₃ and X₄ are as defined hereinabove; and R₁₁-R₁₃ are as defined hereinabove,
   with a compound having the general Formula VIII:

   R₁₅-W₄-R₁₆ Formula VIII

   wherein W₄ is a carboxy (C=O) group; R₁₅ is selected from the group consisting of alkyl, cycloalkyl, and aryl; and R₁₆ is selected from the group consisting of hydrogen, alkyl, cycloalkyl and aryl, as these terms are defined hereinabove,
   in the presence of n-alkyl lithium, so as to obtain a compound having general Formula III, wherein at least one of R₈-R₁₀ comprises a hydroxy or alkoxy.

The methodology used in this process is similar to that described above with respect to compounds having general Formula II or III. However, when an activated compound having Formula V is reacted with a compound having a carboxy group, as in Formula VIII, the product resulting from the addition reaction has an hydroxy or alkoxy functionality, depending on the chemistry of the reacting compound (having Formula VIII).

In a preferred embodiment of this aspect of the present invention, in the compound having Formula V, at least one of R₈-R₁₀ is methyl, such that upon reacting with a compound having Formula VIII, a hydroxy or alkoxy groups at position β to the amine are obtained.

The introduction of a hydroxyl or alkoxy group into the amine-borane compounds may alter the aqueous solubility of these compounds and may be used to change and improve their bioavailability in physiological condition.

The formation of a hydroxy or an alkoxy group β to the amine nitrogen also opens the path incorporation of a fluorine, either radioactive or non-radioactive, within the amine-borane compound, by substitution of the alkoxy or hydroxy groups.

Thus, the process according to this aspect of the present invention is further effected by converting the hydroxy or alkoxy substituent to fluorine. Such a conversion is preferably effected by reacting the compound having the hydroxy or alkoxy substituents with a fluorinating agent.

A representative example of a fluorinating agent that is suitable for use in this context of the present invention comprises a mixture of triflic anhydride and 2,6-lutidine, used to activate the hydroxy or alkoxy groups, followed by cesium fluoride (CsF).

As mentioned above, the introduction of a radioactive isotope of fluorine into the amine-borane compounds of the present invention would produce a radiolabeled compounds which can be used in a variety of application such as, for example, nuclear medicine, imaging and diagnostics.

Biologically active compounds which contain one or more fluorine atoms are commonly used in medical imaging techniques such as, for example, positron emission tomography (PET). Typically, a fluorine atom in these compounds is replaced with the positron emitting radioactive isotope fluorine-18 to produce ¹⁸F-radiolabeled compound. After injection into a patient, a PET scanner can form images of the distribution of ¹⁸F-labeled compound in the body. The images can be assessed by a radiologist to provide diagnoses of various medical conditions and investigate the tissues which absorb a given radiolabeled compound.

PET is a nuclear imaging modality which allows for the four-dimensional, quantitative determination of the distribution of radioactivity within the human body. This imaging modality allows performing accurate measurements of radioactivity concentrations in small volume compartments in vivo, as well as the ability to follow the kinetics of the radiolabeled biomarker.

According to the present invention, amine-borane compounds containing one or more ¹⁸F atoms can be prepared by similar synthetic routes as presented above for non-radiolabeled compounds, by using a ¹⁸F-radiolabeled starting material, or, preferably, employ a step which will substitute one or more non-radioactive atom(s) with radioactive atoms during, or at the end, of the synthesis.

Preferably the compounds of the present invention can be radiolabeled with radioactive halogens such as ¹⁸F (t_{½}, 1.83 hours), ¹²³I (t_{½}, 13 hours), ¹²⁵I (t_{½} 60 days) ¹³¹I (t_{½} 8 days), ⁷⁷Br (t_{½}, 2.4 days) and the likes.

According to the present invention, amine-borane compounds containing one or more ¹⁸F atoms may be used effectively in radioimaging, for example, in diagnosis of many medical conditions, as the required biomarker in radioimaging techniques such as positron emission tomography (PET). Used as labeled bioprobes with high affinity and selectivity for a microorganism, specific receptor, enzyme or membrane, the radiolabeled amine-borane compounds might accumulate in those organs and tissues where the targeted microorganism, protein or membrane component is expressed. Scanning such radiolabeled amine-borane compounds can therefore provide an accurate distribution of these microorganisms, proteins or membrane components within the living human body.

An exemplary ¹⁸F-radiolabeled amine-borane compound according to the present invention, Compound **[¹⁸F] K-I₄,** was prepared and characterized, as demonstrated in the Examples section that follows.

The efficacy of Compound **[¹⁸F]K-I₄** as a radiolabeled amine-borane compound was demonstrated in a PET radioimaging experiment in rats, as presented in the Examples section that follows.

Therefore, according to an embodiment of the present invention the above process wherein the hydroxy or alkoxy groups are exchanged with a fluoro, use a fluorinating agent which includes a radioactive ¹⁸F, *e.g*., Cs¹⁸F or X¹⁸F.

While the incorporation of a radioactive fluorine isotope has been described above, similarly, other radioactive isotopes can be incorporated in the amine-borane compounds disclosed herein, using radiolabeling reagents and techniques known in the art. These include, for example various isotopes of iodine, bromine, boron and carbon.

Thus, according to the present invention there is provided a radiolabeled compound having the general Formulae: 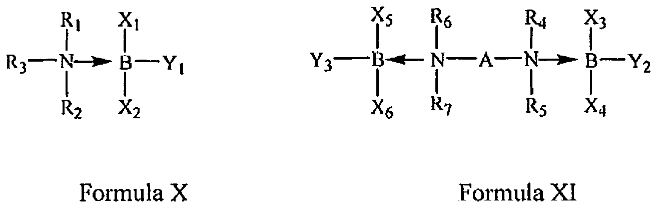 or a pharmaceutically acceptable salt thereof, wherein Y₁₋Y₃ are as defined hereinabove; X₁-X₆ are as defined hereinabove, R₁-R₇ are as defined hereinabove, and A is as defined hereinabove,
whereas at least one of X₁-X₆, R₁-R₇ and/or A comprises a radioactive atom, or, alternatively, the boron is a radioactive boron.

The radioactive atom can be, for example, boron-10 (¹⁰B), carbon-11 (¹¹C), fluorine-18 (¹⁸F), bromine-76 (⁷⁶Br), bromine-77 (⁷⁷Br), iodine-123 (¹²³I), iodine-124 (¹²⁴I) and iodine-131 (¹³¹I).

As used herein, the phrase "radiolabeled compound" or "radioactive atom" (type specified or not) refer to a compound that comprises one or more radioactive atoms or to a radioactive atom with a specific radioactivity above that of background level for that atom. It is well known, in this respect, that naturally occurring elements are present in the form of varying isotopes, some of which are radioactive isotopes. The radioactivity of the naturally occurring elements is a result of the natural distribution of these isotopes, and is commonly referred to as a background radioactive level. However, there are known methods of enriching a certain element with isotopes that are radioactive. The result of such enrichment is a population of atoms characterized by higher radioactivity then a natural population of that atom, and thus the specific radioactivity thereof is above the background level.

Thus, the radiolabeled compounds of the present invention have a specific radioactivity that is higher than the corresponding non-labeled compounds, and can therefore be used as agents for radioimaging and radiotherapy.

The term "radioactive", as used herein with respect to an atom or a derivatizing group, refers to an atom or a derivatizing group that comprise a radioactive atom and therefore the specific radioactivity thereof is above the background level.

Radiolabeled compounds having one or more of the above radioactive atoms, can be used in a variety of applications, depending on the radioactive atom present in the compounds.

Thus, for example, compounds having a fluorine-18, carbon-11, bromine-76 and/or iodine-124 atom can be beneficially used as biomarkers in radioimaging such as PET, as discussed in detail hereinabove.

Compounds having a iodine-123 can be used in radioimaging such as SPECT (single photon emission computed tomography).

Compounds having a bromine-77, boron-10, iodine-124 and/or iodine-131 can be used as radiopharmaceuticals in radiotherapy.

The various chemical features of the radiolabeled compounds according to the present embodiments are similar to those described regarding the non-labeled novel compounds presented hereinabove.

The present invention therefore also encompasses pharmaceutical compositions, uses and methods of treatment, as these are defined hereinabove, utilizing the radiolabeled compounds of the present invention discussed and presented herein.

Additional objects, advantages, and novel features of the present invention will become apparent to one ordinarily skilled in the art upon examination of the following examples, which are not intended to be limiting. Additionally, each of the various embodiments and aspects of the present invention as delineated hereinabove and as claimed in the claims section below finds experimental support in the following examples.

### EXAMPLES

Reference is now made to the following examples, which together with the above descriptions, illustrate the invention in a non limiting fashion.

### EXAMPLE 1

### CHEMICAL SYNTHESES

### Materials and methods:

All reactions were carried out under nitrogen atmosphere. The solvents were dried by conventional methods and were distilled before use. All other chemicals were obtained from Sigma-Aldrich and were used as received without any further purification.

The following amine-boranes were prepared according to published procedures: (2-hydroxy-2-phenyl-ethyl)-dimethyl-amine cyanoborane (K-B) [22], ethyl-dimethyl-amine cyanoborane (K-C) [22], but-3-enyl-dimethyl-amine cyanoborane (K-D) [22], trimethyl-amine cyanodibromoborane (K-E) [Takrouri et al., J. Organometallic Chem.y, 2005, 690, 4150**;** Györi et al., Inorg. Chilli. Acta, 1994, 218, 21], trimethyl-amine cyanoborane (K-F) [Wisian-Neilson et al., Inorg. Chem. 1978, 17, 2327], butyl-dimethyl-amine cyanoborane **(K-G) [22],** pentyl-dimethyl-amine cyanoborane (K-H) [22] and dimethyl-trimethylsilanylmethyl-amine cyanoborane (K-K) [22].

Sonication was performed, for example, on an Astrason^{®} 50/60Hz 1.2Amps sonicator.

¹H, ¹¹B, ¹³C and ¹⁹F NMR spectra were recorded in a CDCl₃ or a D₂O solution on a Varian Unity Spectrometer (300, 96, 75, 282 MHz) using Me₄Si as an internal standard.

LC-MS analyses were performed on a Finnigan LCQDUO Thermo Quad, with electron spray detector.

Infrared spectra for solid samples were measured in KBr disks or NaCl cells on a Bruker Vector 22 FT-IR spectrophotometer.

Elemental analyses were performed using the Perkin-Elmer 2400 series II Analyzer, using a combustion method to convert the sample elements to simple gases. Determination of halogens was performed using the oxygen-flask method (Schoniger application) for the decomposition of organic samples and then potentiometric titration by the Metrohm 686 Titroprocessor

The melting point of the solid samples was measured on a Fisher Scientific melting point apparatus.

### Preparation of amine fluorocyanoboranes from bromocyanoboranes - General Procedure:

Synthesis of amine fluorocyanoboranes (R₁R₂R₃NBHFCN, R₁R₂R₃NBF₂CN and R₁R₂R₃NBFBrCN) wherein R₁, R₂ and R₃ are each an alkyl, is carried out by reacting the corresponding amine bromocyanoboranes (R₁R₂R₃NBHBrCN and R₁R₂R₃NBBr₂CN) with Silver fluoride (AgF) according to a method developed by the present inventors and described in Shalom et al., Organometallics (2004), 23(11), 4396-4399 (see, Scheme 1 below).

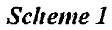

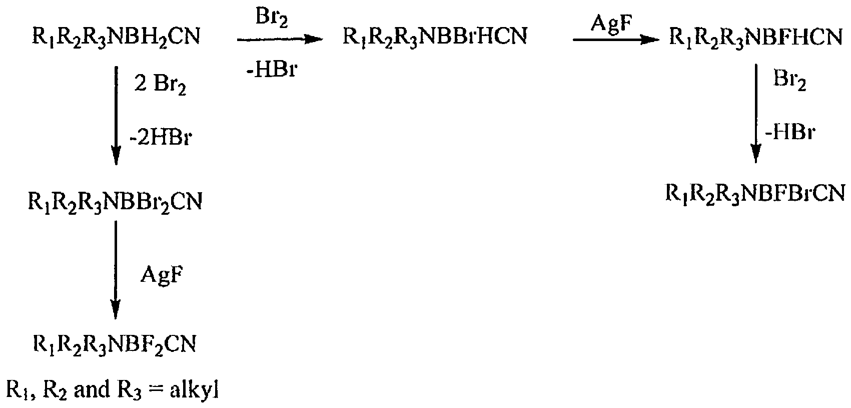

Briefly, amine cyanobromoborane and cyanodibromoborane are prepared as described in the art [Gyoeri, Bela et al., Inorganic Chemistry (1998), 37(20), 5131-5141; Synthesis (1995), (2), 191-4; and J. Organometallic Chem. (1994), 484(1-2), 225-31].

Silver fluoride (AgF, 5-15 equivalents) is added to dry benzene or toluene, and the obtained amine cyanobromoborane or amine cyanodibromoborane (R₁R₂R₃NBHBrCN or R₁R₂R₃NBBr₂CN, 1 equivalent), dissolved in dry benzene or toluene is added dropwise under nitrogen to the silver solution while the mixture is subjected to sonication and stirring for 5 to 15 hours. The reaction mixture is thereafter filtered to remove excess AgF and the solvent is evaporated under reduced pressure to thereby obtain the amine cyanofluoroborane or amine cyanodifluoroborane.

The amine cyanofluorobromoborane is prepared by brominating the corresponding amine cyanodifluoroborane with bromine (1.1 equivalents) to thereby obtain mixed halocyanoborane, namely amine cyanofluorobromoborane.

Alternatively, the bromine to fluorine exchange is carried out by dissolving the corresponding amine cyanobromoborane or amine cyanodibromoborane in toluene. Triethylamine trihydrofluoride (Et₃N/3HF, 3.3 equivalents) is added thereafter and the reaction mixture is refluxed for 24 hours. The solvent is thereafter evaporated under reduced pressure and the corresponding amine cyanofluoroborane, amine cyanofluorobromoborane or amine cyanodifluoroborane are obtained and isolated.

Using this general procedure, the following novel fluorinated amine-boranes were prepared:

### Trimethyl-amine cyanofluoroborane (Compound 1):

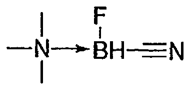

Trimethyl-amine cyanobromoborane (1 mmol, 1 equivalent) was dissolved in 2 ml of dry benzene. Silver fluoride (AgF, 99 % pure, 5 equivalents) was suspended in 5 ml of dry benzene and added to the reaction mixture, and thereafter sonicated for 5 hours. The solution was then filtered and the solvent was removed from the filtrate under reduced pressure.

Compound 1 was obtained as colorless crystals at 65 % yield.
Melting point: 53 °C.
¹H NMR (CDCl₃): δ = 2.69 (s, 9H), 3.60 (s, 3H).
¹³C {¹H} NMR (CDCl₃): δ = 1.23, 48.68 (BC cannot be detected).
¹¹B NMR (CDCl₃): δ = -2.30 (dd *J_{B-H}*= 64 Hz *J_{B-F}*= 64 Hz).
¹⁹F NMR (CDCl₃): δ = -27.1 (qd *J_{F-B}=* 61 Hz, *J_{F-N}=* 18 Hz).
LC-MS: 115.33
IR (Kerosene, cm⁻¹): (2971 cm⁻¹) C-H, (2219cm⁻¹) C=N, (1455cm⁻¹) C-N, (1104) B-F, (2413) B-H stretching vibrations.
Elemental analysis for C₄H₁₀BFN₂: Calculated - C, 41.44; H, 8.69; N, 24.16, F, 16.39; Measured - C, 40.21; H, 8.55; N, 23.51; F, 16.41.

### Ethyldimethylamine cyanofluoroborane (Compound 2):

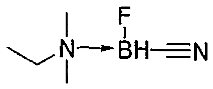

Compound 2 was prepared from the corresponding ethyl-dimethyl-amine cyanobromoborane as described for Compound 1 hereinabove.

Compound 2 was obtained as a yellow oil at 63 % yield.
¹H NMR (CDCl₃): δ = 1.28 (t, 3H, *J* = 7.2 Hz), 2.66 (s, 6H), 3.21 (q, 2H, J= 7.2 Hz).
¹³C {¹H} NMR (CDCl₃): δ = 8.64, 47.1, 56.69, (BC cannot be detected).
¹¹B NMR (CDCl₃): δ = -2.42 (dd *J_{B-H}*, *J_{B-F}* = 64 Hz).
¹⁹F NMR (CDCl₃): δ = -25.9 (qd *J_{F-B}*= 39 Hz, *J_{F-H}*= 15 Hz).
LC-MS: 129.91
IR (Kerosene, cm⁻¹): (2959cm⁻¹) C-H, (2234cm⁻¹) C=N, (1455cm⁻¹) C-N, (1040) B-F, (2452) B-H stretching vibrations.
Elemental analysis for C₅H₁₂BFN₂: Calculated - C, 46.21; H, 9.31; N, 21.55, F, 14.62; Measured - 46.83; H, 9.20; N, 21.32; F, 14.62.

### Butyldimethylamine cyanofluoroborane (Compound 3):

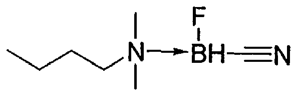

Compound 3 was prepared from the corresponding butyl-dimethyl-amine cyanobromoborane as described for Compound **1** hereinabove.

Compound 3 was obtained as a brown oil at 66 % yield.
¹H NMR (CDCl₃): δ = 0.97 (t, 3H, *J* = 5.4 Hz), 1.32 (m, 2H, *J* = 7.2 Hz), 1.65 (m, 2H, *J*= 3.9 Hz), 2.61 (s, 6H), 2.87 (t, 2H, *J*= 3.6 Hz).
¹¹B NMR (CDCl₃): δ = -2.45 (dd *J_{B-H}, J_{B-F}* = 42 Hz).
¹³C {¹H} NMR (CDCl₃): δ = 13.95, 20.50, 24.5, 45.02, 59.68, 128.57 (BC cannot be detected).
¹⁹F NMR (CDCl₃): δ = -25.5 (qd *J_{F-B}* = 40 Hz, *J_{F-H}=* 16 Hz).
LC-MS: 158.11
IR (Kerosene, cm⁻¹): (2962cm⁻¹) C-H, (2218cm⁻¹) C=N, (1455cm⁻¹) C-N, (1110) B-F, (2418) B-H stretching vibrations.
Elemental analysis for C₇H₁₆BF₂N₂: Calculated - C, 53.20; H, 10.21; N, 17.73, F, 12.02; Measured - C, 53.46; H, 10.36; N, 18.11; F, 12.26.

### Trimethylamine cyanodifluoroborane (Compound 8):

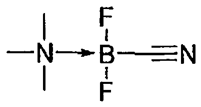

Trimethyl-amine cyanodibromoborane (1 mmol, 1 equivalent) was dissolved in 2 ml of dry benzene. Silver fluoride (AgF, 99 % pure, 5 equivalents) was suspended 5 ml of dry benzene and added to the reaction mixture, and thereafter sonicated for 10 hours. The solution was then filtered and the solvent was removed from the filtrate under reduced pressure.

Compound **8** was obtained as a solid at 67 % yield.
Melting point: 48 °C.
¹H NMR (CDCl₃): δ = 2.69 (s, 9H).
¹³C {¹H} NMR (CDCl₃): δ = 42.92 (BC cannot be detected).
¹¹B NMR (CDCl₃): δ = -2.86 (t *J*= 38.4 Hz).
¹⁹F NMR (CDCl₃): δ = -159.75 (q *J*= 36.6 Hz).
LC-MS : 134.11.
IR (KBr, cm⁻¹): (2955- 2971 cm⁻¹) C-H, (2219-2260 cm⁻¹) C=N, (1455-1493 cm⁻¹) C-N, (1104-1193) B-F, (2413-2473).
Elemental analysis for C₄H₉BF₂N₂: Calculated - C, 35.87; H, 6.77; N, 20.92, F, 28.37; Measured - C, 36.02; H, 6.58; N, 21.16; F, 28.35.

### Trimethylamine cyanofluorobromoborane (Compound 11):

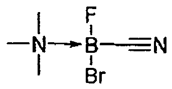

Trimethyl amine cyanofluoroborane (Compound **1**, 0.5 mmol, **1** equivalent) was dissolved in 2 ml of distilled water and cooled to 0 °C, Bromine (0.51 mmol) was dissolved in 5 ml distilled water and was added dropwise to solution containing the amine-borane. The reaction mixture was allowed to stir at 0 °C for 4 hours. The produced precipitate was thereafter filtrated, and recrystallized from hot water.

Compound **11** was obtained as a brown oil at 65 % yield.
¹H NMR (CDCl₃): δ = 2.69 (s, 9H), 3.60 (s, 3H).
¹³C {¹H} NMR (CDCl₃): δ = 1.23, 48.68 (BC cannot be detected).
¹¹B NMR (CDCl₃): δ = -2.30 (q *J*= 64 Hz).
¹⁹F NMR (CDCl₃): δ = -27.1 (q *J*= 61 Hz).
LC-MS: 193.91.
IR (KBr, cm⁻¹): 2954 (C-H) stretching vibrations, 1681 (C=O), 1460 (C-H) bending vibrations, 1204 (C-O), 450 (B-N).
Elemental analysis for C₄H₁₀BFN₂: Calculated - C, 41.44; H, 8.69; N, 24.16, F, 16.39; Measured - C, 24.56; H, 4.56; ; N, 14.38; Br, 40.91; F, 9.75.

Dimethyl-undecyl-amine cyanofluorobromoborane (Compound **K-I₃**) was similarly prepared.

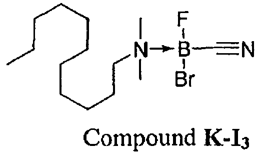

### Preparation of amine fluorocarboxyboranes and esters thereof from bromocarboxyboranes - General Procedure:

Synthesis of amine fluorocarboxyboranes and esters thereof (R₁R₂R₃NBHFCO₂H, R₁R₂R₃NBF₂CO₂H, R₁R₂R₃NBFBrCO₂H, R₁R₂R₃NBHFCO₂Ra, R₁R₂R₃NBF₂CO₂Ra and R₁R₂R₃NBFBrCO₂Ra) wherein R₁, R₂ and R₃ are each an alkyl and Ra is e.g. alkyl, alkenyl and aryl, is carried out by reacting the corresponding amine bromocarboxyboranes, dibromacarboxyboranes or the corresponding amine bromocarboxyborane or dibromocarboxyboranes esters (R₁R₂R₃NBHBrCO₂H, R₁R₂R₃NBBr₂CO₂H, R₁R₂R₃NBHBrCO₂Ra and R₁R₂R₃NBBr₂CO₂Ra) with AgF according to a method developed by the present inventors and described in Shalom el al., Organometallics (2004), 23(11), 4396-4399 (see, Scheme 2 below).

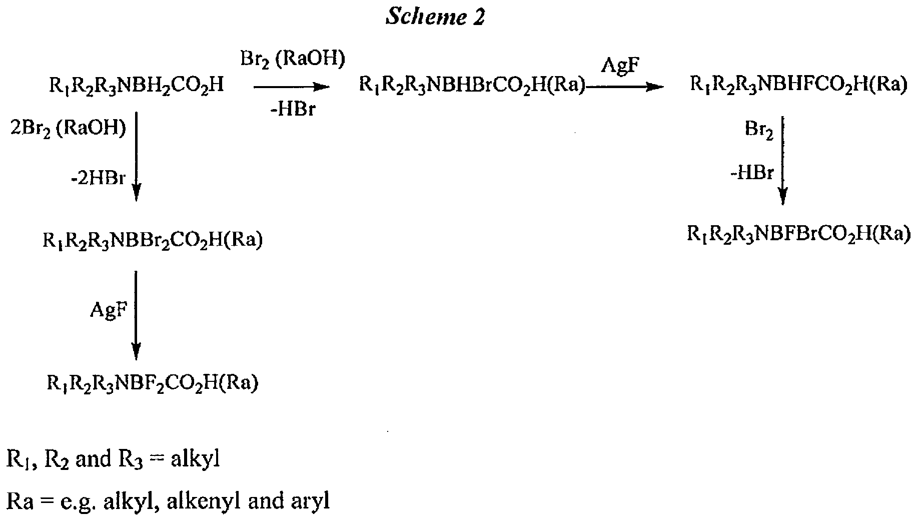

Briefly, amine carboxybromoborane, amine carboxydibromoborane and alkyl esters thereof are prepared as described in the art [Shalom et al., Organometallics 2004, 23, 4396-4399; Gyoeri, Bela et al., Inorganic Chemistry (1998), 37(20), 5131-5141; *Synthesis* (1995), (2), 191-4; and J. Organometallic Chem. (1994), 484(1-2), 225-31].

Silver fluoride (AgF, 5-15 equivalents) is added to dry benzene or toluene, and the obtained amine carboxybromoborane, amine carboxydibromoborane or esters thereof (R₁R₂R₃NBHBrCOOH/R₄ or R₁R₂R₃NBBr₂COOH/R₄, 1 equivalent), dissolved in dry benzene or toluene is added dropwise under nitrogen to the reaction mixture while the mixture is subjected to sonication and stirring for 5 to 15 hours. The reaction mixture is thereafter filtered to remove excess AgF and the solvent is evaporated under reduced pressure to thereby obtain the amine carboxyfluoroborane, the amine carboxydifluoroborane or the esters thereof.

The amine carboxyfluorobromoborane is prepared by brominating the corresponding amine carboxyfluoroborane with bromine (1.1 equivalents) to thereby obtain mixed halocarboxyborane, namely amine carboxyfluorobromoborane.

Alternatively, the bromine to fluorine exchange is carried out by dissolving the corresponding amine carboxybromoborane or amine carboxydibromoborane and the corresponding esters thereof in toluene. Triethylamine trihydrofluoride (Et₃N/3HF, 3.3 equivalents) is added and the reaction mixture is refluxed for 24 hours. The solvent is thereafter evaporated under reduced pressure and the corresponding amine carboxyfluoroborane or amine carboxydifluoroborane and the corresponding esters thereof are obtained and isolated.

Using this general procedure the following novel compounds were prepared:

### Trimethylamine carboxyfluoroborane methyl ester (Compound 4):

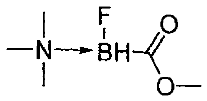

Compound **4** was prepared from the corresponding trimethyl-amine carboxybromoborane methyl ester as described for Compound **1** hereinabove.

Compound **4** was obtained as a yellow oil at 69 % yield.
¹H NMR (CDCl₃): δ = 2.66 (s, 9H), 3.58 (s, 3H).
¹³C {¹H} NMR (CDCl₃): δ = 1.23, 48.68, 128.55 (BC cannot be detected).
¹¹B NMR (CDCl₃): δ = 0.26 (dd *J_{B-H}, J_{B-F} =* 44 Hz).
¹⁹F NMR (CDCl₃): δ = -27.3 (qd *J_{F-B}* = 52 Hz, *J_{F-H}=* 21 Hz).
LC-MS: 149.03.
IR (Kerosene, cm⁻¹): (2955cm⁻¹) C-H, (1696cm⁻¹) C=O, (1455cm⁻¹) C-N, (1030) B-F, (2473) B-H stretching vibrations.
Elemental analysis for C₅H₁₃BFNO₂: Calculated - C, 40.31; H, 8.80; N, 9.40, F, 12.75; Measured - C, 40.43; H, 9.02; N, 9.57; F, 12.73.

### Trimethylamine carboxyfluoroborane ethyl ester (Compound 5):

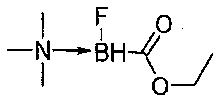

Compound **5** was prepared from the corresponding trimethyl-amine carboxybromoborane ethyl ester as described for Compound **1** hereinabove.

Compound **5** was obtained as a yellow oil at 65 % yield.
¹H NMR (CDCl₃): δ = 1.24 (t, 3H, *J =* 7.2 Hz), 2.67 (s, 9H), 4.08 (q, 2H, *J=* 6.9 Hz).
¹³C {¹H} NMR (CDCl₃): δ = 14.85, 48.69, 57.02, (BC cannot be detected).
¹¹B NMR (CDCl₃): δ = 0.29 (dd *J_{B-H}, J_{B-F}* = 38 Hz).
¹⁹F NMR (CDCl₃): δ = -26.98 (qd *J_{F-B}* = 46, *Hz J_{F-H}=* 20 Hz).
LC-MS: 163.12.
IR (Kerosene, cm⁻¹): (2960cm⁻¹) C-H, (1702m⁻¹) C=O, (1205cm⁻¹) C-O, (1493cm⁻¹) C-N, (1098) B-F, (2473) B-H stretching vibrations.
Elemental analysis for C₆H₁₅BFNO₂: Calculated - C, 44.21; H, 9.28; N, 8.59, F, 11.66; Measured - C, 44.13; H, 8.98; N, 8.57; F, 11.62.

### Ethyldimethylamine carboxyfluoroborane methyl ester (Compound 6):

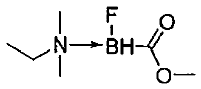

Compound 6 was prepared from the corresponding ethyl-dimethyl-amine carboxyfluoroborane methyl ester as described for Compound 1 hereinabove.

Compound 6 was obtained as a yellow oil at 69 % yield.
¹H NMR (CDCl₃): δ = 1.21 (t, 3H, *J*= 7.2 Hz), 2.59 (s, 6H), 2.97 (q, 2H, *J=* 7.5 Hz), 3.58 (s, 3H).
¹³C {¹H} NMR (CDCl₃): δ = 16.47, 47.75, 52.19, 62.13 (BC cannot be detected).
¹¹B NMR (CDCl₃): δ = -0.23 (*dd J_{B-H}, J_{B-F} =* 76 Hz).
¹⁹F NMR (CDCl₃): δ = -27.7 (qd *J_{F-B}* = 46 Hz, *J_{F-H}=* 20 Hz).
LC-MS: 163.12.
IR (Kerosene, cm⁻¹): (2960cm⁻¹) C-H, (1702cm⁻¹) C=O, (1204cm⁻¹) C-O, (1493cm⁻¹) C-N, (1098) B-F, (2475) B-H stretching vibrations.
Elemental analysis for C₆H₁₅BFNO₂: Calculated - C, 44.21; H, 9.28; N, 8.59, F, 11.66; Measured - C, 44.37; H, 9.56; N, 8.72; F, 11.62.

### Butyldimethylamine carboxyfluoroborane methyl ester (Compound 7):

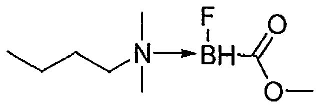

Compound **7** was prepared from the corresponding butyl-dimethyl-amine carboxyfluoroborane methyl ester as described for Compound 1 hereinabove.

Compound **7** was obtained as a brown oil at 59 % yield.
¹H NMR (CDCl₃): δ = 0.97 (t, 3H, *J* = 5.4 Hz), 1.32 (m, 2H, *J =* 7.2 Hz), 1.65 (m, 2H, J= 3.9 Hz), 2.61 (s, 6H), 2.87 (t, 2H, *J=* 3.6 Hz).
¹³C {¹H} NMR (CDCl₃): δ = 13.95, 20.50, 24.5, 45.02, 59.68, 128.57 (BC cannot be detected).
¹¹B NMR (CDCl₃): δ = -2.45 (dd *J_{B-H}, J_{B-F} =* 42 Hz).
¹⁹F NMR (CDCl₃): δ = -25.5 (qd *J_{F-B}* = 40 Hz, *J_{F-H}=* 17 Hz).).
LC-MS: 191.03.
IR (Kerosene, cm⁻¹): (2957cm⁻¹) C-H, (1698cm⁻¹) C=O, (1248cm⁻¹) C-O, (1495cm⁻¹) C-N, (1131) B-F, (2475) B-H stretching vibrations.
Elemental analysis for C₇H₁₆BF₂N₂: Calculated - C, 53.20; H, 10.21; N, 17.73, F, 12.02; Measured - C, 53.46; H, 10.26; N, 18.11; F, 12.06.

### Trimethylamine carboxydifluoroborane methyl ester (Compound 9):

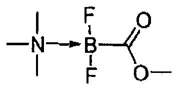

Compound **9** was prepared from the corresponding trimethyl-amine carboxydibromoborane methyl ester as described for Compound **8** hereinabove.

Compound **9** was obtained as a solid at 70 % yield.
Melting point: 66 °C.
¹H NMR (CDCl₃): δ = 2.69 (s, 9H), 3.62 (s, 3H).
¹³C {¹H} NMR (CDCl₃): δ = 47.34, 49.05, (BC cannot be detected).
¹¹B NMR (CDCl₃): δ = -1.47 (t, *J*= 54.91 Hz).
¹⁹F NMR (CDCl₃): δ -167.63 (q *J*=54.7 Hz).
LC-MS: 166.9.
IR (KBr, cm⁻¹): (2960cm⁻¹) C-H, (1702cm⁻¹) C=O, (1204cm⁻¹) C-O, (1493cm⁻¹) C-N, (1098) B-F, (2475).
Elemental analysis for C₅H₁₂BF₂NO₂: Calculated - C, 35.97; H, 7.24; N, 8.39, F; 22.76; Measured - C, 35.82; H, 7.21; N, 8.42; F; 22.84.

### Trimethylamine carboxydifluoroborane ethyl ester (Compound 10):

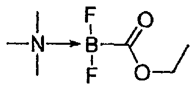

Compound **10** was prepared from the corresponding trimethyl-amine carboxydibromoborane ethyl ester as described for Compound 8 hereinabove.

Compound **10** was obtained as a yellow oil at 66 % yield.
¹H NMR (CDCl₃): δ = 1.26 (t, 3H, *J=* 6.9 Hz), 2.69 (s, 9H), 4.12 (q, 2H, *J=* 7.2 Hz).
¹³C {¹H} NMR (CDCl₃): δ = 16.57, 47.34, 53.05, (BC cannot be detected).
¹¹B NMR (CDCl₃): δ = 1.64 (t *J*= 66.2 Hz).
¹⁹F NMR (CDCl₃): δ = -167.75 (q *J*= 48.8 Hz).
IR (Kerosene, cm⁻¹): (2957cm⁻¹) C-H, (1703cm⁻¹) C=O, (1204cm⁻¹) C-O, (1493cm⁻¹) C-N, (1098) B-F, (2475).
Elemental analysis for C₆H₁₄BF₂NO₂: Calculated - C, 39.82; H, 7.80; N, 7.74, F, 20.99; Measured - C, 40.03; H, 7.99; N, 7.86; F, 20.71.

### Trimethylamine carboxyfluorobromoborane ethyl ester (Compound 12):

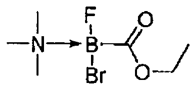

Compound **12** was prepared From the corresponding trimethyl-amine carboxyfluoroborane ethyl ester (Compound **5**) as described for Compound **11** hereinabove.

Compound **12** was obtained as a brown oil at 57 % yield.
¹H NMR (CDCl₃): δ = 1.27 (t, 3H, *J* = 6.3 Hz), 2.93 (s, 9H), 4.16 (q, 2H, J= 6.2 Hz).
¹³C {¹H} NMR (CDCl₃): δ = 14.85,46.69, 58.02, (BC cannot be detected).
¹¹B NMR (CDCl₃): δ = 2.92 (q *J*= 38.4 Hz).
¹⁹F NMR (CDCl₃): δ = -120.77 (q *J*= 91.3 Hz).
IR (Kerosene, cm⁻¹): (2971 cm⁻¹) C-H, (1493 cm⁻¹) C-N, (1705) C=O, (607 cm⁻¹) B-Br, (1193) B-F.
Elemental analysis for C₆H₁₄BBrFNO₂: Calculated - C, 29.79; H, 5.83; N, 5.79, Br, 33.03; F, 7.85; Measured - C, 28.85; H, 6.02; N, 5.82, Br, 32.93, F, 7.87.

### Triethylamine carboxydifluoroborane (Compound 13):

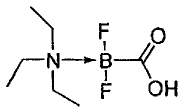

Compound 13 was prepared from the corresponding triethyl-amine carboxydibromoborane ethyl ester as described for Compound 8 hereinabove

Compound 13 was obtained as a colorless oil at 62 % yield.
¹H NMR (CDCl₃): δ = 1.08 (t, 3H, *J* - 7.5 Hz), 2.89 (q, 2H, *J* - 5.4 Hz).
¹³C {¹H} NMR {CDCl₃}: δ = 12, 48.05, (BC cannot be detected).
¹¹B NMR (CDCl₃): δ = -1.05 (t, *J* - 54.91 Hz).
¹⁹F NMR (CDCl₃): δ = -130.51 (q, *J*-54.7 Hz).
LC-MS: 195.33.
IR (Kerosene, cm⁻¹): (2959cm⁻¹) C-H, (1703cm⁻¹) C=O, (1200cm⁻¹) C-O, (1490cm⁻¹) C-N, (1100) B-F.
Elemental analysis for C₇H₁₆BF₂NO₂: Calculated - C, 43.11; H, 8.27; F, 19.48; N, 7.18; Measured - C, 42.95; H, 8.37; F, 19.66; N, 7.27

### Preparation of amine cyanoboranes having a long alkyl chain attached to the amine moiety - General Procedure:

Synthesis of amine cyanoboranes (R₈R₉R₁₀CH₂NBH₂CN) wherein R₁₀ is a high alkyl (long chain alkyl), is carried out according to a method developed by the present inventors and described in Takrouri et al., Organometallics (2004), 23(11), 2817-2820 (see, Scheme 3 below). The corresponding starting amine cyanobromoborane and amine cyanodibromoborane are prepared as described in the art [Takrouri et al., J. Organometallic Chem., 690 (2005) 4150-4158; Shalom et al., Organometallics 2004, 23, 4396-4399; Gyoeri et al., Inorganic Chemistry (1998), 37(20), 5131-5141; Synthesis (1995), (2), 191-4; and J. Organometallic Chem. (1994), 484(1-2), 225-31].

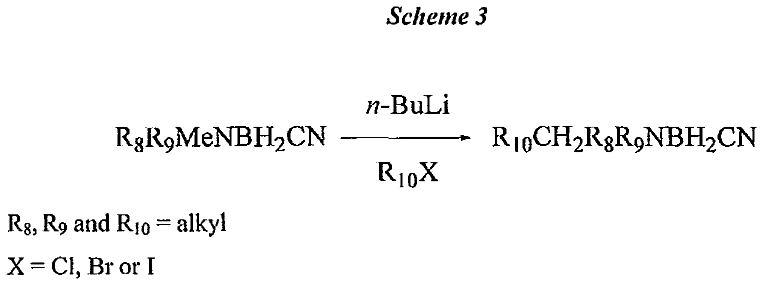

Using this general procedure, the following compounds were prepared:

### Dimethylundecylamine cyanoborane (Compound K-I):

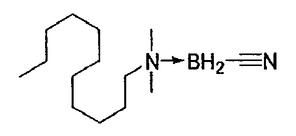

Compound K-I was obtained as white solid at 87 % yield (0.207 gram).
¹H NMR (CDCl₃): δ = 0.86 (t, 3H, *J* - 7.2 Hz), 1.25 (broad s, 14H), 1.66 (hept, 2H, *J* - 3.6), 1.83 (pent, 2H, *J -* 7.2), 2.63 (s, 6H), 2.84 (t, 2H, *J -* 5.0), (HB cannot be detected).
¹³C {¹H} NMR (CDCl₃): δ = 14.34, 22.89, 23.49, 27.13, 29.44, 29.52, 29.65, 32.09, 45.18, 46.97, 50.03, 63.84, (CB cannot be detected).
¹¹B NMR (CDCl₃): δ = -16.67 (t, *J*_{B-H} - 97.1 Hz).
IR (KBr, cm⁻¹): 2919 (B-H), 2852 (C-H), 2358 (C≡N), 1469 (C-N), 440 (BN).
Elemental analysis for C₁₄H₃₁BN₂: Calculated - C, 70.59; H, 13.12; N, 11.76; Measured - C, 70.52; H, 13.11; N, 11.75.

### Dodecyldimethylamine cyanoborane (Compound K-L):

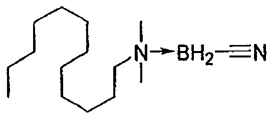

Compound K-L was obtained as yellow oil, 87 % (0.219 grams) yield.
¹H NMR (CDCl₃): δ = 0.84 (t, 3H, *J*= 7.2 Hz), 1.22 (broad s, 16H), 1.64 (hept, 2H, *J=* 3.6), 1.80 (pent, 2H, *J*= 7.2), 2.60 (s, 6H), 2.80 (t, 2H, *J=* 5.0), (HB cannot be detected).
¹³C{¹H} NMR {CDCl₃): δ = **14**.30, 22.87, 23.47, 27.11, 28.36, 28.96, 29.41, 29.64, 29.68, 29.77, 32.09, 33.03, 34.22, 50.00, 63.78, (CB cannot be detected).
¹¹B NMR (CDCl₃): δ = □□-14.80 (t, *J*_{B-H} = 102.6 Hz). IR (KBr, cm⁻¹): 2921 (B-H), 2854 (C-H), 2361 (C≡N), 1541 (C-N), 440 (B-N).

### Dimethylnonylamine cyanoborane (Compound K-P):

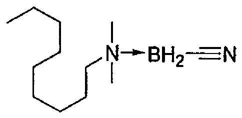

Compound K-P was obtained as white solid, 84 % (0.177 gram) yield.
¹H NMR (CDCl₃): δ = 0.86 (t, 3H, *J*= 7.2 Hz), 1.29 (broad s, 10H), 1.63 (hept, 2H, *J*= 3.6), 1.80 (pent, 2H, *J*= 7.2), 2.65 (s, 6H), 2.83 (t, 2H, *J=* 5.0), (HB cannot be detected).
¹³C{¹H} NMR (CDCl₃): δ = 14.13, 22.81, 23.23, 27.13, 29.44, 29.65, 32.09, 45.18, 50.03, 63.84, (CB cannot be detected).
¹¹B NMR (CDCl₃): δ =□□-16.01 (t, *J*_{B-H} = 97.1 Hz). IR (KBr, cm⁻¹): 2920(B-H), 2850 (C-H), 2358 (C≡N), 1469 (C-N), 432 (B-N).

### Dimethyltridecylamine cyanoborane (Compound K-Q):

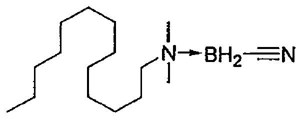

Compond K-Q was obtained as white solid, 84 % (0.224 gram) yield.
¹H NMR (CDCl₃): δ = 0.87 (t, 3H, J= 7.2 Hz), 1.25 (broad s, 16H), 1.41 (m, 2H), 1.65 (hept, 2H, *J*= 3.6), 1.84 (pent, 2H, J= 7.2), 2.63 (s, 6H), 2.84 (t, 2H, *J=* 3.9), (HB cannot be detected).
¹³C{¹H} NMR (CDCl₃): δ =□14.35, 22.91, 23.49, 27.14, 28.40, 29.00, 29.57, 29.67, 29.84, 32.13, 33.06, 34.31, 50.02, 64.00, (CB cannot be detected).
¹¹B NMR (CDCl₃): δ = □□-15.55 (t, *J*_{B-H} = 106.3 Hz). IR (KBr, cm⁻¹): 2921 (B-H), 2852 (C-H), 2345 (C=N), 1542 (C-N), 440 (B-N).

### Dimethylpentadecylamine cyanoborane (Compound K-R):

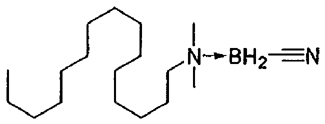

Compound K-R was obtained as white solid, 86 % (0.253 gram) yield.
¹H NMR (CDCl₃): δ = 0.87 (t, 3H, *J*= 7.2 Hz), 1.25 (broad s, 20H), 1.41 (m, 2H), 1.66 (hept, 2H, *J*= 3.6), 1.84 (pent, **2H**, *J*= 7.2), 2.63 (s, 6H), 2.84 (t, 2H, *J*= 3.6 ), (HB cannot be detected).
¹³C{¹H} NMR (CDCl₃): δ = 014.35, 22.92, 23.50, 27.16, 28.41, 29.01, 29.60, 29.68, 29.78, 29.89, 32.16, 33.08, 34.27, 47.04, 50.02, 63.84, (CB cannot be detected).
¹¹B NMR (CDCl₃): δ = □□-15.89 (t, *J*_{B-H} = 106.3 Hz). IR (KBr, cm⁻¹): 2919 (B-H), 2852 (C-H), 2346 (C≡N), 1538 (C-N), 440 (B-N).

### Dimethylheptadecylamine cyanoborane (Compound K-S):

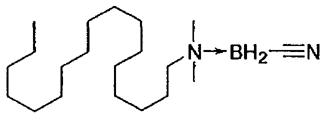

Compound K-S was obtained as white solid, 83 % (0.268 gram) yield.
¹H NMR (CDCl₃): δ = 0.88 (t, 3H, *J=* 7.2 Hz), 1.25 (broad s, 24H), 1.41 (m, 2H), 1.65 (m, 2H), 1.85 (pent, 2H, *J*= 7.2), 2.64 (s, 6H), 2.85 (t, 2H, *J*= 4.2), (HB cannot be detected).
¹³C{¹H} NMR (CDCl₃): δ = □14.36, 22.93, 28.42, 29.01, 29.60, 29.91, 23.16, 33.07, 34.33, 48.25, 52.10, 62.95, (CB cannot be detected).
¹¹B NMR (CDCl₃): δ = □□-16.04 (t, *J*_{B-H} = 106.3 Hz). IR (KBr, cm⁻¹): 2921 (B-H), 2852 (C-H), 2344 (C=N), 1542 (C-N), 433 (B-N).

### Dimethylundecylamine cyanobromoborane (Compound K-I₁):

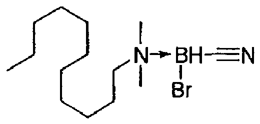

Compound **K-I₁** was obtained as brown oil, at 80 % yield (0.254 grams).
¹H NMR (CDCl₃): δ = 0.83 (t, 3H, *J* - 7.2 Hz), 1.22 (broad s, 14H), 1.65 (hept, 2H, *J* - 3.6), 1.81 (pent, 2H, *J -* 7.2), 2.77 (s, 6H), 2.94 (t, 2H, *J -* 6.9), (HB cannot be detected).
¹³C {¹H} NMR (CDCl₃): δ = 14.35, 22.89, 22.90, 27.00, 29.38, 29.51, 29.61, 32.09, 43.61, 45.24, 47.60, 47.70, 61.98, (CB cannot be detected).
¹¹B NMR (CDCl₃): δ = -10.95 (d, *J*- 177.7 Hz).
IR (KBr, cm⁻¹): 2919 (B-H), 2854 (C-H), 2358 (C=N), 1469 (C-N), 442 (B-N).
Elemental analysis for C₁₄H₃₀BBrN₂: Calculated - C, 53.02; H, 9.54; Br, 25.20; N, 8.83; Measured - C, 53.05; H, 9.51; Br, 25.22; N, 8.80.

### Dimethylundecylamine cyanodibromoborane (Compound K I₂):

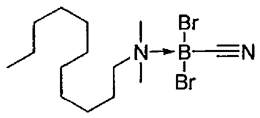

Compound **K-I₂** was obtained as brown oil, at *a* 82 % yield (0.324 gram).
¹H NMR (CDCl₃): δ = 0.85 (t, 3H, *J* - 7.2 Hz), 1.30 (broad s, 14H), 1.68 (hept, 2H, *J* - 3.6), 1.82 (pent, 2H, *J* - 7.2), 2.94 (s, 6H), 3.38 (t, 2H, *J* - 6.9), (HB cannot be detected).
¹³C {¹H} NMR (CDCl₃): δ = 14.35, 22.89, 26.98, 29.37, 29.42, 29.50, 29.66, 29.73, 32.08, 46.01, 47.71, 61.98, (CB cannot be detected).
¹¹B NMR (CDCl₃): δ = -9.36 (s).
IR (KBr, cm⁻¹): 2854 (C-H), 2362 (C=N), 1409 (C-N), 435 (B-N).
Elemental analysis for C₁₄H₂₉BBr₂N₂: Calculated - C, 42.46; H, 7.38; Br, 40.35; N, 7.07; Measured - C, 42.42; H, 3.35; Br, 40.37; N, 7.10.

### Dimethyl-undecyl-amine cyanofluoroborane (Compound K-I₄):

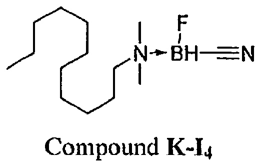

Dimethyl-undecyl-amine cyanofluoroborane (Compound **K-I₄)** was prepared by dissolving Compound **K-I₁** (1 mmol, see synthesis hereinabove) in 2 ml of dry benzene. Silver (I) fluoride 99 % (5 equivalents) was suspended in 5 ml of dry benzene and added to the reaction mixture which was sonicated for 5 hours. Thereafter, the solvent was filtered and removed under high vacuum.

Compound **K-I₄** was obtained as a brown oil at 66 % yield.
¹H NMR (CDCl₃): δ = 0.86 (t, 3H, *J* - 6.9 Hz), 1.25 (broad s, 16H), 1.4 (broad s, 2H) 2.78 (s, 6H), 3.39 (t, 2H, *J -* 6.9 Hz).
³C {¹H} NMR (CDCl₃): δ = 14.07, 22.63, 26.72, 29.33, 29.4, 31.82, 47.4, 61.71, (BC cannot be detected).
¹¹B NMR (CDCl₃): δ = -2.08 (dd, *J*_{B-H} - 75.2, *J*_{B-F} - 64.0 Hz).
¹⁹F NMR (CDCl₃): δ = -25.09 (qd, *J*_{F-B} - 40 Hz, *J*_{F-H} - 16 Hz).
IR (neat, cm⁻¹): 2924 (C-H), 2209 (C_N), 1455 (C-N), 1074 (B-F), 2491 (B-H) stretching vibrations.
Elemental analysis for C₁₃H₂₈BFN₂: Calculated -C, 64.47; H, 11.65; N, 4.46, F, 7.84; Measured - C, 64.21; H, 11.68; N, 4.49, F, 7.86.

### Preparation: of amine bis-cyanobonanes - General Procedure:

Synthesis of amine bis-cyanoboranes (NCBH₂R₄R₅N(CH₂)ₙ₊₂NR₆R₇BH₂CN), wherein n ranges from 1 to 20, is carried out as illustrated in Scheme 4 below according to the procedure described in Takrouri et al., Organometallics (2004), 23(11), 2817-2820. The corresponding starting amine cyano/carboxybromoborane, amine cyano/carboxydibromoborane and alkyl esters thereof are prepared as described in the art [Takrouri et al., J. Organometallic Chem., 690 (2005) 4150-4158; Gyoeri, Bela et al., Inorganic Chemistry (1998), 37(20), 5131-5141; Synthesis (1995), (2), 191-4; and J. Organometallic Chem. (1994), 484(1-2), 225-31].

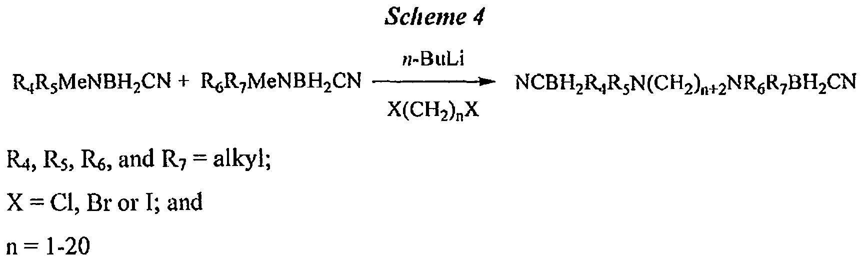

Using this general procedure, the following novel compounds were prepared:

### N,N,N',N'-tetramethyldecane-1,10-diamine bis-cyanoborane (Compound K-J):

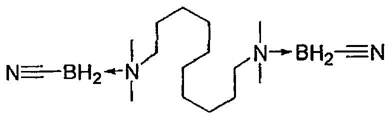

Compound **K-J** was obtained as white solid, at a 90 % yield (0.276 gram).
¹H NMR (CDCl₃): δ = 1.29 (broad s, 8H), 1.66 (m, 4H), 1.84 (m, 4H), 2.63 (s, 12H), 2.84 (t, 4H, *J* - 3.9 Hz), (HB cannot be detected).
¹³C {¹H} NMR (CDCl₃): δ = 23.47, 27.04, 29.27, 29.40, 50.15, 63.78, (CB cannot be detected).
¹¹B NMR (CDCl₃): δ = -16.67 (t,*J*_{B-H}) 101.1 Hz).

IR (KBr, cm⁻¹): 2923 (B-H), 2854 (C-H), 2332 (C≡N), 1542 (C-N), 433 (B-N).
Elemental analysis for C₁₆H₃₆B₂N₄: Calculated - C, 62.78; H, 11.85; N, 18.30; Measured - C, 62.73; H, 11.88; N, 18.33.

### N,N,N',N'-tetramethyldodecane-1,12-diamine bis-cyanoborane (Compound K-M):

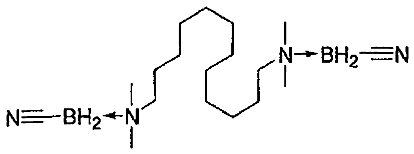

Comound K-M was obtained as yellow solid, 88 % (0,294 gram) yield.
¹H NMR (CDCl₃): δ = 1.27 (broad s, 12H), 1.66 (m, 4H), 1.98 (m, 4H), 2.63 (s, 12H), 2.85 (t, 4H, *J*= 3.9 Hz), (HB cannot be detected).
¹³C{¹H) NMR (CDCl₃): δ = □23.45, 27.06, 29.33, 29.53, 29.86, 50.07, 63.77, (CB cannot be detected).
¹¹B NMR (CDCl₃): δ = □□-15.53 (t, *J*_{B-H} = 106.3 Hz).
IR (KBr, cm⁻¹): 2921 (B-H), 2852 (C-H), 2362 (C=N), 1542 (C-N), 440 (B-N).

### N,N,N',N'-tetramethyltetradecane-1,14-diamine bis-cyanoborane (Compound K-N):

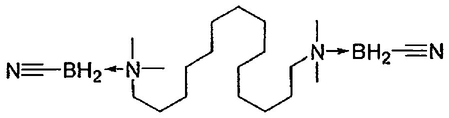

Compound K-N was obtained as white solid, 87 % (0.315 g) yield.
¹H NMR (CDCl₃): δ = 1.22 (broad s, 16H), 1.62 (m, 4H), 1.81 (m, 4H), 2.60 (s, 12H), 2.80 (m, 4H), (HB cannot be detected).
¹³C{¹H} NMR (CDCl₃): δ = □23.49, 27.11, 28.96, 29.62, 33.03, 34.34, 50.07, 63.82, (CB cannot be detected).
¹¹B NMR (CDCl₃): δ = □□-14.86 (t, *J*_{B-H}= 112.3 Hz).
IR (KBr, cm⁻¹): 2920 (B-H), 2853 (C-H), 2334 (C≡N), 1542 (C-N), 440 (B-N).

### N,N,N',N'-tetramethyldecane-1,10-diamine bis-cyanobromoborane (Compound K-J₁):

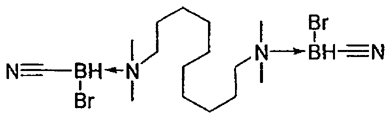

Compound **K-J₁** was obtained as brown oil at 82 % yield (0.380 gram).
¹H NMR (CDCl₃): δ = 1.30 (broad s, 8H), 1.68 (m, 4H), 1.86 (m, 4H), 2.78 (s, 12H), 3.05 (m, 4H), (HB cannot be detected).
¹³C {¹H} NMR (CDCl₃): δ = 22.91, 26.89, 29.17, 29.32, 50.84 61.98, (CB cannot be detected).
¹¹B NMR (CDCl₃): δ = -11.38 (d, *J*_{B-H} - 113.7 Hz).
IR (neat, cm⁻¹): 2927 (B-H), 2856 (C-H), 2341 (C≡N), 1542 (C-N), 440 (B-N).
Elemental analysis for C₁₆H₃₄B₂Br₂N₄: Calculated - C, 41.43; H, 7.39; Br, 34.45; N, 12.08; Measured - C, 41.45; H, 7.41; Br, 34.43; N, 12.12.

### N,N,N',N'-tetramethyldecane-1,10-diamine bis-cyanobromoborane (Compound K-J₂):

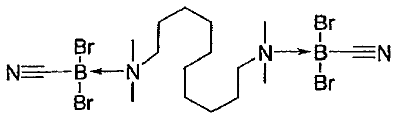

Compound **K-J₂** was obtained as brown oil at 83 % yield (0.516 gram).
¹H NMR (CDCl₃): δ = 1,31 (broad s, 8H), 1.70 (m, 4H), 1.88 (m, 4H), 2.95 (s, 12H), 3.38 (m, 4H), (HB cannot be detected).
¹³C {¹H} NMR (CDCl₃): δ = 22.90, 26.88, 29.17, 29.31, 50.82, 61.97, (CB cannot be detected).
¹¹B NMR (CDCl₃): δ = -9.38 (s).
IR (neat, cm⁻¹): 2856 (C-H), 2362 (C=N), 1542 (C-N), 440 (B-N).
Elemental analysis for C₁₆H₃₂B₂Br₄N₄: Calculated - C, 30.91; H, 5.19; Br, 51.41; N, 9.41; Measured - C, 30.88; H, 5.22; Br, 51.39; N, 9.05.

### N,N,N',N'-tetramethyldecane-1,10-diamine bis-canboxyborane (Compound K-J₃):

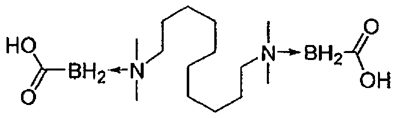

Compound **K-J₃** was obtained as white oil at 78 % yield (0. 268 gram).
¹H NMR (CDCl₃): δ = 1.26 (broad s, 8H), 1.61 (m, 4H), 1.83 (pent, 4H, *J* - 6.9 Hz), 2.66 (s, 12H), 2.91 (t, 4H, *J*- 4.2 Hz), (HB cannot be detected).
¹³C{¹H} NMR (CDCl₃): δ = 23.27, 29.31, 32.91, 34.35, 52.36, 62.78, (CB cannot be detected).
¹¹B NMR (CDCl₃): δ = -8.96 (broad s).
IR (KBr, cm⁻¹): 3450 (B-O), 2960 (B-H), 2716 (C-H), 2371 (C=N), 1459 (C-N), 433 (B-N).
Elemental analysis for C₁₆H₃₈B₂N₂O₄: Calculated - C, 55.85; H, 11.13; N, 8.14; Measured - C, 55.79; H, 11.17; N, 8.21.

### Preparation of β--hydroxyamine cyanoboranes and β-alkoxyamine cyanoboranes - General Procedure:

Synthesis of β-hydroxyamine cyanoboranes and β-alkoxyamine cyanoboranes, with the intention of altering the aqueous solubility of these amine cyanoborane derivatives, is carried out using either long chain aldehydes or ketones according to a method developed by the present inventors and described in Takrouri et al., Organometallics (2004), 23(11), 2817-2820 (see, Scheme 5 below).

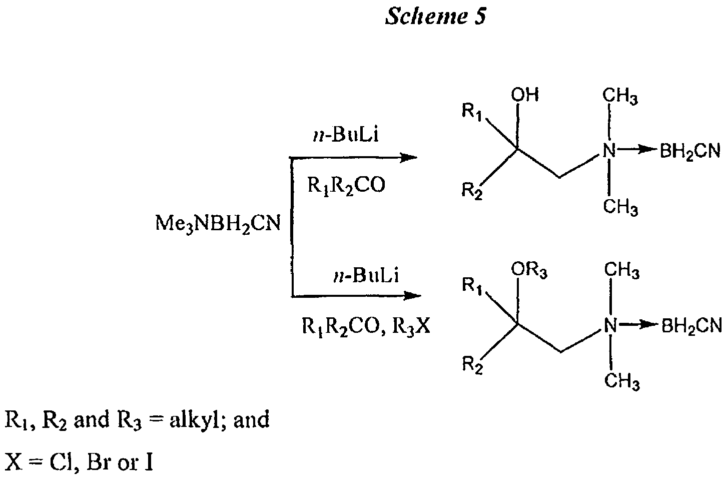

Using this general procedure, the following compounds were prepared:

### 1-dimethy/aminomethyl-cyclopent-2-enol cyanoborane (Compound K-A):

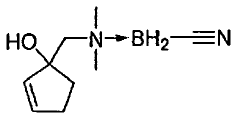

Compound K-A was obtained as yellowish solid at 80 % yield (0.144 gram).
¹H NMR (CDCl₃): δ = 2.00 (m, 2H), 2.15 (m, 2H), 2.87 (s, 3H), 2.89 (s, 3H), 3.14 (s, 1H), 3.16 (s, 1H), 5.78 (m, 1H), 6.01 (m, 1H), (HB cannot be detected).
¹³C{¹H} NMR (CDCl₃): δ = 26.29, 33.03, 54.57, 67.82, 77.60, 129.51, 140.04, (CB cannot be detected).
¹¹B NMR (CDCl₃): δ = -14.861 (t, JB-H) 106.3 Hz).
IR (KBr, cm⁻¹): 3450 (B-O), 2920 (B-H), 2853 (C-H), 2334 (C=N), 1542 (C-N), 440 (B-N).
Elemental analysis for C₉H₁₇BN₂O: Calculated - C, 60.04; H, 9.52; N, 15.56; Measured - C, 59.99; H, 9.31; N, 15.51.

### 1-dimethylamillo-dodecan-2-ol cyanoborane (Compound K-T):

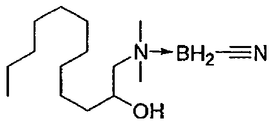

Compound K-T was obtained as yellow oil, 85 % (0.228 gram) yield.
¹H NMR (CDCl₃): δ = 0.80 (t, 3H, *J*= 7.2 Hz), 1.18 (broad s, 14H), 1.45 (m, 4H), 2.67 (s, 3H), 2.72 (s, 3H), 2.85 (m, 2H), 4.07 (m, 1H), (HB cannot be detected).
¹³C {¹H} NMR (CDCl₃): δ = 14.26, 22.83, 25.33, 29.49, 29.76, 31.05, 32.06, 36.84, 43.60, 49.30, 52.49, 52.89, 68.59, (CB cannot be detected).
¹¹B NMR (CDCl₃): δ =□□-14.87 (t, *J*_{B-H} = 102.7 Hz).
IR (KBr, cm⁻¹): 2920 (B-H), 2854 (C-H), 2360 (C≡N), 1464 (C-N), 430 (B-N).

### 1-dimethylamino-2-methyl-octan-2-ol cyanoborane (Compound K-O):

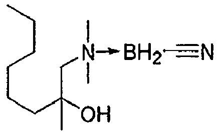

Compound **K-J** was obtained as white solid, at 85 % yield (0.192 gram).
¹H NMR (CDCl₃): δ 0.81 (m, 7H), 1.12 (pent, 2H, J - 6.0 Hz), 1.51 (m, 4H), 2.09 (s, 1H), 2.10 (s, 1H), 2.38 (td, 3H, Jt - 12 Hz, Jd - 6.0 Hz), 2.68 (s, 3H), 2.69 (s, 3H), (HB cannot be detected).
¹³C {¹H} NMR (CDCl₃): δ 14.24, 22.67, 22.82, 31.78, 32.03, 43.61, 45.73, 48.58, 53.01, (CB cannot be detected).
¹¹B NMR (CDCl₃): δ -12.46 (t, JB-H) 104.4 Hz).
IR (neat, cm⁻¹): 3447 (O-H), 2930 (B-H), 2958 (C-H), 2361 (C≡N), 1461 (C-N), 433 (B-N).
Elemental analysis for C₁₂H²⁷BN₂O: Calculated - C, 63.73; H, 12.03; N, 12.39; Measured - C, 63.71; H, 12.01; N, 12.35.

### 1-Dimethylamino-undecan-2-ol cyanoborane (Compound K-U):

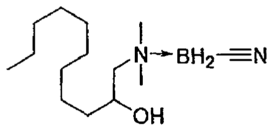

Compound K-U was obtained as yellow oil, 84 % (0.214 gram) yield.
¹H NMR (CDCl₃): δ = 0.86 (t, 3H, *J*= 6.3 Hz), 1.25 (broad s, 14H ), 1.43 (m, 2H), 2.75 (s, 3H), 2.78 (s, 3H), 2.89 (m, 2H), 4.18 (m, 1H), (HB cannot be detected).
¹³C{¹H} NMR (CDCl₃): δ = 14.33, 22.89, 25.35, 29.54, 29.73, 29.79,32.11, 36.87, 43.61, 45.17, 46.99, 53.01, (CB cannot be detected).
¹¹B NMR (CDCl₃): δ= □□-16.67 (t, *J*_{B-H}= 97.1 Hz).
IR (neat, cm⁻¹): 3447 (O-H), 2926 (B-H), 2954 (C-H), 2334 (C=N), 1543 (C-N), 443 (B-N).

### Preparation of β-fluoroamine cyanoboranes and β-fluoroamine carboxyboranes - General Procedure:

Synthesis of β-fluoroalkylamine cyanoboranes is carried out using a β-hydroxy amine cyanoboranbe described hereinabove (see, Scheme 6 below). A β-hydroxy amine cyanoboranbe is treated with triflic anhydride and 2,6-lutidine at 0 °C for I hour. The reaction mixture is then extracted to give the crude β-OTf anime cyanoborane. Without additional purification β-OTf anime cyanoborane is reacted with 5 equivalents of cesium fluoride (CsF) and 90 µl of water in 3 ml acetonitrile, and the reaction mixture is refluxed for 20 minutes at 100 °C, followed by extraction with ether.

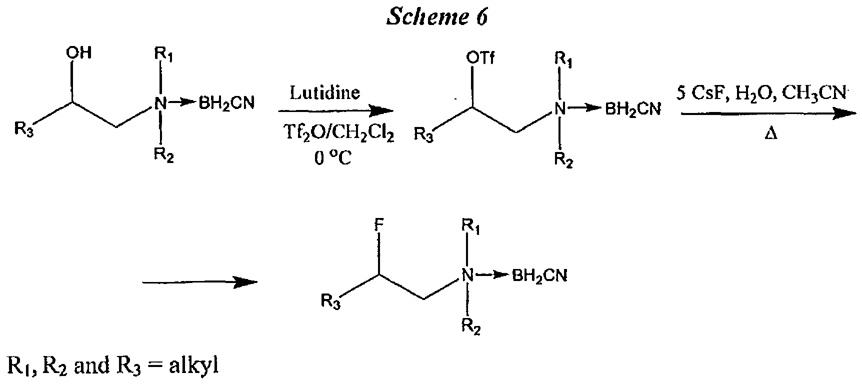

Using this general procedure, the following compound was prepared:

### (2-fluoro-nonyl)-dimethyl-amine cyanoborane (Compound 14):

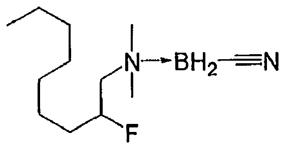

¹H-NMR (CDCl₃): δ = 2.22 (s, 3H), 2.24 (s, 3H).
¹⁹F-NMR (CDCl₃): δ = -74.31 (s).

### Preparation of amine cyanoboranes having all unsaturated alkyl chain attached to the amine moiety - General Procedure:

The presence of an unsaturated C=C bond in one or more of the alkyl *N-*substitutions was found to have a significant effect on the efficacy of the compounds presented herein, as presented in Example 2 that follows below.

Synthesis of amine cyanoboranes (R₁R₂R₃CH₂NBH₂CN) wherein R₃ is an alkyl having an unsaturated C=C bond at it end is carried out according to a method developed by the present inventors and described in Takrouri et al., Organometallics (2004), 23(11), 2817-2820 (see, Scheme 3 above). The corresponding starting amine cyanobromoborane and amine cyanodibromoborane are prepared as described in the art [Takrouri et al., J. Organometallic Chem., 690 (2005) 4150-4158; Shalom et al., Organometallics 2004, 23, 4396-4399; Gyoeri et al., Inorganic Chemistry (1998), 37(20), 5131-5141; Synthesis (1995), (2), 191-4; and J. Organometallic Chem. (1994), 484(1-2), 225-31].

Using this general procedure, the following compounds were prepared:

### Hex-5-enyl-dimethyl-amine cyanoborane (Compound K-V):

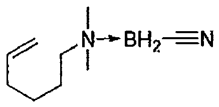

Compound **K-V** was obtained as yellow oil at 87 % yield (0. 144 gram).
¹H NMR (CDCl₃): δ = 1.39 (pent, 2H, *J* - 7.5 Hz), 1.68 (m, 2H), 2.10 (q, 2H, *J* - 7.0 Hz), 2.85 (t, 3H, *J* - 4.5 Hz), 5.00 (td, 2H, *J*_{d} - 0.6 Hz, *J*ₜ - 9.0 Hz), 5.74 (m, 1H), (BH cannot be detected).
¹³C {¹H} NMR (CDCl₃): δ = 22.79, 26.22, 33.34, 50.07, 63.54, 115.72, 137.82, (CB cannot be detected).
¹¹B NMR (CDCl₃): δ = -15.87 (t, *J*_{B-H} - 104.5 Hz).
IR (neat, cm⁻¹): 2909 (B-H), 2360 (C≡N), 1645 (C=C), 1470 (C-N), 443 (B-N).
Elemental analysis for C₉H₁₉BN₂: C, 65.09; H, 11.53; N, 16.87; Measured - C, 65.13; H, 11.47; N, 16.90.

Table 1 below presents the novel amine fluoroboranes prepared using the procedures described above.

**Table 1**

| **Compound** | **Name** | **Structure** |
|---|---|---|
| K-I₃ | dimethyl-undecyl-amine cyanofluorobromoborane | 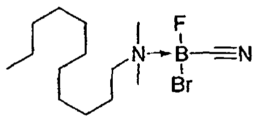 |
| K-I₄ | dimethyl-undecyl-amine cyanofluoroborane | 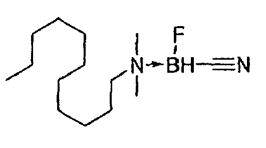 |
| 1 | trimethyl-amine cyanofluoroborane | 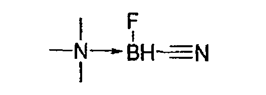 |
| 2 | ethyl-dimethyl-amine cyanofluoroborane | 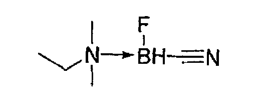 |
| 3 | butyl-dimethyl-amine cyanofluoroborane | 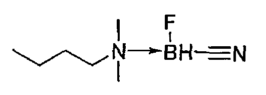 |
| 4 | trimethyl-amine carboxyfluoroborane methyl ester | 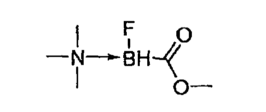 |
| 5 | trimethyl-amine carboxyfluoroborane ethyl ester | 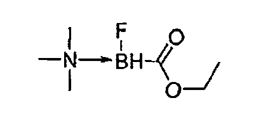 |
| 6 | ethyl-dimethyl-amine carboxyfluoroborane methyl ester | 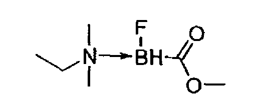 |
| 7 | butyl-dimethyl-amine carboxyfluoroborane methyl ester | 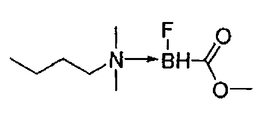 |
| 8 | trimethyl-amine cyanodifluoroborane | 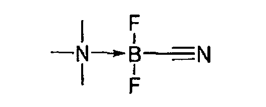 |
| 9 | trimethyl-amine carboxydifluoroborane methyl ester | 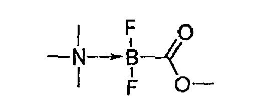 |
| 10 | trimethyl-amine carboxydifluoroborane ethyl ester | 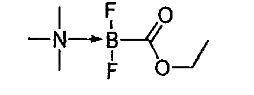 |
| 11 | trimethyl-amine cyanofluorobromoborane | 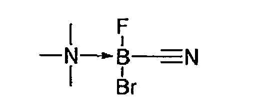 |
| 12 | trimethyl-amine carboxyfluorobromoborane ethyl ester | 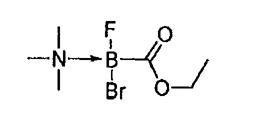 |
| 13 | triethyl-amine carboxydifluoroborane | 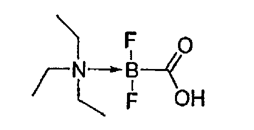 |
| 14 | (2-fluoro-nonyl)-dimethylamine cyanoborane | 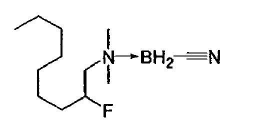 |
| K-V | hex-5-enyl-dimethyl-amine cyanoborane | 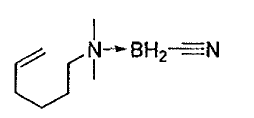 |

### Preparation of amine carboxyboranes and esters thereof - General Procedure:

Synthesis of amine carboxyboranes [17] and amine carboxyboranes esters [23-26] (R₁R₂R₃NBH₂CO₂H and R₁R₂R₃NBH₂CO₂Ra) wherein R₁, R₂ and R₃ are each an alkyl and Ra is alkyl, alkenyl, aryl and the likes, is carried out according to published procedures, for example as illustrated in Scheme 7 below, by hydrolysis of the corresponding R₁R₂R₃NMe₂BH₂CN obtained as described hereinabove.

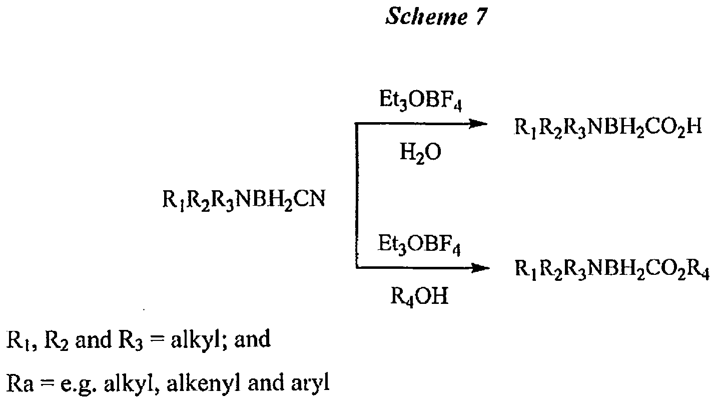

### EXAMPLE 2

### ANTIFUNGAL ACTIVITY

### Antifungal activity assays:

Antifungal activity of the amine-borane compounds was determined using *in vitro* susceptibility tests by microbroth dilution method, as detailed below.

Several yeast strains were used for susceptibility testing, namely *Candida albicans* CBS 562 (a reference type strain of the main common cause of yeast infection in humans; Centraalbureau voor Schimmelcultures, Utrecht, The Netherlands) and two clinical isolates, 607, and 615; *Candida glabrata* 4210; *Candida glabrata* 4475, a yeast strain is known to be a resistant to azoles; *Candida glabrata* 566, 572, 578, 646, and 648 and *Candida krusei* 603 and 638; *Candida g*/*abrata* 4681; *Candida glabrata* 4787; *Saprolegnia parasitica* T1, a water mould which causes a severe disease in tropical fish - *Saprolegniasis* and massive economical damages, with no effective treatment to date as well as the mold *Aspergillus fumigatus* ATCC 64026 (a reference strain; The American Type Culture Collection, Manassas, VA), a mould which is the main cause of mould infection in immunocompromised host also a main cause of fungal infection in fowl. In addition, all isolates were obtained from disseminated candidemia patients from Hadassah-Hebrew University Medical Center (Isolate numbers refer to an internal index).

The *in vitro* susceptibility of the tested strains to each of the tested compounds was determined by the broth micro dilution method according to National Committee for Clinical Laboratory Standards for yeasts (NCCLS/CLSI) recommendations for yeasts (M27-A241) [10] and for filamentous fungi (M-38A42) [27]. Briefly, 2-fold serial dilutions of drugs from stock solutions were prepared in an RPMI-1640 broth medium (Sigma, St. Louis, MO.) buffered to a final pH of 7.0 with 0.165 M morpholinepropanesulfonic acid (MOPS; Sigma) and 1M NaOH, and sterilized by filtration and inoculated with 10⁴ cells per ml. A stock solution of 10 mg/ml was prepared in dimethyl sulfoxide (DMSO, Sigma) for the various amine cyanoborane compounds and for two conventional anti-fungal agents (CAF), amphotericin B (referred to herein as CAF1) and fluconazole (referred to herein as CAF2), which were used as controls. The final drug concentrations in the test ranged from 1024 mg/L to 4 mg/L in a final volume of 0.1 ml.

The minimum fungicidal concentration (MFC) was established as the lowest concentration of drug producing negative subcultures, after plating 20 µl of each clear well after 72 hours incubation at 35 °C on drug-free SDA plate.

Specifically, fungal inocula were prepared from 24-hours (for *Candida sp.)* or 72 hours (for *A. fumigatus*) cultures on SDA plates (Difco, Detroit, MI). The inocula were harvested by harvesting a single colony of yeast into a sterile saline tube. Mold cultures were suspended in plates with sterile saline containing a 0.05 % (v/v) Tween-20 (Difco) suspension and pipeted into sterile tubes and allowed to rest for 30 minutes for the debris to sink down. The supernatant was then transferred to a fresh tube. Both yeast and mold were diluted into RPMI-1640 broth medium to yield a final inoculum concentration of 2X10³ yeast per ml for *Candida* and 2X10⁴ spores per ml for *Aspergillus,* as measured by counting the initial suspension with a hemacytometer. The micro dilution wells, which contained 0.1 ml of the serially diluted drug, were inoculated with 0.1 ml of the resulting suspension. The final inoculum concentration after dilution with the drug suspension was 10³-10⁴ cells per ml. Two wells containing the drug-free medium and inoculum were used as controls. The inoculated plates were incubated at 35 °C for 24 hour (for *Candida sp*.) or 72 hours (for *A. fumigatus*). The growth in each well was then visually estimated. The MICs were determined visually, and were defined as the lowest drug concentration at which there was complete absence of growth (MIC-0).

The results of the antifungal activity of amine-borane compounds are presented in Table 2 below, wherein the MIC values of various compounds against various fungi are given in µmol/liter or mg/liter.
Column A of Table 2 presents the antifungal activity against *Candida albicans* CBS 562;
Column B of Table 2 presents the antifungal activity against *Candida albicans 607;*
Column C of Table 2 presents the antifungal activity against *Candida albicans 615*;
Column D of Table 2 presents the antifungal activity against *Candida glabrata* 4210;
Column E of Table 2 presents the antifungal activity against *Candida glabrata* 4475 (resistant to azoles);
Column F of Table 2 presents the antifungal activity against *Candida glabrata 4681;*
Column G of Table 2 presents the antifungal activity against *Candida glabrata* 4787;
Column H of Table 2 presents the antifungal activity against *Saprolegnia parasitica* T1; and
Column I of Table 2 presents the antifungal activity against *Aspergillus fumigatus* 64026.

As can be seen in Table 2, amine cyanoboranes with diverse structures exhibited considerable antifungal activity. Particularly significant activity was demonstrated with amine cyanoborane compounds such as, for example, Compounds **K-I, K-I₂** and **K-N** (see, entries 9, 11 and 20 respectively in Table 2 below).

### Structure Activity Relationship (SAR) studies:

A number of relationships were observed regarding the effect of the structure of the amine cyanoboranes and carboxyboranes and their derivatives, according to the present invention, on the antifungal activity thereof.

### Effect of length of the N-alkyl chain in alkyldimethylamine cyanoboranes (see, R₈-R₁₀ in Formula III above):

Compound **K-F,** Compound **K-C,** Compound **K-G,** Compound **K-H,** Compound **K-P,** Compound **K-L,** Compound **K-Q,** and Compound **K-R** (see, corresponding entries 6, 5, 7, 8, 22, 18, 23 and 24 in Table 2), have the general structure of RₙNMe₂BH₂C≡N , with varying lengths of the N-alkyl chain (n) in the R=(CH₂)ₙ group.

As can be seen in Table 2, compounds having **a** longer N-alkyl chain exhibited higher antifungal activity. The optimized chain length was for Compound **K-R** (C₁₅ in the N-alkyl group). The chain length was restricted to this length because attempts to use longer chain lengths resulted in solubility problems, as in Compound **K-S,** which has a C₁₇ in the N-alkyl group.

### Effect of length of the N-alkyl-N chain in diamine bis-cyanoboranes for the diamine cyanoboranes (see, A in Formula II above):

Compounds **K-J, K-M,** and **K-N** (see, corresponding entries 13, 19 and 20 in Table 2), have the general structure N≡CBH₂Me₂N-(CH₂)ₙ-NMe₂-BH₂C≡N, with varying N-alkyl-N chain length (n).

As can further be seen in Table 2, compounds having a longer N-alkyl-N chain exhibited higher antifungal activity, until a limit of insolubility is reached. The optimized chain length is exhibited in Compound **K-N** (C₁₄ in the N-alkyl group) as attempts to form compounds with longer chain lengths (C₁₆ and above, data not shown) resulted in solubility problems.

### Effect of halogenating (see, X₁-X₈ in Formulae I, II and III above):

As can be seen in Table 2, bromination of the compounds of the present invention generally enhances the antifungal activity. The bromination of Compound **K-F** to give the dibromo derivative Compound **K-E** enhanced the activity against C. *albicans* CBS 562 from 5100 to 1970 µmol/liter. Also, the bromination of Compound **K-J** to the monobromo derivative Compound **K-J₁** and the dibromo derivative Compound **K-J₂** enhanced the activity against C. *albicans* CBS 562 from 408 µmol/liter for Compound **K-J** to 140 and 104 µmol/liter for Compound **K-J₁** and Compound **K-J₂,** respectively, whereas the bromination or the fluorination of Compound **K-I** to give Compound **K-I₁,** Compound **K-I₂,** and Compound **K-I₄** had no such effect.

### Effect of an unsaturated alkyl chain in alkyldimethylamine cyanoboranes (see, R₈-R₁₀ in Formula III above.):

As can further be seen in Table 2, the presence of unsaturated C=C double bond in the alkyl group dramatically enhanced the activity as seen in Compound **K-G** and Compound **K-D,** where the MIC value against *C*. *albicans* CBS 562 was reduced from 3570 to 470 µmol/L in the presence of a C=C double bond at the end of the alkyl group. For Compound **K-H** and Compound **K-V,** the effect on activity with increased N-alkyl chain length was even greater, taking into account the difference of one methylene (CH₂) unit.

### Effect of conversion of the amine cyanoborane to the amine carboxyborane derivative:

As can be seen in Table 2 below the antifungal activity against C. *albicans* CBS 562 was enhanced from 408 µmol/L for Compound **K-J** (see, entry 13 in Table 2) to 188 µmol/L for its carboxyborane derivative Compound **K-J₃** (see, entry 16 in Table 2).

### Effect of other modifications:

As can further be seen in Table 2, other structural modifications of amine cyanoboranes, such as, for example, addition of a hydroxyl group in one of R₈-R₁₀ in General Formula III as in Compound **K-O,** Compound **K-U,** Compound **K-T,** Compound **K-A** and Compound **K-R,** an aromatic group in one of R₈-R₁₀ as in Compound **K-B,** an unsaturated cyclic group as in Compound **K-A,** and a silyl group as in Compound **K-K,** did not result in significant enhancement in the antifungal activity of the compounds.

As can further be seen in Table 2, the **amine** cyanoboranes and derivatives thereof possess activity against *Candida glabrata* 4475, known to be resistant to azoles (see, column 6 in Table 2 below).

As can be concluded from Table 2 below, the presently most promising compound is dimethyl-undecyl-amine cyanoborane (Compound **K-I)** which was also tested against moulds, typically more resistant to antifungal agents, and exhibited dramatic activity against both *Saprolegnia* and ***Aspergillus.*** Furthermore, Compounds **K-I, K-I₁** and **K-I₃** showed little activity against *Rhizopus oryzae* with MIC of 63 µg/ml.

### Comparisons of antifungal activity to that of conventional antifungal agents against resistant strains:

The novel amine-boranes described herein were further tested for their activity against fungal strains which are known to be resistant to conventional anti-fungal drugs, using the protocol described hereinabove.

Table 3 below summarizes the results of the anti-fungal assays conducted for the presently leading amine cyanoborane Compounds **K-I** and **K-I₂,** as they are reflected by the MIC values of Compounds **K-I** and **K-I₂** against various fungal strains and particularly fungal strains that are resistant to fluconazole namely *Candida globrata* 566, *Candida globrata* 572, *Candida globrata* 578, *Candida globrata* 646, *Candida globrata* 648, *Candida krusei* 603 and *Candida krusei* 638, given in µmol/L or µg/ml and compared to two conventional anti-fungal agents amphotericin B (CAF1) and fluconazole CAF2).

As can be seen in Table 3, Compound **K-I** is highly active against all the tested fungi. As can further be seen in Table 3, isolates of *C. glabrata,* which generate considerably high fluconazole MICs, and isolates of *C. krusei,* which are known as intrinsically resistant to fluconazole, were highly susceptible to the lead Compound **K-I** and its dibromo derivative Compound **K-I₂,** exhibiting much lower MIC values than those obtained with fluconazole. These results strongly suggest that these compounds can become potent antifungal agents, particularly in cases where conventional drugs are involved with development of resistance.

**Table 3**

| | **MIC (µmol/L)** | | | |
|---|---|---|---|---|
| **Fungal Strain** | **K-I** | **K-I₂** | **CAF1** | **CAF2** |
| *Candida globrata 566* | 32.5 | 20.2 | 0.13 | 26.1 |
| *Candida globrata 572* | 32.5 | 40.4 | 1.07 | >836 |
| *Candida globrata 578* | 32.5 | 40.4 | 2.14 | >836 |
| *Candida globrata 646* | 32.5 | 20.2 | 0.41 | 52.2 |
| *Candida globrata 648* | 32.5 | 20.2 | 0.82 | 26.1 |
| *Candida krusei 603* | 16.25 | 10.05 | 4.32 | 104.5^{a} |
| *Candida krusei* 638 | 16.25 | 10.05 | 4.32 | 104.5^{a} |
| *Candida albicans 563* | 2-4^{b} | | | |
| *Cryptococcus neoformans* H-99 | 0.4 ^{b} | | | |
| *Cryptococcus neoformans* B-3501 | 0.8 ^{b} | | | |
| *Saprolegnia parasitica* T-1 | 2-4 ^{b} | | | |
| *Saprolegnia parasitica* CBS 540.67 | 2-4 ^{b} | | | |

| | | | | |
|---|---|---|---|---|
| ^{a} *Candida krusei* is intrinsically resistant to fluconazole; ^{b} Values given in µg/ml. | | | | |

### EXAMPLE 3

### ANTILISHEMANIAN ACTIVITY

Compound **K-I**, an exemplary amine-borane compound according to the present embodiments was further tested for anti-lishmenial activity (against *Leishmania*, a parasitic flagellated protozoan which causes diseases in animals and humans). To this end, various strains of leishmania were grown under optimal conditions, either in Schneiders or in RPMI 1640 medium (Bet-Haemek, Israel), containing 20 % fetal calf serum. Some strains were isolated from humans infected with *L. tropica, L. major* and *L. infantum.* Some strains, including *L. donovani,* were received from the World Health Organization Leishmania Strain Bank which is located in the Kuvin Centre, in the Hebrew University of Jerusalem. These strains depict CL and VL leishmaniasis. Promastigotes and axenic amastigotes were grown as published [28].

The *in vitro* antilishmenial effect of Compound **K-I** was determined on promastigotes (the flagellate stage of the *Leishmania* parasites). Parasites, after 3 or 4 days of growth, were washed and resuspended in fresh medium containing fetal calf serum to a concentration of 15 x 10⁴ promastigotes in 200 µl and distributed into flat bottom wells. The plates were incubated at 28 °C in a wet chamber in an atmosphere of 5 % CO₂, 5 % O₂, and 90 % N₂. After 24 hours, 0.5 µCi of [³H]thymidine was added to each well, the parasites were incubated for additional 24 hours, and then harvested on glass-microfiber filters. Radioactivity was measured in a liquid scintillation counter. Each treatment was performed in triplicate; the results are expressed as percent growth inhibition. [(100 - cpm of wells with drug)/cpm of untreated wells] x 100 (cpm stands for counts per minute), representing the MIC value in µg/ml [Golenser, J. et al., 1999, Antimicrob. Agents Chemother., 43:2209-2214].

The *in vitro* antilishmenial effect of Compound **K-I** was further tested on amastigotes (an intracytoplasmic, nonflagellated form of the *Leishmania* parasites). Peritoneal macrophages were obtained from 10-week-old to 12-week-old outbred mice previously stimulated for 4 or 5 days with 3 % thioglycolate. The macrophages were distributed into 8-well slides to 2.5 x 10⁵ cells in 300 µl per well and incubated at 37 °C and 5 % CO₂ for 3 hours in order to allow for cell adhesion. The supernatant was discarded, and promastigotes (8 x 10⁵ to 10 x 10⁵ per well) were added. After incubation overnight, fresh medium containing Compound **K-I** was added for an additional 24 to 48 hours. At the end of this period, the wells were washed, fixed with methanol, and stained with Giemsa (CAS No.: 51811-82-6). Each treatment was done in duplicate wells; 100 macrophages in each well and the number of amastigotes they contained were counted, representing the MIC value in µg/ml.

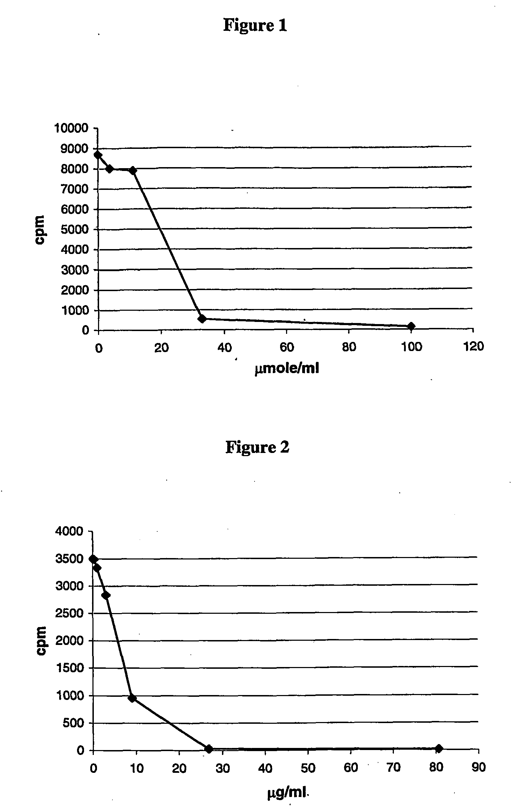

The antilishmenial effect of Compound **K-I** was determined as described above. As can be seen in Figure 1, the measured radioactivity which is indicative of the live parasite diminishes rapidly as a dose of the amine-borane increases, indicating a strong antilishmenial effect. The results of the antilishmenial effect assay are presented in Table 4 below, wherein the concentration of the amine-borane is given in µg/ml versus the counts per minute recorded for the parasite sample.

**Table 4**

| **µg/ml** | **cpm** |
|---|---|
| 0 | 8699 |
| 3.6 | 8003 |
| 11 | 7898 |
| 33 | 537 |
| 100 | 138 |

### EXAMPLE 4

### ANTI-MALARIAL ACTIVITY

Cultures of *P falciparum* were grown in human erythrocytes by standard methods under a low-oxygen atmosphere. The culture medium was RPMI 1640, supplemented with 40 mM HEPES, 25 mg/liter gentamicin sulfate, 10 mM D-glucose, 2 mM glutamine and 8 % heat-inactivated plasma.

*P falciparum* growth was assessed by measuring the incorporation of the radiolabeled nucleic acid precursor [³H]hypoxanthine according to the method described in Desjardins, R.E. et al., 1979, Antimicrob. Agents Chemother., 16: 710-718.

The antimalarial effect of Compound **K-I,** an exemplary amine-borane compound of the present invention was determined as described above. Compound K-I was dissolved in DMSO and eleven samples of concentrations ranging from 0.5 to 100 µg/ml (final concentrations) were distributed in duplicate in 96-well culture plates and dried in a laminar flow hood. The *in vitro* response of the parasite was determined by the isotopic microtest developed by Desjardins *et al..* Infected erythrocytes were suspended in the complete RPMI 1640 medium with 10 % fetal bovine serum at a 1.0-2.5 % hematocrit media. The suspension (200 µl) was distributed into each well. Parasitemia was adjusted to 0.6 % by adding fresh uninfected erythrocytes if the initial parasitemia was ≥1 %. Parasite growth was assessed after incubation at 37°C for 24 hours by adding ³H-hypoxanthine. The cells were incubated for further 15 hours, and then collected by filtration on glass fiber filters and radioactivity was counted. The mean values for parasite control uptake and non-parasitized erythrocyte control uptake of [G-³H]hypoxanthine were calculated from the disintegrations per minute. The IC₅₀ was determined by nonlinear regression fitting of the data using the Sigmaplot computer program.

As can be seen in Figure 2, the measured radioactivity which is indicative of the live parasite diminishes rapidly as a dose of the amine-borane increases, indicating a strong antimalarial effect. The results of the antimalarial effect assay are presented in Table 5 below, wherein the concentration of the amine-borane is given in µg/ml versus the counts per minute recorded for the parasite sample.

**Table 5**

| **µg/ml** | **cpm** |
|---|---|
| 0 | 3500 |
| 0.3 | 3491 |
| 1 | 3335 |
| 3 | 2836 |
| 9 | 959 |
| 26.8 | 33 |
| 80.6 | 13 |

### EX4MPLE 5

### TOXICITY STUDIES

### Acute toxicity studies in vivo:

The acute toxicity of the novel compounds described herein was determined according to the method described by Falk *et al*. [29]. Briefly, male ICR mice weighing about 30 grams were injected through the tail vein with various doses of exemplary amine-borane compounds as presented herein and Fungizone (amphotericin B deoxycholate micellar formulation, Bristol-Myers-Squibb, Dublin, Ireland) as control. Each dosage form (Fungizone 0.1 mg/ml; and exemplary amine-borane compounds 1-2 mg/ml in saline) was administered intravenously as single bolus injections (0.12 ml) of the same dose every 10 minutes to a group of three mice until death was observed. The survival of mice that received the maximal tolerated dose (MTD) was monitored for 8 days.

To study the safety of the compounds of the present invention, the MTD that did not kill ICR mice within the 8 days of the experimental period was determined for two exemplary compounds, namely Compound **K-I** and Compound **K-I₂**. using Fungizone as a control. The average calculated MTD for both compounds were 121.9 and 73.1 µmol/kg, respectively. The MTD for Fungizone was 2.6 µmol/kg. No changes in clinical signs, body weights, and the gross necropsy (on day 8) of mice that received these dosages were observed. These MTD values were well above that of the in vitro MIC values obtained with Compound **K-I** (see, Table 2), and the ratio between the MTD and MIC values are comparable to those obtained with amphotericin B, indicating reduced toxicity and therapeutic potential.

### EXAMPLE 6

### RADIOSYNTHESES

The introduction of a halogen atom during the preparation of the compounds described herein enables to provide radiolabeled aminoboranes and thus harness their biologic activity in a variety of analytical, diagnostic and therapeutic applications which are based on the use of radiolabeled biologically active compounds.

Most practically, the compounds of the present invention can be radiolabeled with radioactive halogens such as ¹⁸F (t_{½} 1.83 hours), ¹²³I (t_{½} 13 hours), ¹²⁵I (t_{½} 60 days) ¹³¹I (t_{½} 8 days), ⁷⁷Br (t_{½} 2.4 days) and the likes.

Handling radioactive materials should be restricted or avoided so as to minimize the exposure of personnel to significant levels of radioactivity. In order to exercise this precaution, radiosyntheses are oftentimes practiced in a fully or semiautomatic procedures. To this end, the present inventors have designed a system for performing such semiautomatic procedure for affording a ¹⁸F-radiolabeled amine-borane, as is detailed hereinbelow.

### Semiautomatic synthesis of ¹⁸F-radiolabeled amine-borane compounds - General Procedure:

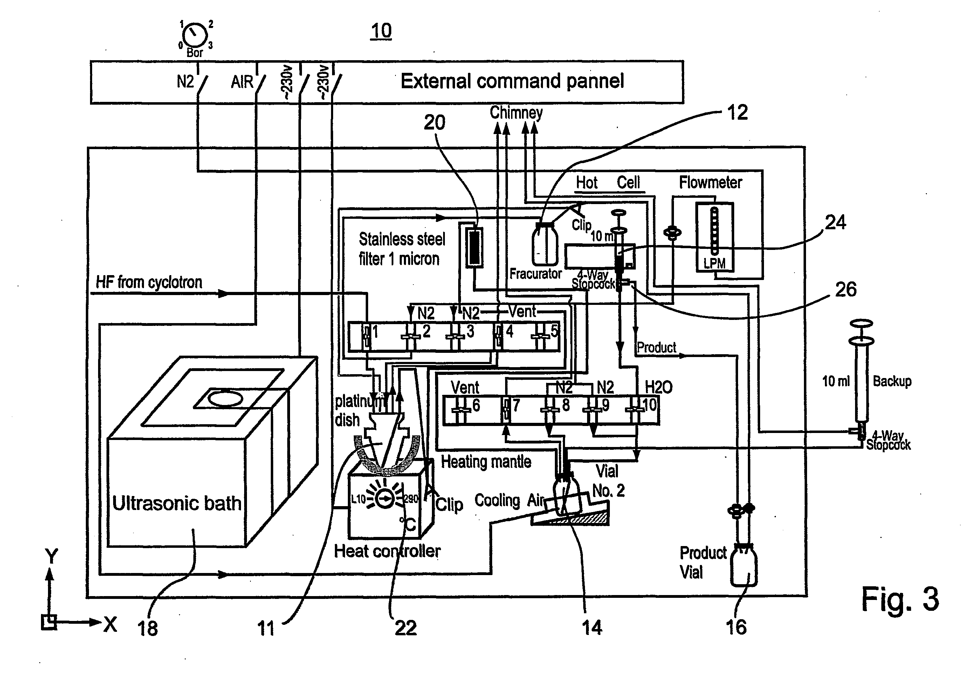

The radiosynthesis of a ¹⁸F-radiolabeled amine-borane is performed using a novel semiautomatic synthesis system designed to suit the reaction conditions, as is schematically illustrated in Figure 3 and is further detailed hereinbelow.

The radiosynthesis is performed in three steps. In the first step, the fluorinating reagent Ag¹⁸F is prepared in a platinum dish. Thereafter the precursor, namely a brominated amine-borane according to the present invention, such as, for example, Compounds **K-E, K-I₁, K-I₂, K-I₃, K-J₁, and K-J₂,** is added under sonication. The obtained [¹⁸F] product is thereafter transferred to another vial, the solvent is removed, and the product is re-dissolved in a physiologic solution (saline).

The setup and flow diagram of semiautomated radio-synthesis system 10 is presented in Figure 3. The semiautomated radiosynthesis of [¹⁸F] labeled amine cyanoboranes is carried out using a modified module which is inserted inside a hot cell (see, Figure 3). All the reaction steps are handled using manipulator arms. Platinum is the element of choice for reaction vessel since platinum can resist both HF and high temperatures. Sonication is necessary for the nucleophilic substitution to occur in the fluorination reaction. Since benzene (the reaction solvent) can dissolve plastic stopcocks, special clips are added to hold the silicon crochets (Degania Silicon Ltd.) from the outside in order to prevent contact of benzene with the plastic stopcocks. Prior to the start of synthesis, Ag₂CO₃ is loaded into platinum reaction vessel **11** that is thereafter closed using a rubber septum (Sigma-Aldrich). Solutions containing the appropriate reagents are loaded into glass vials type 1 (Bunderglass) which are sealed with silicon rubber septa. Precursor vial **12,** intermediate vial **14** and platinum reaction vessel **11** are all equipped with an inlet line for nitrogen gas to pressurize the vial, and silicon line (Degania Silicon Ltd.) outlets for delivery of precursor vial **12** contents to platinum reaction vessel 11 and platinum reaction vessel **11** contents to intermediate vial **14.**

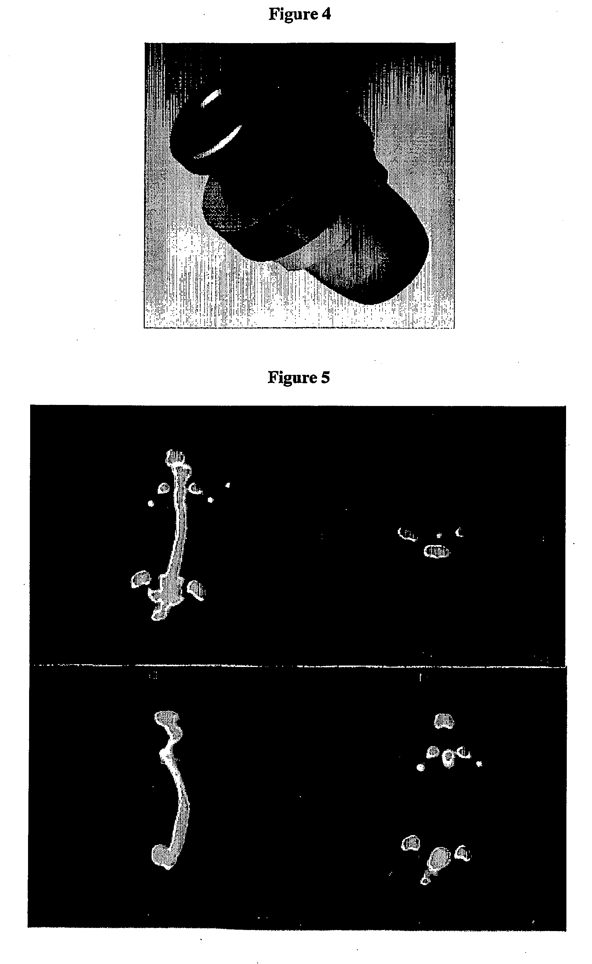

The 7 ml precursor vial **12** is fitted with silicon rubber stopper (ABX). The 12 ml intermediate vial **14** is fitted with latex rubber stopper (West). Product vial 16 is a 25 ml glass vial (Mallinckrodt) which is used to collect the product. The 25 ml platinum reaction vessel 11 is designed to be hermetically sealed with an O-ring and latex septum in order to withstand the high pressure caused by nitrogen (see, Figure 4).

### Semiautomatic synthesis of [¹⁸F]dimethyl-undecyl-amine cyanofluoroborane (Compound [¹⁸F]K-I₄):

The radiosynthesis of [¹⁸F]dimethyl-undecyl-amine cyanofluoroborane (Compound **[¹⁸F]K-I₄),** an exemplary amine-borane according to the present invention, was performed using semiautomated radiosynthesis system 10 (see, Figure 3) as presented hereinabove.

### Materials and Methods:

Dimethyl-undecyl-amine cyanobromoborane (Compound **K-I₁)** was synthesized as described hereinabove.

H₂¹⁸O was purchased from Rotem laboratories.

H¹⁸F was produced using an IBA cyclotron at 10/5 MeV.

Sonication was performed with an (Astrason^{®} 50/60Hz, 1.2 amps).

One hundred mCi of H¹⁸F were dissolved in 1.1 ml of H₂¹⁸O and added to platinum reaction vessel 11 containing 15 mg of Ag₂CO₃. The reaction mixture was thereafter heated to 250 °C to obtain the desired radioactive fluorinating reagent, Ag¹⁸F (see, Scheme 8 below).

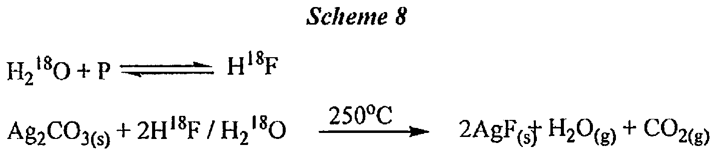

[¹⁸F]Dimethyl-undecyl-amine cyanofluoroborane (Compound **[¹⁸F]K-I₄)** started was prepared by first adding 100 mCi of H¹⁸F dissolved in 1.1 ml of H₂¹⁸O to platinum reaction vessel **11** to dissolve the Ag₂CO₃. Immediately thereafter, the reaction mixture was heated to 250°C for less then 5 minutes to afford the desired Ag¹⁸F as a precipitate. Platinum reaction vessel **11** was transferred to ultrasonic bath **18**, and cooled for 3 minutes. Thereafter, 0.15 mg of dimethyl-undecyl-amine cyanobromoborane (Compound **K-I₁**) dissolved in 4 ml of dry benzene was added to platinum reaction vessel **11** and ultrasonic bath **18** was operated for **50** minutes, which was found to be the optimized time in order to complete the chemical conversion as determined by sampling the reaction on TLC (silica gel F₂₅₄). The product was then transferred and filtered through stainless steel filter 2**0** containing a 0.22 µm Teflon membrane. The nitrogen pressure was set at 1.1 Bar and controlled by a 0.4 liter per minute flow rate into intermediate vial **14.** Intermediate vial 14 was thereafter transferred to heater **22** and the benzene was vaporized at 110 °C for 15 minutes while being vented with nitrogen. Thereafter the vial was sampled once and injected in a GC to verify that no benzene was present. Intermediate vial **14** was then cooled with external air for 3 minutes. Thereafter the product was dissolved in saline (0.9 % NaCl in water) by inserting syringe **24** containing 5 ml saline and injecting the saline into intermediate vial **14** using a needle that reached to the bottom of the vial, and the entire content of the vial was retracted back into syringe **24.** 4-Way stopcock **26** was turned on to transfer the final product into product vial 16 via a 0.22 µm filter as AgF is a solid and does not pass through the 0.22 µm filter.

The final [¹⁸F] labeled dimethyl-undecyl-amine cyanofluoroborane was subjected to a number of quality control measures including assessment of radiochemical purity, γ-detector and pH measurements. Silica TLC tests were performed to assess the radiochemical purity. TLC of the product on silica plates with 100 % methanol demonstrated a single radioactive peak with an R_{f} of 0.23 which was identical to the R_{f} of the non-radioactive dimethyl-undecyl-amine cyanofluoroborane. The ¹⁸F was detected with Wallac Wizard γ-counter with sodium iodide detector and showed 511 KeV. The tested pH of the radioactive [¹⁸F] labeled product solution in saline was 6.3.

The chemical purity was evaluated using radiometric TLC. The radiochemical purity was 100 %. T_{1/2} of ¹⁸F was tested according to the ¹⁸F specification. TLC was performed utilizing Merck silica gel F₂₅₄ plates with a mobile phase of 100 % methanol and analyzed with radiometric detection. The R_{f} of the radio-labeled product was compared with the observed R_{f} of the non-radioactive form. The pH was determined using pH test papers in the range 3.8-5.5 and 6.0-8.1. The ¹⁸F was detected with Wallac Wizard γ-counter with sodium iodide detector.

A 22 % radiochemical yield was obtained in product vial 16 at the end of synthesis with specific radioactivity of 455 mCi/mg. The saline solution of the final product (4.5 mCi/ml) was then shielded to prevent irradiation.

The final product was tested on TLC (silica gel 60 F₂₅₄) after 10 hours and found stable.

### EXAMPLE 7

### PET IMAGING

### Positron emission tomography (PET) imaging using Compound [¹⁸F]K-I₄:

The exemplary [¹⁸F] labeled amine fluorocyanoborane according to the present embodiments, Compound **[¹⁸F]K-I₄**, was injected into normal rats, and was monitored by a PET/CT Philips camera. The images detected the bio-distribution and showed an uptake in the rat's bones.

Four normal, female Sprague-Dawley rats (250-300 grams each) were injected via the tail vein with 50 µCi of Compound **[¹⁸F]K-I₄** solution in saline (4.5 mCi/ml). Fifteen minutes after injection, the rats were anesthetized with 45 mg Ketamine and 24 mg Acepromazin, and after 30 minutes each rat was imaged for 15 minutes.

Figure 5 presents the PET-images obtained, and clearly show uptake of the radiolabeled amine-borane compound into the bones of the tested rats, demonstrating that [¹⁸F] labeling of the amine-borane compounds of the present invention can be practically used as a diagnostic tool and further shed light on their mode of action in the body.

**Table 2**

| **No.** | **Code** | **Name** | **Structure** | **A** | **B** | **C** | **D** | **E** | **F** | **G** | **H** | **I** |
|---|---|---|---|---|---|---|---|---|---|---|---|---|
| 1 | K-A | 1-dimethylaminomethylcyclopent-2-enol cyanoborane | 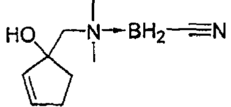 | 1388 | 2776 | 2776 | 500 ^{c} | >500^{c} | | | | |
| 2 | K-B | (2-hydroxy-2-phenylethyl)-dimethyl-amine cyanoborane | 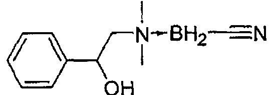 | 245 | >245 | >2450 | >500^{c} | >500^{c} | | | | |
| 3 | K-C | ethyl-dimethyl-amine cyanoborane | 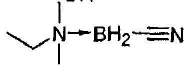 | 4460 | 4460 | 4460 | 500^{c} | 500^{c} | | | | |
| 4 | K-D | but-3-enyl-dimethylamine cyanoborane | 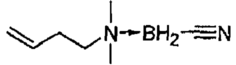 | 470 | 3620 | >3620 | 500^{c} | 500^{c} | | | | >3620 |
| 5 | K-E | trimethyl-amine cyanodibromoborane | 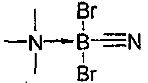 | 1970 | >1970 | >1970 | 500^{c} | >500^{c} | | | | |
| 6 | K-F | trimethyl-amine cyanoborane | 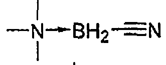 | 5100 | >5100 | >5100 | | | | | | >5100 |
| 7 | K-G | butyl-dimethyl-amine cyanoborane | 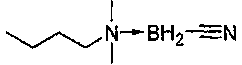 | 3570 | >3570 | >3570 | | | | | | >3570 |
| 8 | K-H | pentyl-dimethyl-amine cyanoborane | 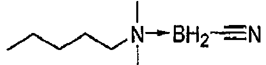 | 810 | 3240 | 3240 | | | | | | 3240 |
| 9 | K-I | dimethyl-undecyl-amine cyanoborane | 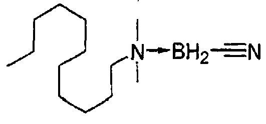 | 32.5 | 32.5 | 32.5 | 7.8^{c} | 15.6^{c} | | | 1-3 | 32.5 |
| 10 | K-11 | dimethyl-undecyl-amine cyanobromoborane | 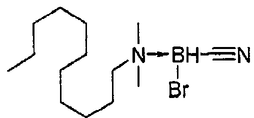 | 49 | 49 | 49 | 7.8^{c} | 7.8^{c} | 7.8^{c} | 15.6 | | 131 |
| 11 | K-I₂ | dimethyl-undecyl-amine cyanodibromoborane | 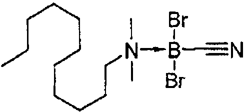 | 39 | 39 | 39 | 15.6^{c} | | 15.6^{c} 32^{c} 32 | | | 79 |
| 12 | K-I₃ | dimethyl-undecyl-amine cyanofluorobromoborane | 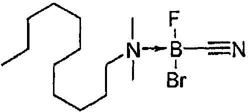 | 7.8 | | | | | | | | |
| 13 | K-J | N,N,N',N'-tetramethyldecane-1,10-diamine cyanoborane | 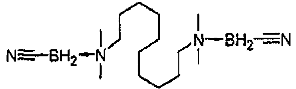 | 408 | 408 | 817 | 32^{c} | 65*^{c}* | | | | 817 |
| 14 | K-J₁ | N,N,N,N-tetramethyldecane-1,10-diamine bis-cyanobromoborane | 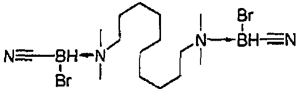 | 140 | 140 | 140 | 65^{c} | 65^{c} | 65^{c} | 65 | | |
| 15 | K-J₂ | N,N,N',N'-tetramethyldecane-1,10-diamine bis-cyanodibromoborane | 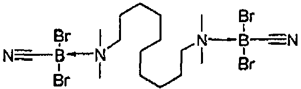 | 104 | 104 | 104 | 65^{c} | 65^{c} | 65^{c} | 65 | | |
| 16 | K-J₃ | N,N,N',N'-tetramethyldecane-1,10-diamine bis-carboxyborane | 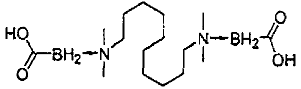 | 188 | 188 | 907 | 65^{c} | 31.2^{c} | 65^{c} | 65 | | |
| 17 | K-K | dimethyltrimethylsilanylmethylamine cyanoborane | 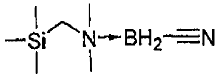 | 2940 | 2940 | 2940 | | | | | | 2940 |
| 18 | K-L | dodecyl-dimethyl-amine cyanoborane | 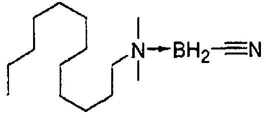 | 61 | 61 | 61 | 65^{c} | 15.6^{c} | 7.8^{c} | 32 | | |
| 19 | K-M | N,N,N',N'-tetramethyldodecane-1,12-diamine bis-cyanoborane | 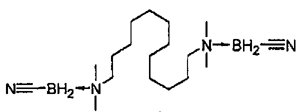 | 96 | 194 | 46 | >250^{c} | >250^{c} | 125^{c} | 250 | | |
| 20 | K-N | N,N,N',N'-tetramethyltetradecane-1,14-diamine bis-cyanoborane | 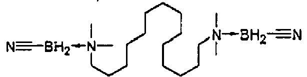 | 43 | 43 | 43 | 7.8^{c} | 15.6^{c} | | | | 86 |
| 21 | K-O | 1-dimethylamino-2-methyl-octan-2-ol cyanoborane | 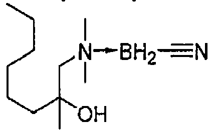 | 2210 | 1100 | 1100 | >250^{c} | >250^{c} | | | | |
| 22 | K-P | dimethyl-nonyl-amine cyanoborane | 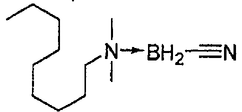 | 150 | 150 | 300 | | | | | | 300 |
| 23 | K-Q | dimethyl-tridecyl-amine cyanoborane | 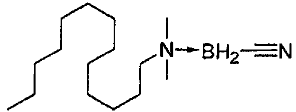 | 58 | 58 | 117 | | | | | | 58 |
| 24 | K-R | dimemyl-pentadecylamine cyanoborane | 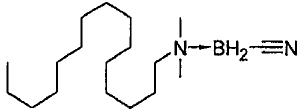 | 53 | 53 | 106 | | | | | | 53 |
| 25 | K-S | heptadecyl-dimethylamine cyanoborane | 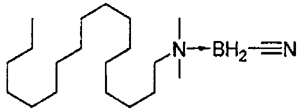 | | | | | | | | | |
| 26 | K-T | 1-dimethylamino-dodecan-2-ol cyanoborane | 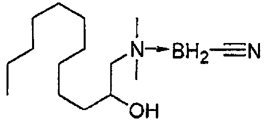 | | | | | | | | | |
| 27 | K-U | 1-dimethylamino-undecan-2-ol cyanoborane | 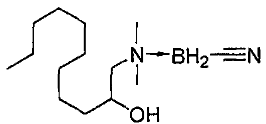 | 492 | 492 | 983 | | | | | | 492 |
| 28 | K-I₄ | dimethyl-undecyl-amine cyanofluoroborane | 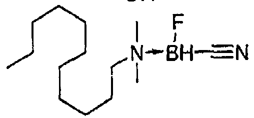 | 64 | 64 | 64 | | | | | | 129 |
| 29 | K-V | hex-5-enyl-dimethylamine cyanoborane | 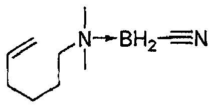 | 94 | 94 | 94 | | | | | | 94 |
| 30 | CAF1 | Amphotericin B | | 0.54 | 0.27 | 0.27 | | | | | | 0.5 |
| 31 | CAF2 | Fluconazole | | 1.6 | 1.6 | 1.6 | | | | | | >208 |

| | | | | | | | | | | | | |
|---|---|---|---|---|---|---|---|---|---|---|---|---|
| ^{c} Values given in µg per liter. | | | | | | | | | | | | |

It is appreciated that certain features of the invention, which are, for clarity, described in the context of separate embodiments, may also be provided in combination in a single embodiment. Conversely, various features of the invention, which are, for brevity, described in the context of a single embodiment, may also be provided separately or in any suitable subcombination.

Although the invention has been described in conjunction with specific embodiments thereof, it is evident that many alternatives, modifications and variations will be apparent to those skilled in the art. Accordingly, it is intended to embrace all such alternatives, modifications and variations that fall within the spirit and broad scope of the appended claims. In addition, citation or identification of any reference in this application shall not be construed as an admission that such reference is available as prior art to the present invention.

### REFERENCES CITED BY NUMERALS

### (Other references are cited in the text)

[1] Hall, I.H.; Starnes, C.O.; Mcphail, A.T.; Wisian-Neilson, P.; Das, M.K. Harchelroad, F. ; Jr. , Spielvogel, B.F. J.Pharm.Sci. 1980, 69(9), 1025.
[2] Kemp, B.; Kalbag, S.; Geanangel, R. A., Inorganic Chemistry (1984), 23(20), 3063-5.
[3] Hall, I. H., Hall, E. S., Chi, L. K., Shaw, B. R., Sood, A. & Spielvogel, B. F. (1992). Antineoplastic activity of boron-containing thymidine nucleosides in Tmolt3 leukemic cells. Anticancer Res 12, 1091-1097.
[4] Miller III, M.C.; A. Sood; Spielvogel, B.F.; Hall, I.H. Appl.Organometal. Chem. 1998, 12, 87.
[5] Hall, I.H.; Starnes, C.O.; Spielvogel, B.F.; Wisian-Neilson, P.; Das, M.K.; Wojnowich, L.J.Pharm.Sci.1979, 68(6),685.
[6] Spielvogel, B.F.; Wojnowich, L.; Das, M.K.; McPhail, A.T.; Hargrave, K.D.; J.Am. Chem Soc.1976, 98(18), 5702.
[7] Miller III, M.C.; Sood, A., Spielvogel, B.F.; Hall, I.H. Arch.Pharm.Med. Chem 1998, 331,153.
[8] Miller III, M.C.; Woods, C.M.; Murphy, M.E. ; Elkins, A.; Spielvogel, B.F.; Hall, I.H. Biomed. & Pharmacother 1998, 52,169.
[9] Hall, I. H., Chen, S. Y., Rajendran, K. G., Sood, A., Spielvogel, B. F. & Shih, J. (1994). Hypolipidemic, anti-obesity, anti-inflammatory, anti-osteoporotic, and anti-neoplastic properties of amine carboxyboranes. Environ Health Perspect 102 Suppl 7,21-30.
[10] National Committee for Clinical Laboratory Standards (1997). National Committee for Clinical Laboratory Standards Reference method for broth dilution antifungal susceptibility testing of yeasts. (M27-A, A. s., Ed), Wayne, Pa.
[11] Spielvogel, B.F.; Das, M.K.; McPhail, A.T.; Onan, K.D.; Hall, I.H., J.Am. Chemt.Soc.1980,102, 6344.
[12] Murphy, M.E.; Elkins, A.L.; Shrewsbury, R.P.; Sood, A.; Speilvogel, B.F. ; Hall, I.H. Metal Based Drugs 1996, 3, 31.
[13] Hall, I.H.; Burnham, B.S.; Chen, S.Y.; Sood, A.; Spielvogel, B.F., Morse, K.W. Metal Based Drugs 1995, 2(1),1.
[14] Hall, I.H.; Williams, W.L.; Jr.; Gilbert, C.J.; McPhail, A.T.; Spielvogel, B.F. J.Pharm. Sci. 1980,73(7), 973.
[15] Hall, I.H.; Gilbert, C.J.; McPhail, A.T.; Morse, K.W.; Hassett, K.; Speilvogel, B.F. J.Pharm. Sci. 1985, 74(7),755.
[16] Sood, C.K.; Sood, A.; Speilvogel, B.F.; Yousef, J.A.; Burnham, B.; Hall, I.H. J.Pharm.Sci.1991, 80(12),1133.
[17] Dembitsky, V.M.; Srebnik, M., Tetrahedron 2003, 59,579.
[18] Spielvogel B F; Sood A; Morse K W; Wong O T; Hall I H, Die Pharmazie (1991 Aug), 46(8), 592-4.
[19] Hall, I. H.; Das, M. K.; Hacchelroad, F., Jr.; Wisian-Neilson, P.; McPhail, A. T.; Spielvogel, B. F., Journal of Pharmaceutical Sciences (1981), 70(3), 339-41
[20] Hall, Iris H.; Burnham, Bruce S.; Chen, Shang Y.; Sood, Anup; Spielvogel, Bernard F.; Morse, Karen W., Metal-Based Drugs (1995), 2(1), 1-12
[21] Hall, Iris H.; Spielvogel, Bernard F.; Sood, Anup; Ahmed, Fahim; Jafri, Samir, Journal of Pharmaceutical Sciences (1987), 76(5), 359-65.
[22] Takrouri et al., Organometallics (2004), 23(11), 2817-2820.
[23] Berdy, J., Handbook of Antibiotic Compounds. Part IV, CRC: Boca Raton, Florida, 1980.
[24] Fink, K., Fink, R. M., Science, 1948, 108, 358-9.
[25] Hunt, S., Methods Enzymol. 1984, 107, 413-438.
[26] Jimenez, E. C., Craig, A. G., Watkins, M., Hillyard, D. R., Gray, W. R., Gulyas, J., Rivier, J. E., Cruz, L. J., Olivera, B. M., Biochemistry 1997, 36, 984-989.
[27] National Committee for Clinical Laboratory Standards (2002). Reference method for broth dilution antifungal susceptibility testing of filamentous fungi. In: Approved Standard - Second Edition M38-A). National Committee for Clinical Laboratory Standards, Wayne, Pa.
[28] Ephros M, Waldman E, Zilberstein D. 1997. Pentostam induces resistance to antimony and the preservative chlorocresol in L. donovani promastigotes and axenically grown amastigotes. Antimicrob Agents Chemother. 41: 1064-68.
[29] Falk, R.; Domb, A. J.; Polacheck, I. A novel injectable water-soluble amphotericin B-arabinogalactan conjugate. Antimicrob. Agents Chemother. 1999, 43, 1975-1981.

## Claims

1. A compound having a general formula selected from the group consisting of Formula I, II and III: 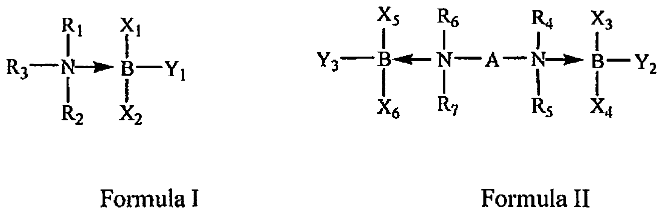 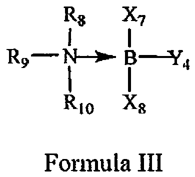 or a pharmaceutically acceptable salt thereof,
wherein:
A is a substituted or non-substituted, saturated or non-saturated hydrocarbon having from 5 to 20 carbon atoms;
Y₁-Y₄ are each independently selected from the group consisting of a cyano group (-C≡N), a -C(=O)Ra group, amine and alkyl, whereas Ra is hydrogen, halogen, hydroxy, alkoxy, thiohydroxy, thioalkoxy, aryloxy, thioaryloxy, thiol and amine;
X₁-X₈ are each independently selected from the group consisting of hydrogen, alkyl, halogen, cycloalkyl, and aryl, provided that at least one of X₁ and X₂ in Formula I is a non-radioactive or radioactive fluorine; and
R₁-R₁₀ are each independently selected from the group consisting of hydrogen, alkyl, alkenyl, alkynyl, cycloalkyl and aryl or, alternatively, two of R₁-R₃, R₄ and R₅ R₆ and R₇ and/or R₈-R₁₀ form a carbocyclic ring, provided that:
at least one of R₈-R₁₀ in Formula III is a saturated alkyl having 11-15carbon atoms;
at least one of R₈-R₁₀ in Formula III is an unsaturated alkyl having 5-20 carbon atoms; and/or
at least one of R₈-R₁₀ in Formula III comprises at least one non-radioactive or radioactive fluorine.

2. The compound of claim 1, having the general Formula I.

3. The compound of claim 2, wherein Y₁ is selected from the group consisting of a cyano group -(C≡N) and a -C(=O)Ra group.

4. The compound of claim 2, wherein X₁ is fluorine and X₂ is selected from the group consisting of hydrogen, fluorine, bromine and iodine.

5. The compound of claim 2, wherein each of R₁-R₃ is alkyl.

6. The compound of claim 2, wherein at least one of R₁-R₃ is a C₅-C₂₀ alkyl.

7. The compound of claim 2, being selected from the group consisting of dimethyl-undecyl-amine cyanofluorobromoborane, trimethyl-amine cyanofluoroborane, ethyl-dimethyl-amine cyanofluoroborane, butyl-dimethyl-amine cyanofluoroborane, trimethyl-amine carboxyfluoroborane methyl ester, trimethylamine carboxyfluoroborane ethyl ester, ethyl-dimethyl-amine carboxyfluoroborane methyl ester, butyl-dimethyl-amine carboxyfluoroborane methyl ester, trimethylamine cyanodifluoroborane, trimethyl-amine carboxydifluoroborane methyl ester, trimethyl-amine carboxydifluoroborane ethyl ester, trimethyl-amine cyanofluorobromoborane, trimethyl-amine carboxyfluorobromoborane ethyl ester and triethyl-amine carboxydifluoroborane.

8. The compound of claim 1, having the general Formula II.

9. The compound of claim 8, wherein at least one of X₃, X₄, X₅ and X₆ is bromine.

10. The compound of claim 8, being selected from the group consisting of N,N,N',N'-tetramethyl-decane-1,10-diamine bis-cyanoborane, N,N,N',N'-tetramethyl-decane-1,10-diamine bis-cyanobromoborane, N,N,N',N'-tetramethyl-decane-1,10-diamine bis-cyanodibromoborane, N,N,N',N'-tetramethyl-decane-1,10-diamine bis-carboxyboranes, N,N,N',N'-tetramethyl-dodecane-1,12-diamine bis-cyanoborane and N,N,N',N'-tetramethyl-tetradecane-1,14-diamine bis-cyanoborane.

11. The compound of claim 1, having the general Formula III.

12. The compound of claim 11, wherein at least one of R₈-R₁₀ comprises a hydroxy, whereas said hydroxy is at position β to the amine nitrogen.

13. The compound of claim 11, wherein at least one of R₈-R₁₀ comprises a fluorine, whereas said fluorine is at position β to the amine nitrogen.

14. The compound claim 11, wherein at least one of X₇ and X₈ is bromine.

15. The compound of claim 11, being selected from the group consisting of dimethyl-undecyl-amine cyanoborane, dimethyl-undecyl-amine cyanobromoborane, dimethyl-undecyl-amine cyanodibromoborane, dodecyl-dimethyl-amine cyanoborane, 1-dimethylamino-2-methyl-octan-2-ol cyanoborane, dimethyl-nonyl-amine cyanoborane, dimethyl-tridecyl-amine cyanoborane, dimethyl-pentadecyl-amine cyanoborane, heptadecyl-dimethyl-amine cyanoborane, 1-dimethylamino-dodecan-2-ol cyanoborane, 1-dimethylamino-undecan-2-ol cyanoborane, dimethyl-undecyl-amine cyanofluorobromoborane, and (2-fluoro-nonyl)-dimethyl-amine cyanoborane.

16. A pharmaceutical composition comprising, as an active ingredient, the compound of any of claims 1-15 and a pharmaceutically acceptable carrier.

17. Use of the compound of any of claims 1-15 as a medicament.

18. The use of claim 17, wherein said medicament is for the treatment of a medical condition associated with a pathogenic microorganism.

19. The use of claim 18, wherein said pathogenic microorganism is a drug-resistant microorganism.

20. A process of preparing the compound of claim 2, the process comprising:
reacting a compound having the general Formula IV: 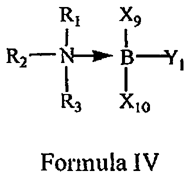 or a pharmaceutically acceptable salt thereof,
wherein:
Y₁ is selected from the group consisting of a cyano group -(C≡N), a -C(=O) Ra group, amine and alkyl, whereas Ra is hydrogen, halo, hydroxy, alkoxy, thiohydroxy, thioalkoxy, aryloxy, thioaryloxy, thiol, and amine;
X₉ and X₁₀ are each independently selected from the group consisting of hydrogen, alkyl, halogen, cycloalkyl, aryl, provided that at least one of X₉ and X₁₀ is bromine; and
R₁-R₃ are each independently selected from the group consisting of hydrogen, alkyl, cycloalkyl and aryl or, alternatively, two of R₁-R₃ form a carbocyclic ring,
with a fluorinating agent, thereby obtaining the compound having general Formula I.

21. A process of preparing the compound of claim 8, the process comprising:
reacting a compound having the general Formula V: 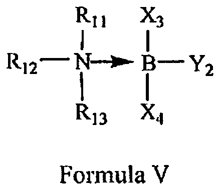 or a pharmaceutically acceptable salt thereof,
wherein:
Y₂ is selected from the group consisting of a cyano group -(C≡N), a -C(=O) Ra group, amine and alkyl, whereas Ra is hydrogen, halogen, hydroxy, alkoxy, thiohydroxy, thioalkoxy, aryloxy, thioaryloxy, thiol and amine;
X₃ and X₄ are each independently selected from the group consisting of hydrogen, alkyl, halogen, cycloalkyl and aryl; and
R₁₁-R₁₃ are each independently selected from the group consisting of hydrogen, alkyl, cycloalkyl and aryl or, alternatively, two of R₁₁-R₁₃ form a carbocyclic ring, provided that at least one of R₁₁-R₁₃ in Formula V is methyl,
with a compound having the general Formula VI:
W₁-A-W₂ Formula VI
wherein:
A is a substituted or non-substituted, saturated or non-saturated hydrocarbon having from 5 to 20 carbon atoms; and
W₁ and W₂ are each independently a functional group,
in the presence of n-alkyl lithium, thereby obtaining the compound having general Formula II.

22. A process of preparing the compound of claim 11, wherein at least of R₈-R₁₀ is said saturated alkyl having 11-15 carbon atoms or an unsaturated alkyl having 5-20 carbon atoms, the process comprising:
reacting a compound having the general Formula V: 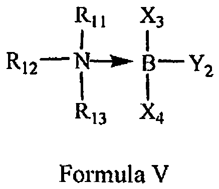 or a pharmaceutically acceptable salt thereof,
wherein:
Y₂ is selected from the group consisting of a cyano group -(C≡N), a -C(=O)Ra group, amine and alkyl, whereas Ra is hydrogen, halogen, hydroxy, alkoxy, thiohydroxy, thioalkoxy, aryloxy, thioaryloxy, thiol and amine;
X₃ and X₄ are each independently selected from the group consisting of hydrogen, alkyl, halogen, cycloalkyl and aryl; and
R₁₁-R₁₃ are each independently selected from the group consisting of hydrogen, alkyl, cycloalkyl and aryl or, alternatively, two of R₁₁-R₁₃ form a carbocyclic ring, provided that at least one of R₁₁-R₁₃ in Formula V is methyl,
with a compound having the general Formula VII:
R₁₄-W₃ Formula VII
wherein:
R₁₄ is selected from the group consisting of saturated alkyl having at least 10 carbon atoms or an unsaturated alkyl having at least 4 carbon atoms; and
W₃ is a functional group,
in the presence of n-alkyl lithium, thereby obtaining the compound having general Formula III.

23. A process of preparing the compound of claim 11, wherein at least of R₈-R₁₀ comprises a fluorine, the process comprising:
reacting a compound having the general Formula V: 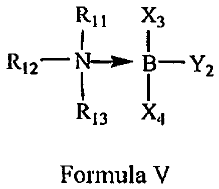 or a pharmaceutically acceptable salt thereof,
wherein:
Y₂ is selected from the group consisting of a cyano group -(C≡N), a -C(=O) Ra group, amine and alkyl, whereas Ra is hydrogen, halogen, hydroxy, alkoxy, thiohydroxy, thioalkoxy, aryloxy, thioaryloxy, thiol and amine;
X₃ and X₄ are each independently selected from the group consisting of hydrogen, alkyl, halogen, cycloalkyl and aryl; and
R₁₁-R₁₃ are each independently selected from the group consisting of hydrogen, alkyl, cycloalkyl and aryl or, alternatively, two of R₁₁-R₁₃ form a carbocyclic ring, provided that at least one of R₁₁-R₁₃ in Formula V is methyl,
with a compound having the general Formula VIII:
R₁₅-W₄-R₁₆ Formula VIII
wherein:
W₄ is a carboxy (C=O) group;
R₁₅ is selected from the group consisting of alkyl, cycloalkyl, and aryl; and
R₁₆ is selected from the group consisting of hydrogen, alkyl, cycloalkyl and aryl,
in the presence of n-alkyl lithium, to thereby obtaining a compound having said general Formula III, wherein at least one of R₈-R₁₀ comprises a hydroxy or alkoxy; and
converting said hydroxy or said alkoxy to said fluorine.

24. A radiolabeled compound having the general Formula X or XI: 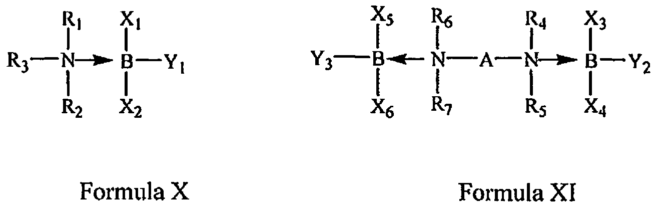 or a pharmaceutically acceptable salt thereof,
wherein:
Y₁-Y₃ are each independently selected from the group consisting of a cyano group (-C≡N), a -C(=O) Ra group, amine and alkyl, whereas Ra is hydrogen, halogen, hydroxy, alkoxy, thiohydroxy, thioalkoxy, aryloxy, thioaryloxy, thiol and amine;
X₁-X₆ are each independently selected from the group consisting of hydrogen, alkyl, halogen, cycloalkyl and aryl,
R₁-R₇ are each independently selected from the group consisting of hydrogen, alkyl, cycloalkyl and aryl or, alternatively, two of R₁-R₃, R₄ and R₅ and/or R₆ and R₇ form a carbocyclic ring, and
A is a substituted or non-substituted, saturated or non-saturated hydrocarbon having from 5 to 20 carbon atoms,
whereas at least one of X₁-X₆, R₁-R₇ and A comprises a radioactive atom and/or at least one boron atom is a radioactive boron atom.

25. A pharmaceutical composition comprising, as an active ingredient, the radiolabeled compound of claim 24 and a pharmaceutically acceptable carrier.

26. Use of the radiolabeled compound of claim 24 or of the composition of claim 25 in radioimaging and/or radiotherapy.

27. Use of the radiolabeled compound of claim 24 in the preparation of a diagnostic agent.

## Patentansprüche

1. Verbindung einer allgemeinen Formel, ausgewählt aus der Gruppe bestehend aus Formel I, II und III: 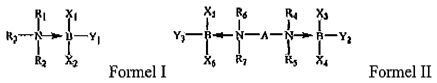 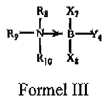 oder ein pharmazeutisch akzeptables Salz davon,
wobei:
A ein substituierter oder nicht substituierter, gesättigter oder nicht gesättigter Kohlenwasserstoff mit 5 bis 20 Kohlenstoffatomen ist;
Y₁-Y₄ jeweils unabhängig aus der Gruppe bestehend aus einer Cyanogruppe (-C=N), einer -C(=O)Ra-Gruppe, Amin und Alkyl ausgewählt sind, wobei Ra Wasserstoff, Halogen, Hydroxy, Alkoxy, Thiohydroxy, Thioalkoxy, Aryloxy, Thioaryloxy, Thiol und Amin ist;
X₁-X₈ jeweils unabhängig aus der Gruppe bestehend aus Wasserstoff, Alkyl, Halogen, Cycloalkyl und Aryl ausgewählt sind, unter der Maßgabe, dass zumindest eines von X₁ und X₂ in Formel I ein nicht radioaktives oder radioaktives Fluor ist; und
R₁-R₁₀ jeweils unabhängig aus der Gruppe bestehend aus Wasserstoff, Alkyl, Alkenyl, Alkynyl, Cycloalkyl und Aryl ausgewählt sind oder alternativ zwei von R₁-R₃, R₄ und R₅, R₆ und R₇ und/oder R₈-R₁₀ einen carbozyklischen Ring bilden, unter der Maßgabe, dass:
zumindest eines von R₈-R₁₀ in Formel III ein gesättigtes Alkyl mit 11 bis 15 Kohlenstoffatomen ist;
zumindest eines von R₈-R₁₀ in Formel III ein nicht gesättigtes Alkyl mit 5 bis 20 Kohlenstoffatomen ist; und/oder
zumindest eines von R₈-R₁₀ in Formel III zumindest ein nicht radioaktives oder radioaktives Fluor enthält.

2. Verbindung nach Anspruch 1 der allgemeinen Formel I.

3. Verbindung nach Anspruch 2, wobei Y₁ aus der Gruppe bestehend aus einer Cyanogruppe -(C≡N) und einer -C(=O)Ra-Gruppe ausgewählt ist.

4. Verbindung nach Anspruch 2, wobei X₁ Fluor ist, und wobei X₂ aus der Gruppe bestehend aus Wasserstoff, Fluor, Brom und Jod ausgewählt ist.

5. Verbindung nach Anspruch 2, wobei R₁-R₃ jeweils Alkyl ist.

6. Verbindung nach Anspruch 2, wobei zumindest eines von R₁-R₃ ein C₅-C₂₀-Alkyl ist.

7. Verbindung nach Anspruch 2, ausgewählt aus der Gruppe bestehend aus Dimethylundecylamincyanofluorbromboran, Trimethylamincyanofluorboran, Ethyldimethylamincyanofluorboran, Butyldimethylamincyanofluorboran, Trimethylamincarboxyfluorboranmethylester, Trimethylamincarboxyfluorboranethylester, Ethyldimethylamincarboxyfluorboranmethylester, Butyldimethylamincarboxyfluorboranmethylester, Trimethylamincyanodifluorboran, Trimethylamincarboxydifluorboranmethylester, Trimethylamineafboxydifluorboranethylester, Trimethylamincyanofluorbromboran, Trimethylamincarboxyfluorbromboranethylester und Triethylamincarboxydifluorboran.

8. Verbindung nach Anspruch 1 der allgemeinen Formel II.

9. Verbindung nach Anspruch 8, wobei zumindest eines von X₃, X₄, X₅ und X₆ Brom ist.

10. Verbindung nach Anspruch 8, ausgewählt aus der Gruppe bestehend aus N,N,N',N'-tetramethyldecan-1,10-diaminbiscyanoboran, N,N,N',N'-tetramethyldecan-1,10-diaminbiscyanobromboran, N,N,N',N'-tetramethyldecan-1,10-diaminbiscyanodibromboran, N,N,N',N'-tetramethyldecan-1,10-diaminbiscarboxyboranen, N,N,N',N'-tetramethyldodecan-1,12-diaminbiscyanoboran und N,N,N`,N`-tetramethyltetradecan-1,14-diaminbiscyanoboran.

11. Verbindung nach Anspruch 1 der allgemeinen Formel III.

12. Verbindung nach Anspruch 11, wobei zumindest eines von R₈-R₁₀ ein Hydroxy umfasst, wobei das Hydroxy an Position β zum Aminstickstoff ist.

13. Verbindung nach Anspruch 11, wobei zumindest eines von R₈-R₁₀ ein Fluor umfasst, wobei das Fluor an Position β zum Aminstickstoff ist.

14. Verbindung nach Anspruch 11, wobei zumindest eines von X₇ und X₈ Brom ist.

15. Verbindung nach Anspruch 11, ausgewählt aus der Gruppe bestehend aus bimethylundecylamincyanoboran, Dimethylundecylamincyanobromboran, Dimethylundecylamincyanodibromboran, Dodecyldimethylamincyanoboran, 1-Dimethylamino-2-methyloktan-2-ol-cyanoboran, Dimethylnonylamincyanoboran, Dimethyltridecylamincyanoboran, Dimethylpentadecylamincyanoboran, Heptadecyldimethylamincyanoboran, 1-Dimethylaminododecan-2-ol-cyanoboran, 1-Dimethylaminoundecan-2-ol-cyanoboran, Dimethylundecylamincyanofluorbromboran und (2-Fluornonyl)-dimethylamincyanoboran.

16. Pharmazeutische Zusammensetzung, umfassend als Wirkstoff die Verbindung nach einem der Ansprüche 1 bis 15 und einen pharmazeutisch akzeptablen Träger.

17. Verwendung der Verbindung nach einem der Ansprüche 1 bis 15 als Medikament.

18. Verwendung nach Anspruch 17, wobei das Medikament zur Behandlung einer medizinischen Erkrankung vorgesehen ist, die mit einem pathogenen Mikroorganismus assoziiert ist.

19. Verwendung nach Anspruch 18, wobei der pathogene Mikroorganismus ein arzneimittelresistenter Mikroorganismus ist.

20. Verfahren zum Herstellen der Verbindung nach Anspruch 2, wobei das Verfahren umfasst:
Reagieren einer Verbindung der allgemeinen Formel IV: 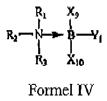 oder eines pharmazeutisch akzeptablen Salzes davon,
wobei:
Y₁ aus der Gruppe bestehend aus einer Cyanogruppe -(C≡N), einer -C(=O)Ra-Gruppe, Amin und Alkyl ausgewählt ist, wobei Ra Wasserstoff, Halo, Hydroxy, Alkoxy, Thiohydroxy, Thioalkoxy, Ayloxy, Thioaryloxy, Thiol und Amin ist;
X₉ und X₁₀ jeweils unabhängig aus der Gruppe bestehend aus Wasserstoff, Alkyl, Halogen, Cycloalkyl, Aryl ausgewählt sind, unter der Maßgabe, dass zumindest eines von X₉ und X₁₀ Brom ist; und
R₁-R₃ jeweils unabhängig aus der Gruppe bestehend aus Wasserstoff, Alkyl, Cycloalkyl und Aryl ausgewählt sind, oder alternativ zwei von R₁-R₃ einen carbozyklischen Ring bilden,
mit einem Fluorierungsmittel, wodurch die Verbindung der allgemeinen Formel I erhalten wird.

21. Verfahren zum Herstellen der Verbindung nach Anspruch 8, wobei das Verfahren umfasst:
Reagieren einer Verbindung der allgemeinen Formel V: 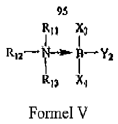 oder eines pharmazeutisch akzeptablen Salzes davon,
wobei:
Y₂ aus der Gruppe bestehend aus einer Cyanogruppe -(C=N), einer -C(=O)Ra-Gruppe, Amin und Alkyl ausgewählt ist, wobei Ra Wasserstoff, Halogen, Hydroxy, Alkoxy, Thiohydroxy, Thioalkoxy, Aryloxy, Thioaryloxy, Thiol und Amin ist;
X₃ und X₄ jeweils unabhängig aus der Gruppe bestehend aus Wasserstoff, Alkyl, Halogen, Cycloalkyl und Aryl ausgewählt sind; und
R₁₁-R₁₃ jeweils unabhängig aus der Gruppe bestehend aus Wasserstoff, Alkyl, Cycloalkyl und Aryl ausgewählt sind oder alternativ zwei von R₁₁-R₁₃ einen carbozyklischen Ring bilden, unter der Maßgabe, dass zumindest eines von R₁₁-R₁₃ in Formel V Methyl ist,
mit einer Verbindung der allgemeinen Formel VI:
W₁-A-W₂ Formel VI
wobei:
A ein substituierter oder nicht substituierter, gesättigter oder nicht gesättigter Kohlenwasserstoff mit 5 bis 20 Kohlenstoffatomen ist; und
W₁ und W₂ jeweils unabhängig eine funktionelle Gruppe sind,
in der Gegenwart von n-Alkyllithium, wodurch die Verbindung der allgemeinen Formel II erhalten wird.

22. Verfahren zum Herstellen der Verbindung nach Anspruch 11, wobei zumindest eines von R₈-R₁₀ das gesättigte Alkyl mit 11 bis 15 Kohlenstoffatomen oder ein nicht gesättigtes Alkyl mit 5 bis 20 Kohlenstoffatomen ist, wobei das Verfahren umfasst:
Reagieren einer Verbindung der allgemeinen Formel V: 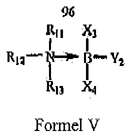 oder eines pharmazeutisch akzeptablen Salzes davon,
wobei:
Y₂ aus der Gruppe bestehend aus einer Cyanogruppe -(C≡N), einer -C(=O)Ra-Gruppe, Amin und Alkyl ausgewählt ist, wobei Ra Wasserstoff, Halogen, Hydroxy, Alkoxy, Thiohydroxy, Thioalkoxy, Aryloxy, Thioaryloxy, Thiol und Amin ist;
X₃ und X₄ jeweils unabhängig aus der Gruppe bestehend aus Wasserstoff, Alkyl, Halogen, Cycloalkyl und Aryl ausgewählt sind; und
R₁₁-R₁₃ jeweils unabhängig aus der Gruppe bestehend aus Wasserstoff, Alkyl, Cycloalkyl und Aryl ausgewählt sind oder alternativ zwei von R₁₁-R₁₃ einen carbozyklischen Ring bilden, unter der Maßgabe, dass zumindest eines von R₁₁-R₁₃ in Formel V Methyl ist,
mit einer Verbindung der allgemeinen Formel VII:
R₁₄-W₃ Formel VII
wobei:
R₁₄ aus der Gruppe bestehend aus einem gesättigten Alkyl mit zumindest 10 Kohlenstoffatomen oder einem nicht gesättigten Alkyl mit zumindest 4 Kohlenstoffatomen ausgewählt ist; und
W₃ eine funktionelle Gruppe ist,
in der Gegenwart von n-Alkyllithium, wodurch die Verbindung der allgemeinen Formel III erhalten wird.

23. Verfahren zum Herstellen der Verbindung nach Anspruch 11, wobei zumindest eines von R₈-R₁₀ ein Fluor umfasst, wobei das Verfahren umfasst:
Reagieren einer Verbindung der allgemeinen Formel V: 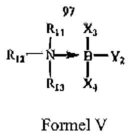 oder eines pharmazeutisch akzeptablen Salzes davon,
wobei:
Y₂ aus der Gruppe bestehend aus einer Cyanogruppe -(C≡N), einer -C(=O)Ra-Gruppe, Amin und Alkyl ausgewählt ist, wobei Ra Wasserstoff, Halogen, Hydroxy, Alkoxy, Thiohydroxy, Thioalkoxy, Aryloxy, Thioaryloxy, Thiol und Amin ist;
X₃ und X₄ jeweils unabhängig aus der Gruppe bestehend aus Wasserstoff, Alkyl, Halogen, Cycloalkyl und Aryl ausgewählt sind; und
R₁₁-R₁₃ jeweils unabhängig aus der Gruppe bestehend aus Wasserstoff, Alkyl, Cycloalkyl und Aryl ausgewählt sind oder alternativ zwei von R₁₁-R₁₃ einen carbozyklischen Ring bilden, unter der Maßgabe, dass zumindest eines von R₁₁-R₁₃ in Formel V Methyl ist,
mit einer Verbindung der allgemeinen Formel VIII:
R₁₅-W₆-R₁₆ Formel VIII
wobei:
W₄ eine Carboxy-(C=O)-Gruppe ist;
R₁₅ aus der Gruppe bestehend aus Alkyl, Cycloalkyl und Aryl ausgewählt ist; und
R₁₆ aus der Gruppe bestehend aus Wasserstoff, Alkyl, Cycloalkyl und Aryl ausgewählt ist,
in der Gegenwart von n-Alkyllithium, um somit eine Verbindung der allgemeinen Formel III zu erhalten, wobei zumindest eines von R₈-R₁₀ ein Hydroxy oder Alkoxy umfasst; und
Umsetzen des Hydroxy oder des Alkoxy zu Fluor.

24. Radioaktiv markierte Verbindung der allgemeinen Formel X oder XI: 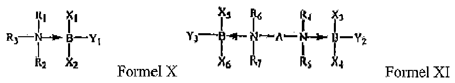 oder ein pharmazeutisch akzeptables Salz davon,
wobei:
Y₁-Y₃ jeweils unabhängig aus der Gruppe bestehend aus einer Cyanogruppe (-C≡N), einer -C(=O)Ra-Gruppe, Amin und Alkyl ausgewählt sind, wobei Ra Wasserstoff, Halogen, Hydroxy, Alkoxy, Thiohydroxy, Thioalkoxy, Aryloxy, Thioaryloxy, Thiol und Amin ist;
X₁-X₆ jeweils unabhängig aus der Gruppe bestehend aus Wasserstoff, Alkyl, Halogen, Cycloalkyl und Aryl ausgewählt sind,
R₁-R₇ jeweils unabhängig aus der Gruppe bestehend aus Wasserstoff, Alkyl, Cycloalkyl und Aryl ausgewählt sind oder alternativ zwei von R₁-R₃, R₄ und R₅ und/oder R₆ und R₇ einen carbozyklischen Ring bilden, und
A ein substituierter oder nicht substituierter, gesättigter oder nicht gesättigter Kohlemvasserstoff mit 5 bis 20 Kohlenstoffatomen ist,
wobei zumindest eines von X₁-X₆, R₁-R₇ und A ein radioaktiv markiertes Atom enthält und/oder zumindest ein Boratom ein radioaktives Boratom ist.

25. Pharmazeutische Zusammensetzung, umfassend als Wirkstoff die radioaktiv markierte Verbindung nach Anspruch 24 und einen pharmazeutisch akzeptablen Träger.

26. Verwendung der radioaktiv markierten Verbindung nach Anspruch 24 oder der Zusammensetzung nach Anspruch 25 bei der Strahlenbildgebung und/oder Strahlentherapie.

27. Verwendung der radioaktiv markierten Verbindung nach Anspruch 24 bei der Herstellung eines Diagnostikums.

## Revendications

1. Composé ayant une formule générale choisie dans le groupe constitué des Formules I, II et III : 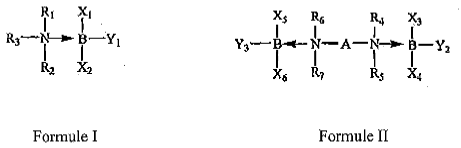 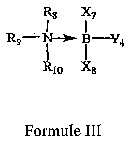 ou sel pharmaceutiquement acceptable de celui-ci,
formules dans lesquelles :
A représente un hydrocarbure saturé ou non saturé, substitué ou non substitué comportant de 5 à 20 atomes de carbone ;
les radicaux Y₁ à Y₄ sont chacun indépendamment choisis dans le groupe constitué d'un groupe cyano (-C≡N), d'un groupe -C(=O)Ra, amine et alkyle, tandis que Ra représente un atome d'hydrogène, un atome d'halogène, un groupe hydroxy, alcoxy, thiohydroxy, thioalcoxy, aryloxy, thioaryloxy, thiol et amine ;
les radicaux X₁ à X₈ sont chacun indépendamment choisis dans le groupe constitué de l'atome d'hydrogène, d'un groupe alkyle, d'un atome d'halogène, d'un groupe cycloalkyle et aryle, à condition qu'au moins l'un des radicaux X₁ et X₂ dans la Formule I représente un atome de fluor non radioactif ou radioactif; et
les radicaux R₁ à R₁₀ sont chacun indépendamment choisis dans le groupe constitué d'un atome d'hydrogène, d'un groupe alkyle, alcényle, alcynyle, cycloalkyle et aryle ou, en variante, deux des radicaux R₁ à R₃, R₄ et R₅, R₆ et R₇ et/ou R₈ à R₁₀ forment un noyau carbocyclique, à condition que :
au moins l'un des radicaux R₈ à R₁₀ dans la Formule III représente un groupe alkyle saturé comportant de 11 à 15 atomes de carbone ;
au moins l'un des radicaux R₈ à R₁₀ dans la Formule III représente un groupe alkyle insaturé comportant de 5 à 20 atomes de carbone ; et/ou
au moins l'un des radicaux R₈ à R₁₀ dans la Formule III comprend au moins un atome de fluor non radioactif ou radioactif.

2. Composé selon la revendication 1, répondant à la formule générale I.

3. Composé selon la revendication 2, dans lequel Y₁ est choisi dans le groupe constitué d'un groupe cyano -(C≡N) et d'un groupe -C(=O)Ra.

4. Composé selon la revendication 2, dans lequel X₁ représente un atome de fluor et X₂ est choisi dans le groupe constitué d'un atome d'hydrogène, de fluor, de brome et d'iode.

5. Composé selon la revendication 2, dans lequel chacun des radicaux R₁ à R₃ représente un groupe alkyle.

6. Composé selon la revendication 2, dans lequel au moins l'un des radicaux R₁ à R₃ représente un groupe alkyle en C₅ à C₂₀.

7. Composé selon la revendication 2, étant choisi dans le groupe constitué du cyanofluorobromoborane de diméthyl-undécyl-amine, du cyanofluoroborane de triméthyl-amine, du cyanofluoroborane d'éthyl-diméthyl-amine, du cyanofluoroborane de butyl-diméthyl-amine, de l'ester méthylique de carboxyfluoroborane de triméthyl-amine, de l'ester éthylique de carboxyfluoroborane de triméthyl-amine, de l'ester méthylique de carboxyfluoroborane d'éthyl-diméthyl-amine, de l'ester méthylique de carboxyfluoroborane de butyl-diméthyl-amine, du cyanodifluoroborane de triméthyl-amine, de l'ester méthylique de carboxydifluoroborane de triméthyl-amine, de l'ester éthylique de carboxydifluoroborane de triméthyl-amine, du cyanofluorobromoborane de triméthyl-amine, de l'ester éthylique de carboxyfluorobromoborane de triméthyl-amine et du carboxydifluoroborane de triéthyl-amine.

8. Composé selon la revendication 1, répondant à la formule générale II.

9. Composé selon la revendication 8, dans lequel au moins l'un des radicaux X₃, X₄, X₅ et X₆ représente un atome de brome.

10. Composé selon la revendication 8, étant choisi dans le groupe constitué du bis-cyanoborane de N,N,N',N'-tétraméthyl-décane-1,10-diamine, du bis-cyanobromoborane de N,N,N',N'-tétraméthyl-décane-1,10-diamine, du bis-cyanodibromoborane de N,N,N',N'-tétraméthyl-décane-1,10-diamine, des bis-carboxyboranes de N,N,N',N'-tétraméthyl-décane-1,10-diamine, du bis-cyanoborane de N,N,N',N'-tétraméthyl-dodécane-1,12-diamine et du bis-cyanoborane de N,N,N',N'-tétraméthyl-tétradécane-1,14-diamine.

11. Composé selon la revendication 1, répondant à la formule générale III.

12. Composé selon la revendication 11, dans lequel au moins l'un des radicaux R₈ à R₁₀ comprend un groupe hydroxy, tandis que ledit groupe hydroxy est en position β par rapport à l'atome d'azote du groupe amine.

13. Composé selon la revendication 11, dans lequel au moins l'un des radicaux R₈ à R₁₀ comprend un atome de fluor, tandis que ledit atome de fluor est en position β par rapport à l'atome d'azote du groupe amine.

14. Composé selon la revendication 11, dans lequel au moins l'un des radicaux X₇ et X₈ représente un atome de brome.

15. Composé selon la revendication 11, étant choisi dans le groupe constitué du cyanoborane de diméthyl-undécyl-amine, du cyanobromoborane de diméthyl-undécyl-amine, du cyanodibromoborane de diméthyl-undécyl-amine, du cyanoborane de dodécyl-diméthyl-amine, du cyanoborane de 1-diméthylamino-2-méthyl-octan-2-ol, du cyanoborane de diméthyl-nonyl-amine, du cyanoborane de dimethyl-tridécyl-amine, du cyanoborane de diméthyl-pentadécyl-amine, du cyanoborane d'heptadécyl-diméthyl-amine, du cyanoborane de 1-diméthylamino-dodécan-2-ol, du cyanoborane de 1-diméthylamino-undécan-2-ol, du cyanofluorobromoborane de diméthyl-undécyl-amine et du cyanoborane de (2-fluoro-nonyl)-diméthyl-amine.

16. Composition pharmaceutique comprenant, en tant que substance active, le composé selon l'une quelconque des revendications 1 à 15 et un support pharmaceutiquement acceptable.

17. Utilisation du composé selon l'une quelconque des revendications 1 à 15 en tant que médicament.

18. Utilisation selon la revendication 17, dans laquelle ledit médicament est destiné au traitement d'une affection médicale associée à un microorganisme pathogène.

19. Utilisation selon la revendication 18, dans laquelle ledit microorganisme pathogène est un microorganisme résistant aux médicaments.

20. Procédé de préparation du composé selon la revendication 2, le procédé comprenant :
la mise en réaction d'un composé répondant à la formule générale IV : 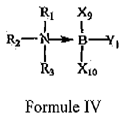 ou d'un sel pharmaceutiquement acceptable de celui-ci,
formule dans laquelle :
Y₁ est choisi dans le groupe constitué d'un groupe cyano -(C≡N), d'un groupe -C(=O) Ra, amine et alkyle, tandis que Ra représente un atome d'hydrogène, un atome d'halogène, un groupe hydroxy, alcoxy, thiohydroxy, thioalcoxy, aryloxy, thioaryloxy, thiol et amine ;
X₉ et X₁₀ sont chacun indépendamment choisis dans le groupe constitué d'un atome d'hydrogène, d'un groupe alkyle, d'un atome d'halogène, d'un groupe cycloalkyle, aryle, à condition qu'au moins l'un des radicaux X₉ et X₁₀ représente un atome de brome ; et
les radicaux R₁ à R₃ sont chacun indépendamment choisis dans le groupe constitué d'un atome d'hydrogène, d'un groupe alkyle, cycloalkyle et aryle ou, en variante, deux des radicaux R₁ à R₃ forment un noyau carbocyclique,
avec un agent de fluoration, pour ainsi obtenir le composé répondant à la formule générale I.

21. Procédé de préparation du composé selon la revendication 8, le procédé comprenant :
la mise en réaction d'un composé répondant à la formule générale V : 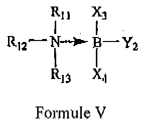 ou d'un sel pharmaceutiquement acceptable de celui-ci,
formule dans laquelle :
Y₂ est choisi dans le groupe constitué d'un groupe cyano -(C≡N), d'un groupe -C(=O) Ra, amine et alkyle, tandis que Ra représente un atome d'hydrogène, un atome d'halogène, un groupe hydroxy, alcoxy, thiohydroxy, thioalcoxy, aryloxy, thioaryloxy, thiol et amine ;
X₃ et X₄ sont chacun indépendamment choisis dans le groupe constitué d'un atome d'hydrogène, d'un groupe alkyle, d'un atome d'halogène, d'un groupe cycloalkyle et aryle ; et
les radicaux R₁₁ à R₁₃ sont chacun indépendamment choisis dans le groupe constitué d'un atome d'hydrogène, d'un groupe alkyle, cycloalkyle et aryle ou, en variante, deux des radicaux R₁₁ à R₁₃ forment un noyau carbocyclique, à condition qu'au moins l'un des radicaux R₁₁ à R₁₃ dans la Formule V représente un groupe méthyle,
avec un composé répondant à la formule générale VI :
W₁-A-W₂ Formule VI
formule dans laquelle :
A représente un hydrocarbure saturé ou non saturé, substitué ou non substitué comportant de 5 à 20 atomes de carbone ; et
W₁ et W₂ représentent chacun indépendamment un groupe fonctionnel,
en présence de n-alkyllithium, pour ainsi obtenir le composé répondant à la formule générale II.

22. Procédé de préparation du composé selon la revendication 11, dans lequel au moins l'un des radicaux R₈ à R₁₀ représente ledit groupe alkyle saturé comportant de 11 à 15 atomes de carbone ou un groupe alkyle insaturé comportant de 5 à 20 atomes de carbone, le procédé comprenant :
la mise en réaction d'un composé répondant à la formule générale V : 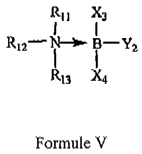 ou d'un sel pharmaceutiquement acceptable de celui-ci,
formule dans laquelle :
Y₂ est choisi dans le groupe constitué d'un groupe cyano -(C≡N), d'un groupe -C(=O)Ra, amine et alkyle, tandis que Ra représente un atome d'hydrogène, un atome d'halogène, un groupe hydroxy, alcoxy, thiohydroxy, thioalcoxy, aryloxy, thioaryloxy, thiol et amine ;
X₃ et X₄ sont chacun indépendamment choisis dans le groupe constitué d'un atome d'hydrogène, d'un groupe alkyle, d'un atome d'halogène, d'un groupe cycloalkyle et aryle ; et
les radicaux R₁₁ à R₁₃ sont chacun indépendamment choisis dans le groupe constitué d'un atome d'hydrogène, d'un groupe alkyle, cycloalkyle et aryle ou, en variante, deux des radicaux R₁₁ à R₁₃ forment un noyau carbocyclique, à condition qu'au moins l'un des radicaux R₁₁ à R₁₃ dans la Formule V représente un groupe méthyle,
avec un composé répondant à la formule générale VII :
R₁₄-W₃ Formule VII
formule dans laquelle :
R₁₄ est choisi dans le groupe constitué d'un groupe alkyle saturé comportant au moins 10 atomes de carbone ou un groupe alkyle insaturé comportant au moins 4 atomes de carbone ; et
W₃ représente un groupe fonctionnel,
en présence de n-alkyllithium, pour ainsi obtenir le composé répondant à la formule générale III.

23. Procédé de préparation du composé selon la revendication 11, dans lequel au moins l'un des radicaux R₈ à R₁₀ comprend un atome de fluor, le procédé comprenant :
la mise en réaction d'un composé répondant à la formule générale V : 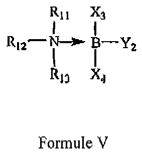 ou d'un sel pharmaceutiquement acceptable de celui-ci,
formule dans laquelle :
Y₂ est choisi dans le groupe constitué d'un groupe cyano -(C≡N), d'un groupe -C(=O) Ra, amine et alkyle, tandis que Ra représente un atome d'hydrogène, un atome d'halogène, un groupe hydroxy, alcoxy, thiohydroxy, thioalcoxy, aryloxy, thioaryloxy, thiol et amine ;
X₃ et X₄ sont chacun indépendamment choisis dans le groupe constitué d'un atome d'hydrogène, d'un groupe alkyle, d'un atome d'halogène, d'un groupe cycloalkyle et aryle ; et
les radicaux R₁₁ à R₁₃ sont chacun indépendamment choisis dans le groupe constitué d'un atome d'hydrogène, d'un groupe alkyle, cycloalkyle et aryle ou, en variante, deux des radicaux R₁₁ à R₁₃ forment un noyau carbocyclique, à condition qu'au moins l'un des radicaux R₁₁ à R₁₃ dans la Formule V représente un groupe méthyle,
avec un composé répondant à la formule générale VIII ;
R₁₅-W₄-R₁₆ Formule VIII
formule dans laquelle :
W₄ représente un groupe carboxy (C=O) ;
R₁₅ est choisi dans le groupe constitué des groupes alkyle, cycloalkyle et aryle; et
R₁₆ est choisi dans le groupe constitué d'un atome d'hydrogène, d'un groupe alkyle, cycloalkyle et aryle,
en présence de n-alkyllithium, pour ainsi obtenir un composé répondant à ladite formule générale III, dans lequel au moins l'un des radicaux R₈ à R₁₀ comprend un groupe hydroxy ou alcoxy ; et
la conversion dudit groupe hydroxy ou dudit groupe alcoxy en ledit atome de fluor.

24. Composé marqué répondant à la formule générale X ou XI : 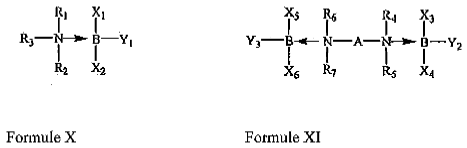 ou sel pharmaceutiquement acceptable de celui-ci,
formules dans lesquelles :
les radicaux Y₁ à Y₃ sont chacun indépendamment choisis dans le groupe constitué d'un groupe cyano (-C≡N), d'un groupe -C(=O) Ra, amine et alkyle, tandis que Ra représente un atome d'hydrogène, un atome d'halogène, un groupe hydroxy, alcoxy, thiohydroxy, thioalcoxy, aryloxy, thioaryloxy, thiol et amine ;
les radicaux X₁ à X₆ sont chacun indépendamment choisis dans le groupe constitué d'un atome d'hydrogène, d'un groupe alkyle, d'un atome d'halogène, d'un groupe cycloalkyle et aryle,
les radicaux R₁ à R₇ sont chacun indépendamment choisis dans le groupe constitué d'un atome d'hydrogène, d'un groupe alkyle, cycloalkyle et aryle ou, en variante, deux des radicaux R₁ à R₃, R₄ et R₅ et/ou R₆ et R₇ forment un noyau carbocyclique, et
A représente un hydrocarbure saturé ou non saturé, substitué ou non substitué comportant de 5 à 20 atomes de carbone,
tandis qu'au moins l'un des radicaux X₁ à X₆, R₁ à R₇ et A comprend un atome radioactif et/ou au moins un atome de bore est un atome de bore radioactif.

25. Composition pharmaceutique comprenant, en tant que substance active, le composé marqué selon la revendication 24 et un support pharmaceutiquement acceptable.

26. Utilisation du composé marqué selon la revendication 24 ou de la composition selon la revendication 25 dans une radio-imagerie et/ou une radiothérapie.

27. Utilisation du composé marqué selon la revendication 24 dans la préparation d'un agent diagnostique.
